# EUROPEAN PATENT SPECIFICATION

(11) **EP 4 153 051 B1**
(45) Date of publication and mention of the grant of the patent: **19.08.2026**
(21) Application number: 21809747.5
(22) Date of filing: 21.05.2021
(51) Int. Cl.: A61B 5/24, A61B 5/12, A61B 5/38, A61B 5/00

(54) **SYSTEMS AND COMPUTER PROGRAMS FOR DETECTING THE PRESENCE OF ELECTRICALLY EVOKED COMPOUND ACTION POTENTIALS (ECAPS)**
SYSTEME UND COMPUTERPROGRAMME ZUR ERKENNUNG DES VORHANDENSEINS ELEKTRISCH EVOZIERTER VERBINDUNGSAKTIONSPOTENZIALE (ECAPS)
SYSTÈMES ET PROGRAMMES INFORMATIQUES POUR LA DÉTECTION DE LA PRÉSENCE DE POTENTIELS D'ACTION COMPOSÉS ÉVOQUÉS ÉLECTRIQUEMENT (ECAPS)

(30) Priority: 22.05.2020 US 202063028677 P; 29.01.2021 US 202163143689 P; 30.04.2021 US 202163182402 P
(43) Date of publication of application: 29.03.2023
(73) Proprietor: Ohio State Innovation Foundation, Columbus, OH 43210 (US); THE REGENTS OF THE UNIVERSITY OF MICHIGAN, Ann Arbor, MI 48109-2590 (US); UMC Utrecht Holding B.V., 3584 CS Utrecht (NL)
(72) Inventor: SKIDMORE, Jeffrey, Columbus, Ohio 43201 (US); HE, Shuman, Columbus, Ohio 43201 (US); NING, Xia, Columbus, Ohio 43201 (US); PFINGST, Bryan E., Ann Arbor, Michigan 48109-2590 (US); RAMEKERS, Dyan, 3552EH Utrecht (NL)
(74) Representative: Zimmermann & Partner Patentanwälte mbB
(86) International application number: PCT/US2021/033604
(87) International publication number: WO 2021/237059

(56) References cited:
- US-A1- 2006 276 722
- US-A1- 2015 126 897
- US-A1- 2018 146 927
- US-A1- 2019 275 331
- US-A1- 2020 054 879
- US-B2- 8 165 687
- US-B2- 9 044 155

## Description

### BACKGROUND

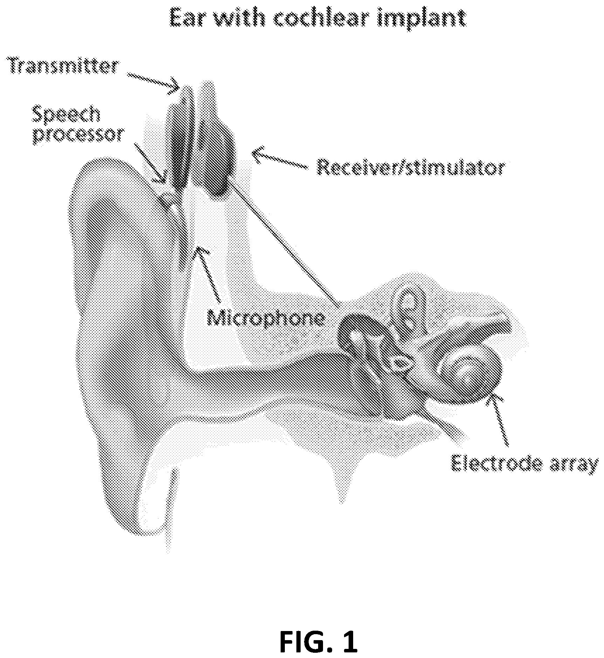

As shown in FIG. 1, cochlear implants convert sounds (picked up by a microphone) into electrical representations (in the sound processor) which are transmitted through the head and stimulate nerves surrounding the cochlea. Cochlear implants bypass the normal hearing pathway (i.e. middle ear bones and inner ear hair cells), which is usually not functional when somebody is deaf.

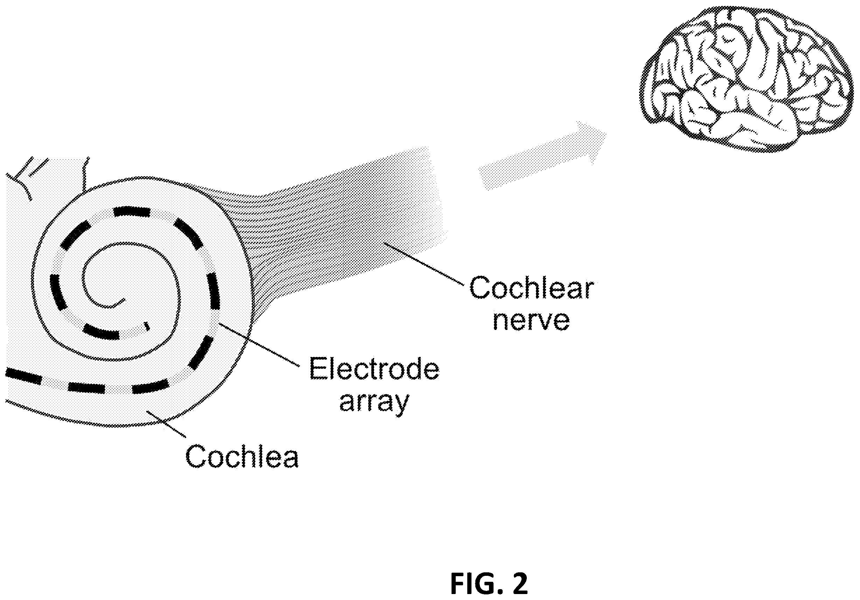

However, cochlear implants still rely on the cochlear nerve to be functional in order to carry the signal to the brain for further processing and interpretation (as shown in FIG. 2).

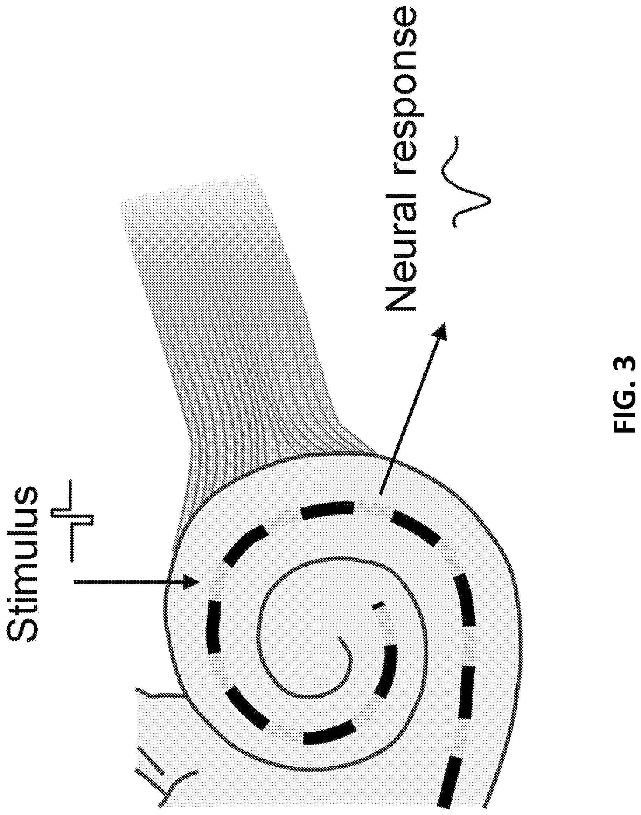

In humans, there is no way to directly evaluate how well the cochlear nerve functions. However, as shown in FIG. 3, user defined stimuli can be sent through one electrode to stimulate the surrounding neurons. The neurons then respond and the electrical response can be recorded by a neighboring electrode. The neural response is called the electrically-evoked compound action potential (eCAP). The eCAP response can be characterized as an indirect measure of neural function.

However, a challenge for clinical application of eCAP technology is identifying the presence of an eCAP instead of measurement noise. Identifying eCAPs is typically done visually by a highly trained researcher and/or audiologist. This takes considerable amount of time and training, which prevents the clinical application of objective eCAP measures.

Therefore, systems and methods are desired that overcome challenges in the art, some of which are described above. More specifically, there is a need for systems and methods to automatically detecting the presence of electrically evoked compound action potentials (eCAPs) in cochlear implant patients.

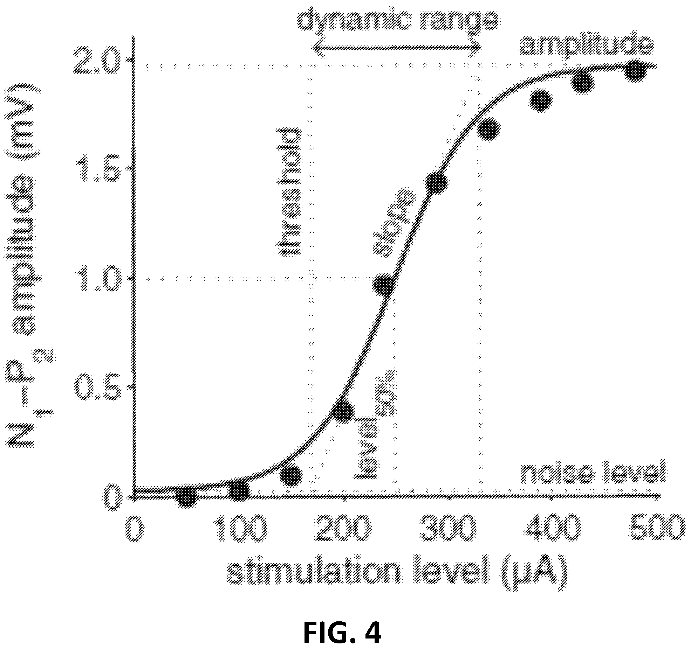

Furthermore, the most used parameter to characterize the response is the slope of the eCAP amplitude growth function (AGF). The AGF is recorded by measuring the eCAP response amplitude voltage (y-axis), with increasing stimulation levels/current (x-axis), as shown in FIG. 4, which shows AGF data for an anesthetized animal that resembles a sigmoidal function. The slope of the eCAP AGF has been shown to be strongly correlated with the number of surviving auditory neurons in animals (Ramekers et al., 2014; Pfingst et al., 2015; Pfingst et al., 2017).

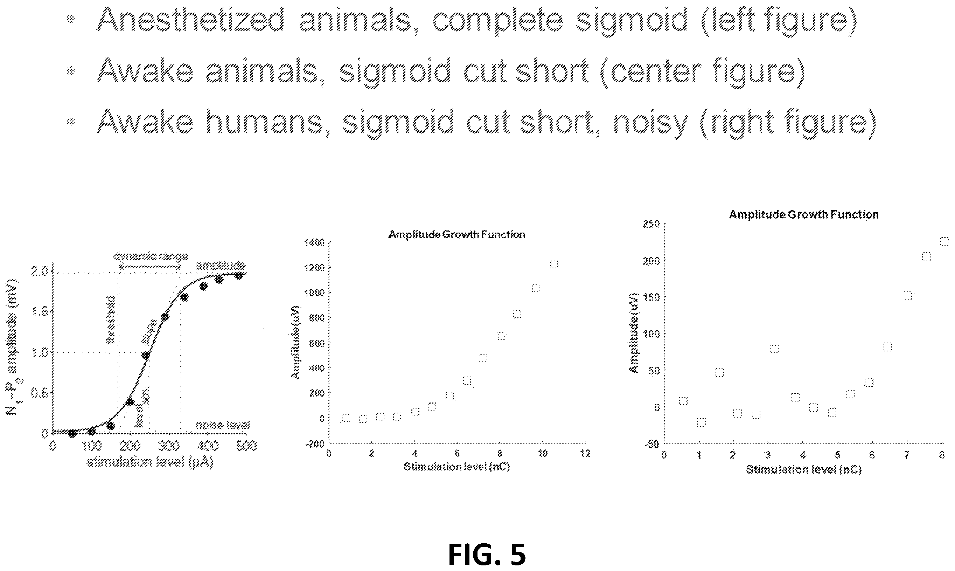

There are three general categories of AGF data of interest (see FIG. 5): Anesthetized animals, awake animals, and awake humans. Anesthetized animals can be stimulated at high enough levels that the function saturates (i.e. reaches a plateau). These AGFs are easily fit with a sigmoidal function (s-shape function with a plateau curving to a linear region, curving to another plateau), and the slope is easily obtained. The slope is defined as the line tangent to the sigmoidal fit at the inflection point (level 50% in the left figure in FIG. 5). See Ramekers et al., 2014, detailing the sigmoidal function (Equation 1) and slope calculation.

For awake animal and awake humans (FIG. 5, center and right figures), saturation of the AGF is rarely obtained because it is not possible to stimulate at high enough levels to reach the upper plateau. This is due to subject/patient discomfort and/or exceeds the stimulation capabilities (i.e. voltage level) of the implant device. Therefore, the AGF usually resembles a 'partial sigmoid.' Moreover, these datasets are not as 'clean' as for the anesthetized animals, meaning there is a lot more 'noise' or measurement error/variability.

Researchers and cochlear implant manufacturers have been trying to quantify the slope of the eCAP AGF in humans (e.g., Brown et al., 1990; Kim et al, 2010; He et al., 2018, Schvartz-Leyzac and Pfingst, 2016, 2018, ). Because the sigmoidal fitting does not work well for these datasets, different research groups and cochlear implant manufacturers/suppliers use different approaches to quantify the eCAP AGF slope. Due to the well-known limitations of sigmoidal and linear regression in calculating the slope, most researchers now create custom methods for their specific data set. This involves the expert researcher manually looking at the raw data, deciding which data points to exclude, and then fitting a line with linear regression. This is not translatable to clinical care because it requires an expert user and is not automated. Some groups make blanket criteria to exclude data points (e.g., any value less than 100 uV). However, these techniques only work for the specific dataset being analyzed. They do not apply generally to all AGFs, including multiple patient populations with different neural response characteristics (e.g., children and adults).

Currently; however, there is no standard way to quantify the slope in humans. Therefore, researchers and cochlear implant manufacturers/providers calculate the slope in different ways. However, each current method used to quantify the slope has limitations. Therefore, systems and methods are desired that overcome challenges in the art, some of which are described above. There is a need for systems and methods to quantify the slope of the eCAP AGF that addresses all of these limitations and can work for any eCAP AGF of all animals, whether human (adults and children) or non-human.

Finally, older cochlear implant (CI) patients generally have worse speech perception 62 capabilities than younger CI patients (e.g., Sladen & Zappler, 2005; Lenarz et al., 2012; 63 Lin et al., 2012; Roberts et al., 2013). While declining cognitive function with advancing age may contribute to these speech perception deficits in older CI patients (Budenz et al., 2011; Lin et al., 2011, 2012), poor speech perception has also been attributed to deteriorations in the auditory system (Friedland et al., 2010; Roberts et al., 2013). The mechanisms underlying speech perception deficits in older CI patients still remain unclear, which creates challenges in providing clinical care for these patients. As a step toward understanding the neurophysiological mechanisms underlying speech perception deficits in older CI patients, the effects of advanced age on how effectively a CI electrode stimulates the targeted cochlear nerve (CN) fibers (i.e., the electrode-neuron interface [ENI]) (Bierer, 2010) is examined.

Multiple factors affect the quality of the ENI, including the position of the electrode array within the cochlea, the impedances of intracochlear tissues, and the number and responsiveness of CN fibers (Bierer, 2010). Electrical stimuli are delivered by the CI to nearby CN fibers which then encode and transmit the information to higher-level neural structures for further processing and interpretation. Therefore, the quality of the ENI should theoretically be an important factor for speech perception. Numerous studies provide results that support this theory (e.g., Kim et al., 2010; Kirby et al., 2010, 2012; Garadat et al., 2013; Long et al., 2014; Pfingst et al., 2015; He et al., 2018; Skidmore et al., 2021a).

In CI users, the quality of the ENI can be assessed using neurophysiological measures of the eCAP. Like the ENI, neurophysiological measures of the eCAP are affected by electrode position, intracochlear resistance, and CN fiber density (e.g., Eisen & Franck 2004; Shepherd et al., 2004; Brown et al., 2010; Ramekers et al. 2014; Schvartz-Leyzac & Pfingst, 2016; Pfingst et al. 2015, 2017; He et al., 2018; Schvartz-Leyzac et al., 2020). Specifically, animals with higher densities of spiral ganglion neurons (SGNs) tend to have shorter refractory times, larger eCAP amplitudes, and larger slopes of eCAP AGFs than animals with fewer functional SGNs (Shepherd et al., 2004; Ramekers et al. 2014; Pfingst et al. 2015, 2017). In humans, eCAP thresholds and the slopes of eCAP AGFs have been shown to be affected by electrode position and intracochlear resistance (e.g., Young & Grohne, 2001; Eisen & Franck, 2004; Brown et al., 2010; Schvartz-Leyzac & Pfingst, 2016; Schvartz-Leyzac et al., 2020). Additionally, children with small or absent CNs present in imaging results (i.e., children with cochlear deficiency [CND]) have longer refractory times, smaller eCAP amplitudes, higher eCAP thresholds, and smaller eCAP AGF slopes compared to age-matched children with normal-sized CNs (He et al., 2018). Therefore, eCAP measures can be considered as a functional readout for the quality of the ENI.

eCAPs have been used to compare the quality of the ENI between pediatric and adult CI users. Human eCAP data suggests that the interface between CI electrodes and the target CN fibers differs between children and adults with CIs. Specifically, multiple studies have demonstrated that children and young adults have larger eCAP AGF slopes than older CI patients (Hughes et al., 2001; Cafarelli Dees et al., 2005; Brown et al., 2010; 107 Jahn & Arenberg, 2020). However, these differences in age groups may be reflective of differences in etiology between patients who are pre-lingually deafened vs post-lingually deafened (Bodmer et al., 2007; Brown et al., 2010; Zarowski et al., 2020). To date, the effect of advancing age on the quality of the ENI in post-lingually deafened adult CI patients has not been well established.

US20180146927A1 relates to Eliminating acquisition-related artifacts in electrophysiological recording. Therefore, systems and methods are desired that overcome challenges in the art, some of which are described above. There is a need for systems and methods to quantify the quality of the local (i.e., electrode-specific) ENI, and assess the effects of advanced age on the local ENI in CI patients.

### SUMMARY

The invention is defined in the appended set of claims.

Disclosed and described herein are systems and methods to address the above-described challenges with a system according to claim 1 and a computer-program product according to claim 8.

In particular, systems and methods are disclosed to automatically detect the presence of eCAPs in cochlear implant patients. As noted above, the eCAP is a measure of the responses of auditory nerve fibers that can recorded directly from a cochlear implant. A challenge for clinical application of eCAP technology is identifying the presence of an eCAP instead of measurement noise. The disclosed systems and methods automatically discern whether a measured value comprises an eCAP or is noise. In broad terms, the method compares neural response waveforms with a template eCAP waveform to determine if an eCAP exists or not in the neural response. Systems are also disclosed to implement the methods

Further, systems and methods are disclosed to quantify the slope of neural response functions. The disclosed systems and methods have several advantages including enabling individualized clinical care to improve hearing capabilities for existing cochlear implant users [including selection and/or adjustment (manual and/or automated) of the cochlear implant on a patient-specific basis]; and standardization by use of the disclosed methods, which can be used by all researchers to easily compare results across studies. Disclosed herein are embodiments of a system and a method of refining raw data of an electrically evoked compound action potential (eCAP) amplitude growth function (AGF) and utilizing maximum (i.e., steepest) slope from moving linear regression to effectively estimate cochlear nerve function. The disclosed methods provide an appropriate estimate for the slope for any raw AGF and correlates with an estimated number of surviving neurons in the cochlear nerve. Once the estimated number of surviving neurons in the cochlear nerve are determined, this information can be used to provide patients with a better clinical experience including design, selection and/or specification of the cochlear implant as well as adjustment of the cochlear implant (which can be manual and/or automated).

One aspect of the method to quantify the slope of neural response functions includes 1) receiving raw data comprised of a plurality of data points of AGF data (i.e., x,y pairs of stimulation level and eCAP amplitude); 2) resampling the raw data into a plurality (e.g., 100) of linearly spaced data points of AGF data; 3) perform linear regression on a moving window comprised of a subset (N) of the plurality of linearly spaced data points of AGF data (e.g., N= 50 points), 3)(a), perform linear regression on a first window of comprised of data points 1 to N of the plurality of linearly spaced data points of AGF data (e.g., linear regression on data points [1-50] of the linearly spaced data points of AGF data) to determine a slope of the first window, 3)(b) move the window by one point (the subset is still comprised of the same number of data points) to form a second window and perform linear regression on data points 2 to N+1 (e.g., perform linear regression on data points [2-51]) to determine a slope of this second window, 3)(c) continue to perform linear regression on the data points of the plurality of linearly spaced data points of AGF data using the same size subset (N) of data points until the end of the plurality of linearly spaced data points of AGF data is reached (e.g., perform linear regression on data points [50-100]) to determine a slope of each of the plurality of different moving windows; 4) determine the steepest (i.e., maximum) slope among the slopes calculated in the plurality of different windows; and 5) correlate the selected steepest slope with surviving neurons in the cochlear nerve.

Finally, systems and methods are disclosed to estimate the quality of the electrode neuron interface (ENI) ( at individual electrode locations in cochlear implant (CI) users. The quality of the ENI is significantly affected by the advanced age of CI patients. This aging effect appears be stronger in the basal region than in the middle and basal regions of the cochlea. However, this result is likely due to the combined effect of advanced age and etiologies that cause sensorineural hearing loss.

Additional advantages will be set forth in part in the description which follows or may be learned by practice. The advantages will be realized and attained by means of the elements and combinations particularly pointed out in the appended claims. It is to be understood that both the foregoing general description and the following detailed description are exemplary and explanatory only and are not restrictive.

### BRIEF DESCRIPTION OF THE DRAWINGS

The accompanying drawings, which are incorporated in and constitute a part of this specification, illustrate embodiments and together with the description, serve to explain the principles of the methods and systems.
FIG. 1 is an image illustrating an example of a cochlear implant.
FIG. 2 is an image illustrating how cochlear implants still rely on the cochlear nerve to be functional in order to carry the signal to the brain for further processing and interpretation.
FIG. 3 is an illustration of how user defined stimuli can be sent through one electrode of a cochlear implant to stimulate the surrounding neurons, with the compound neural response (i.e., eCAP) being recorded by a different electrode of the cochlear implant.
FIG. 4 illustrates an exemplary amplitude growth function (AGF) that is recorded by measuring the eCAP response amplitude voltage (y-axis), with increasing stimulation levels/current (x-axis), which resembles a sigmoidal function.
FIG. 5 is an illustration of three general categories of AGF data of interest.
FIG. 6A illustrates an exemplary flowchart for a method of determining if an eCAP exists or not in a neural response.
FIG. 6B illustrates an example of a template eCAP waveform.
FIG. 6C illustrates an example of a comparison of the recorded neural response and the re-sampled neural response.
FIG. 6D shows the recorded neural response and the re-sampled neural response after the mean voltages recorded in the a first time period (e.g., 600 µ-sec) are subtracted from the template and neural response waveforms.
FIG. 6E illustrates an example of scaling the template waveform vertically (voltage) to match the neural response waveform.
FIG. 6F illustrates an example of scaling the template waveform horizontally (time) to match the neural response waveform.
FIG. 6G illustrates an example of trimming the waveforms to the time period that overlaps after scaling.
FIG. 6H illustrates an example of re-sampling the waveforms at the same time periods as each other with higher resolution sampling occurring before the first time period (e.g., 600 µ-sec) to place emphasis on the first part of the waveforms in the correlation analysis.
FIG. 6I illustrates an example of calculating a correlation between the template and neural response waveform.
FIG. 6J illustrates an example correlation where the neural response is an eCAP (e.g., correlation value greater than 0.6).
FIG. 6K illustrates an example correlation where the neural response is not an eCAP (e.g., less than 0.6 correlation value).
FIG. 7 illustrates an exemplary flowchart for a method of providing an estimate for the slope of a neural response function characterized by a series of stimulation levels (i.e., input data) and corresponding amplitude of neural responses (i.e., output data).
FIG. 8 illustrates some of the steps of a method of providing an estimate for the slope of a neural response function characterized by a series of stimulation levels (i.e., input data) and corresponding amplitude of neural responses (i.e., output data).
FIGS. 9A-9P illustrate comparisons of calculating the slope of the AGF data using the disclosed embodiments and conventional methods of calculating the slope, in both animals and humans.

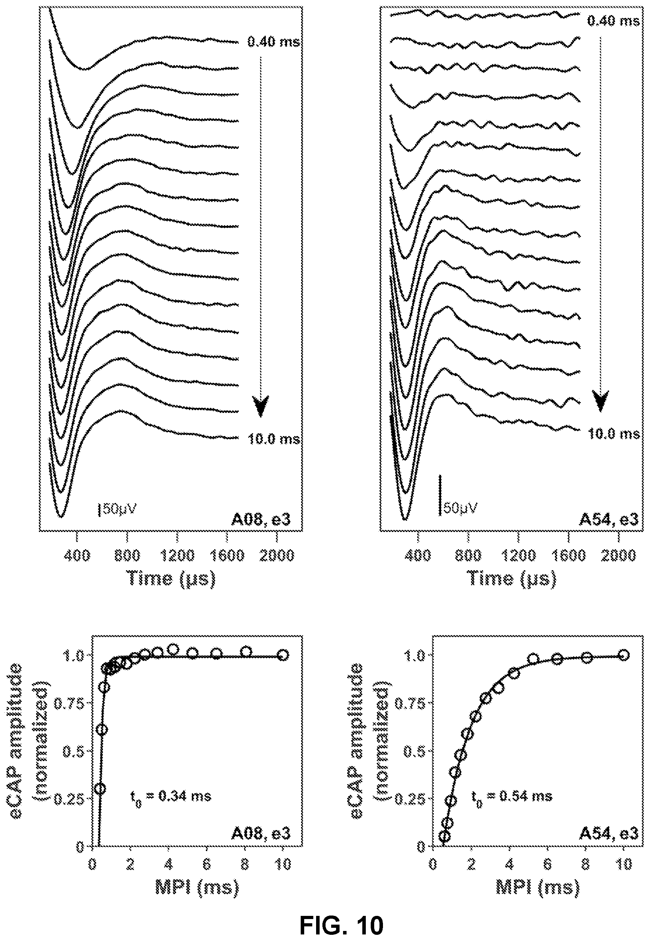

FIG. 10, upper panels, illustrate eCAP waveforms measured at different masker-probe intervals (MPIs) for stimulating electrode 3 in one adult younger than 68 years (A08), and one adult older than 68 years (A54). Waveforms are arranged based on MPI duration (in ms), with responses evoked by short MPIs displayed at the top. FIG. 10 lower panels, illustrate refractory recovery functions (round symbols) obtained from the waveforms in the upper panels. The fitted exponential decay function for each refractory recovery function (black line) and the resulting estimation for the refractory time (t0) are also provided. The participant and electrode number are included in the lower right corner of each panel. The stimulations were performed at the maximum comfortable level for each participant and electrode.

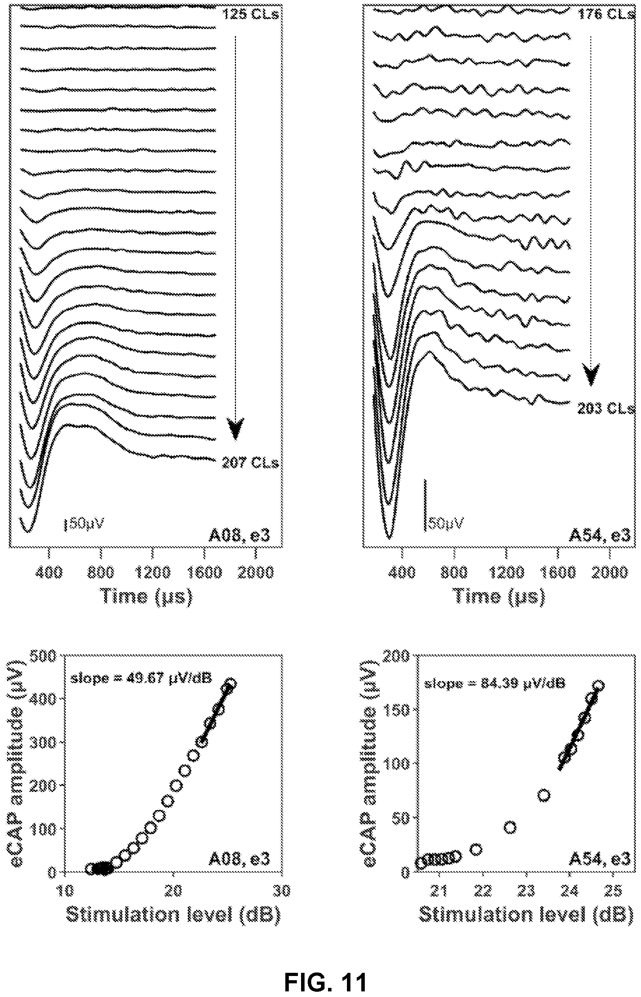

FIG. 11, upper panels, illustrate eCAP waveforms measured at different stimulation intensities for electrode 3 in one adult younger than 68 years (A08) and one adult older than 68 years (A54). Waveforms are arranged based on stimulation level (in current levels [CLs]), with responses evoked by the smallest stimulation level (i.e., eCAP threshold) displayed at the top. The largest stimulation level presented was the maximum comfortable level (i.e., C level). FIG. 11, lower panels, illustrate eCAP amplitude growth functions (AGFs, round symbols) obtained from the waveforms in the upper panels after converting stimulation levels to logarithmic units (dB re 1 nanocoulomb [nC]). The slope is provided for each eCAP AGF, along with a representation of the slope estimated with sliding window linear regression (solid black line). The participant and electrode number are included in the lower right corner of each panel.
FIG. 12 illustrates results of eCAP parameters (mean and standard deviation) measured for children with cochlear nerve deficiency (CND) and children with normal-sized cochlear nerves (NSCNs) used for model training. Ordered from left to right are the estimated absolute refractory recovery times (i.e., *t*0), eCAP thresholds, slopes of eCAP AGFs, and N1 peak latencies. Significant group differences are indicated with asterisks.
FIGS. 13A and 13B illustrate local electrode-neuron interface indices for children with normal-sized cochlear nerves and sensorineural hearing loss (S, FIG. 13A) and children with cochlear nerve deficiency (CND, FIG. 13B) included in the validation dataset.
FIG. 14 illustrates results of eCAP parameters (means and standard deviations) measured for adults younger than 68 years (black bars) and adults older than 68 years (white bars) at three electrode locations. Ordered in rows from top to bottom are the estimated absolute refractory recovery times (i.e., *t*0), eCAP thresholds, slopes of eCAP AGFs, and N1 peak latencies.
FIG. 15 illustrates the means and standard deviations of local electrode-neuron interface indices for adults younger than 68 years (black bars) and older than 68 years (white bars) at three electrode locations.
FIG. 16 illustrates local electrode-neuron interface indices for adult cochlear implant patients as a function of age at three electrode locations. Results from linear regression analyses are also shown.
FIG. 17 illustrates an exemplary computing device that can be used according to embodiments described herein.

### DETAILED DESCRIPTION

Before the present methods and systems are disclosed and described, it is to be understood that the methods and systems are not limited to specific synthetic methods, specific components, or to particular compositions. It is also to be understood that the terminology used herein is for the purpose of describing particular embodiments only.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal embodiment. "Such as" is not used in a restrictive sense, but for explanatory purposes.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

As will be appreciated by one skilled in the art, the methods and systems may take the form of an entirely hardware embodiment, an entirely software embodiment, or an embodiment combining software and hardware aspects. Furthermore, the methods and systems may take the form of a computer program product on a computer-readable storage medium having computer-readable program instructions (e.g., computer software) embodied in the storage medium. More particularly, the present methods and systems may take the form of web-implemented computer software. Any suitable computer-readable storage medium may be utilized including hard disks, CD-ROMs, optical storage devices, or magnetic storage devices.

Embodiments of the methods and systems are described below with reference to block diagrams and flowchart illustrations of methods, systems, apparatuses and computer program products. It will be understood that each block of the block diagrams and flowchart illustrations, and combinations of blocks in the block diagrams and flowchart illustrations, respectively, can be implemented by computer program instructions. These computer program instructions may be loaded onto a general purpose computer, special purpose computer, or other programmable data processing apparatus to produce a machine, such that the instructions which execute on the computer or other programmable data processing apparatus create a means for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including computer-readable instructions for implementing the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions specified in the flowchart block or blocks.

Accordingly, blocks of the block diagrams and flowchart illustrations support combinations of means for performing the specified functions, combinations of steps for performing the specified functions and program instruction means for performing the specified functions. It will also be understood that each block of the block diagrams and flowchart illustrations, and combinations of blocks in the block diagrams and flowchart illustrations, can be implemented by special purpose hardware-based computer systems that perform the specified functions or steps, or combinations of special purpose hardware and computer instructions.

The present methods and systems may be understood more readily by reference to the following detailed description of preferred embodiments and the Examples included therein and to the Figures and their previous and following description.

### I. DETECTING THE PRESENCE OF eCAPS

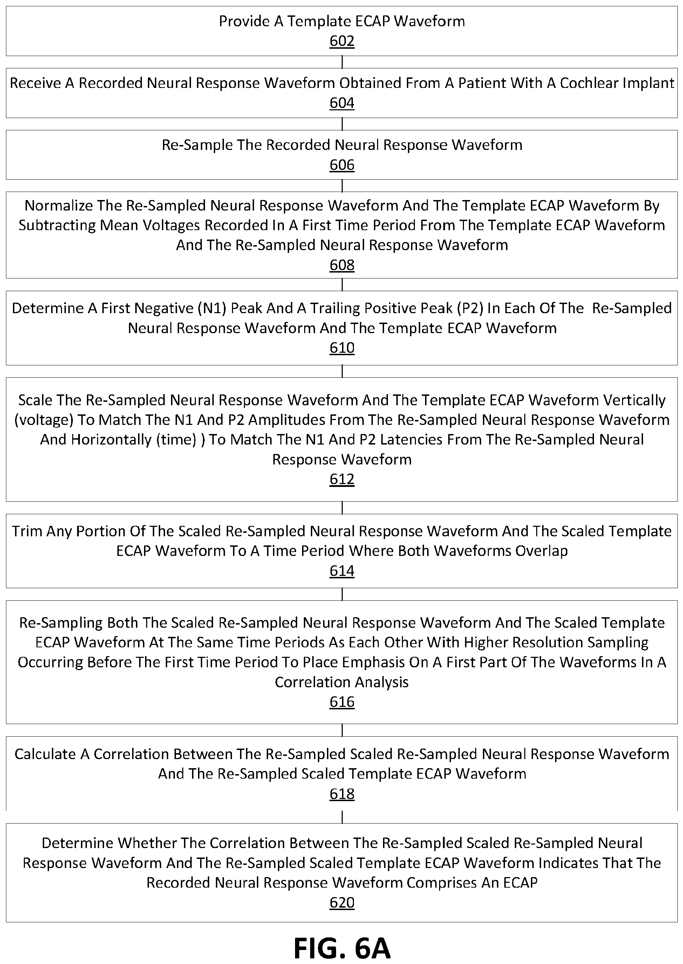

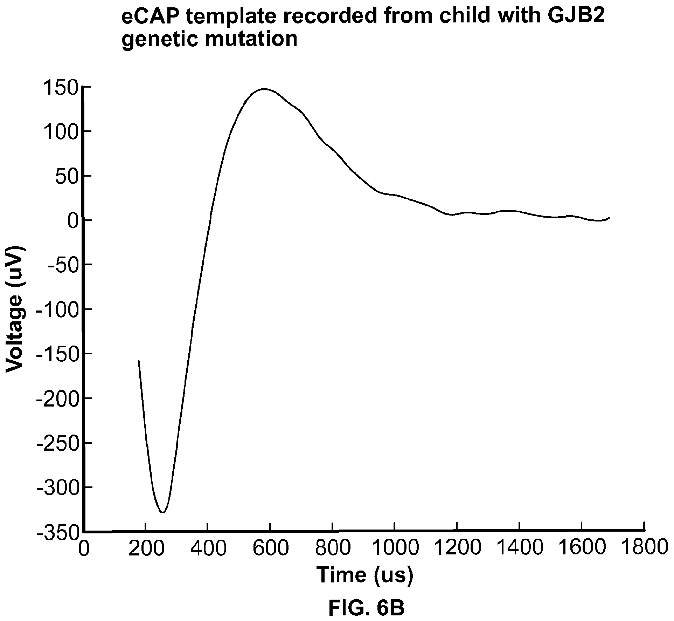

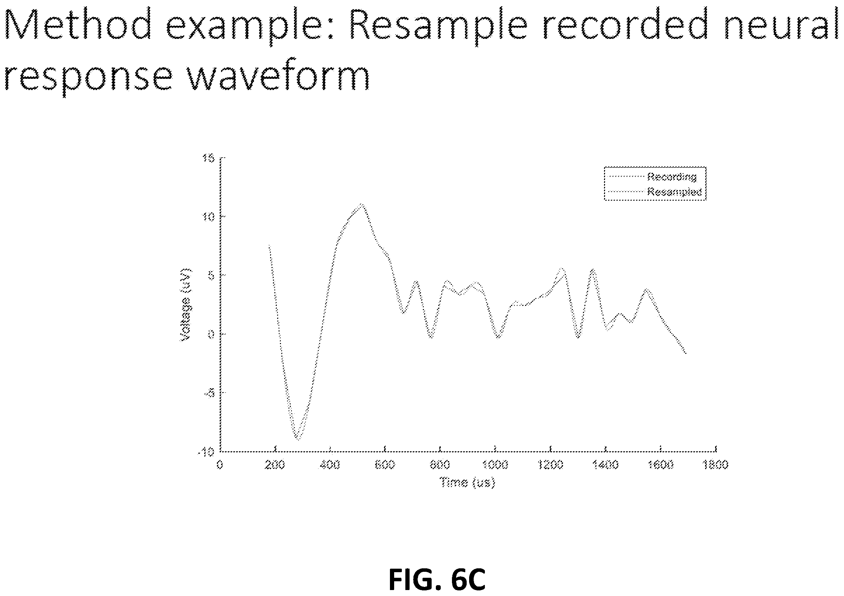

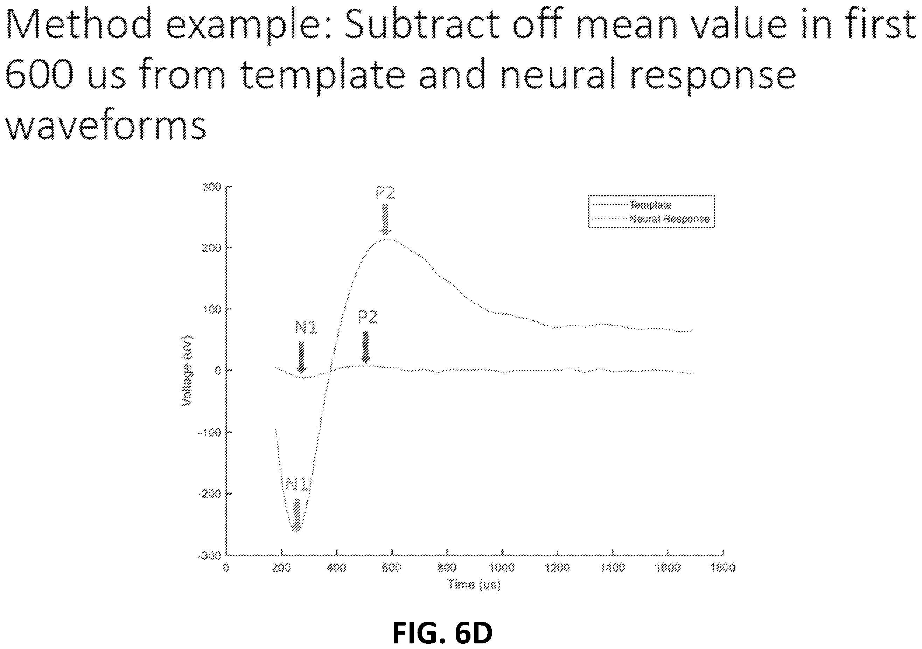

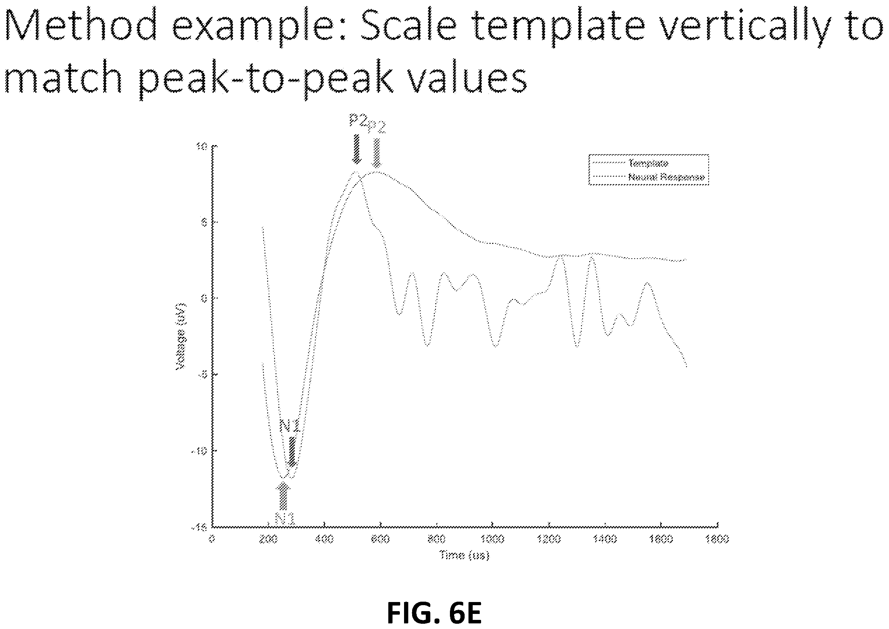

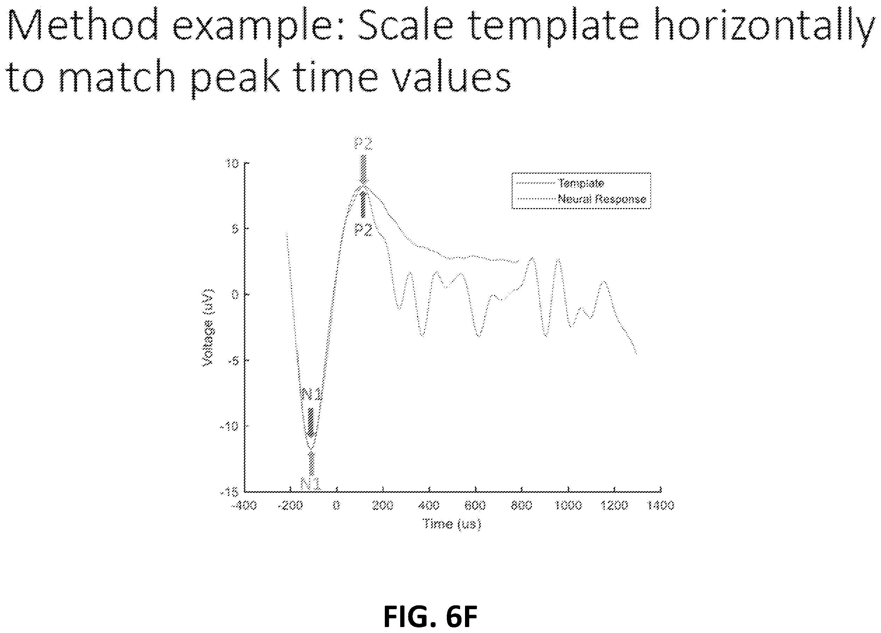

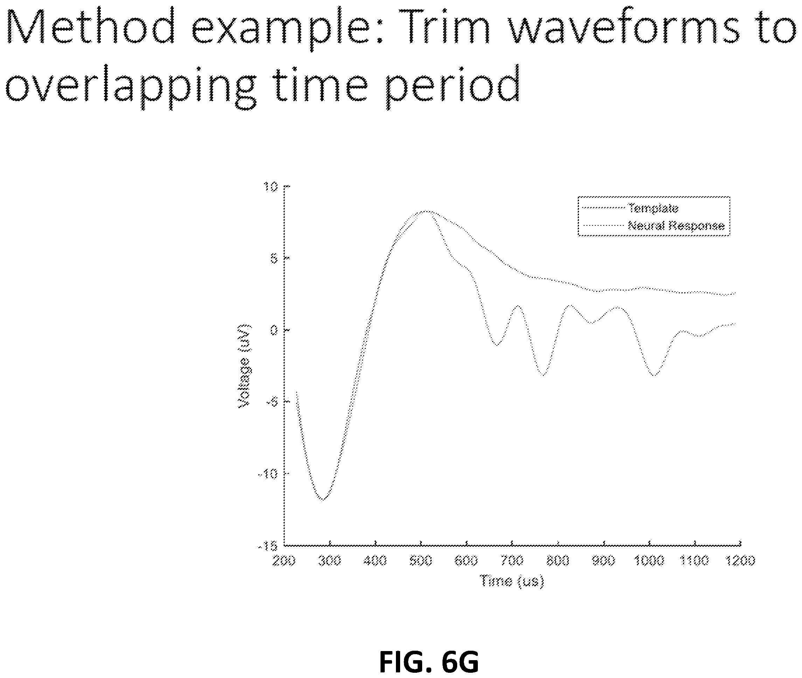

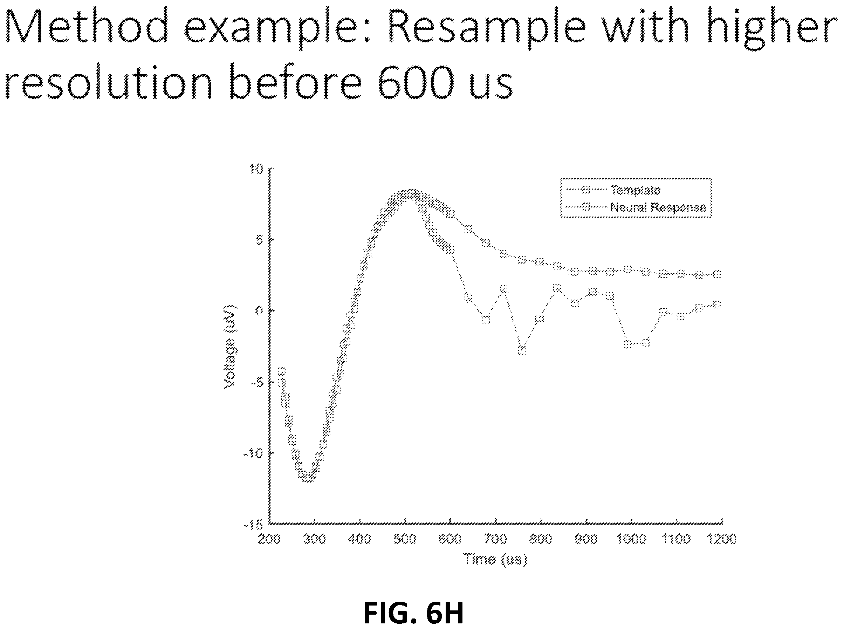

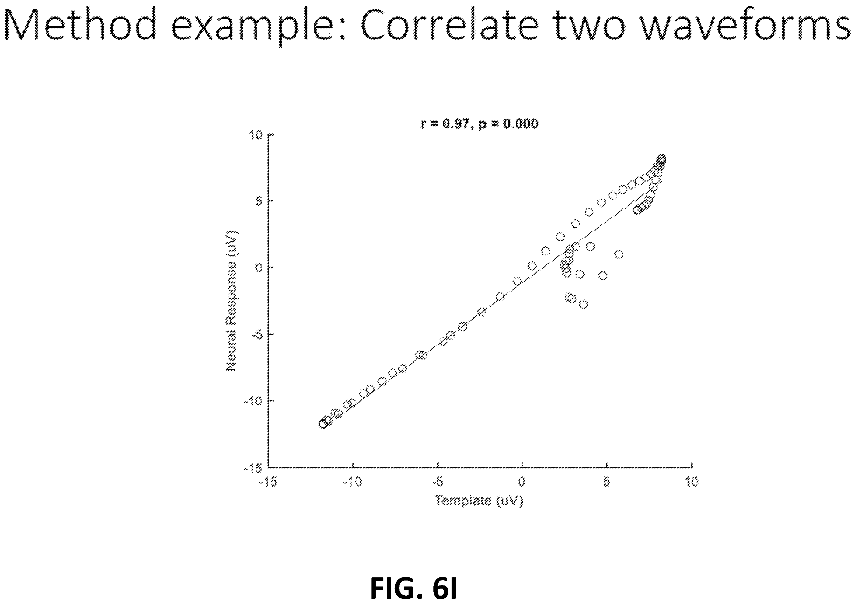

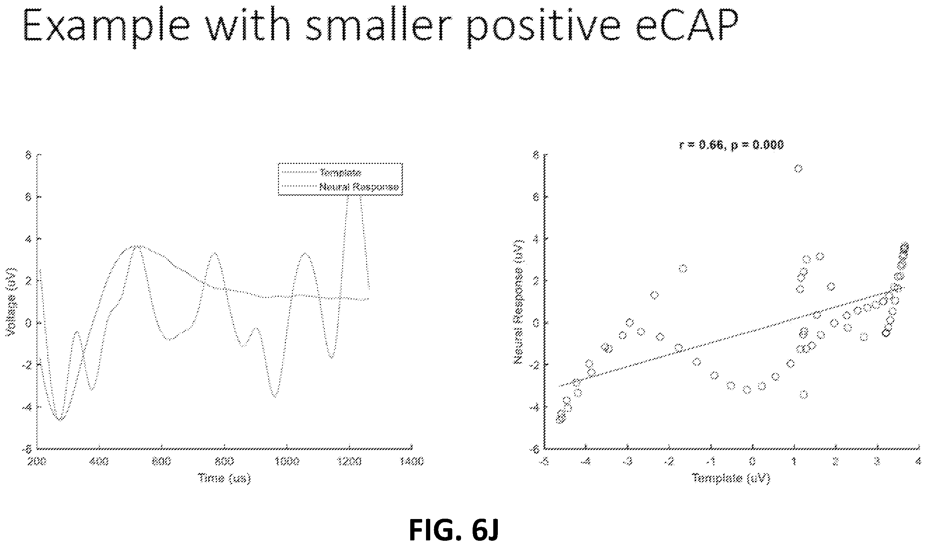

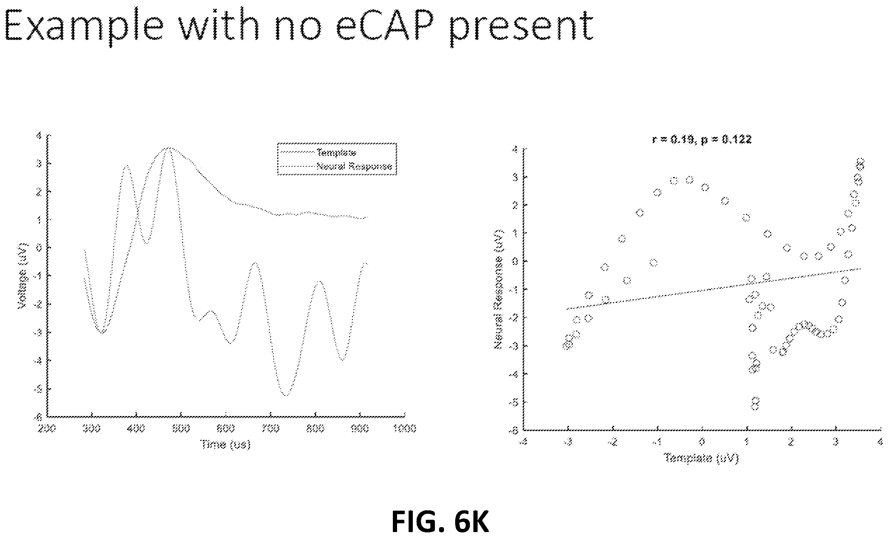

FIG. 6A illustrates an exemplary flowchart for a method of determining if an eCAP exists or not in a neural response. At 602, a template eCAP waveform is provided. FIG. 6B illustrates an example of a template eCAP waveform that was made from a neural response measured in a child with a GJB2 genetic mutation that causes hearing loss without impacting the auditory nerve. Therefore, the illustrated neural response is considered to be a template for a neural response. At 604, a recorded neural response obtained from a patient with a cochlear implant is received. As noted above, user defined stimuli can be sent through one electrode to stimulate the surrounding neurons. The neurons then respond and the electrical response can be recorded by a neighboring electrode, which comprises the neural response. At 606, the recorded neural response waveform is resampled. For example, the recorded neural response waveform may be up-sampled via cubic spline interpolation to create a smooth waveform at a higher effective sampling rate. FIG. 6C illustrates a comparison of the recorded neural response and the re-sampled neural response. At 608, the mean voltages recorded in the first 600 µ-sec are subtracted from the template and neural response waveforms. This is illustrated in FIG. 6D. At 610, the first negative (N1) peak and trailing positive peak (P2) are found in each waveform. This is also shown in FIG. 6D. At 612, the template waveform is scaled. As shown in FIG. 6E, the template waveform is scaled vertically (voltage) to match the N1 and P2 amplitudes from the neural response waveform. As shown in FIG. 6F, the template waveform is then scaled horizontally (time) to match the N1 and P2 latencies from the neural response waveform. At 614, the waveforms are then trimmed to the time period that overlaps after scaling. This is illustrated in FIG. 6G. At 616, the waveforms are resampled at the same time periods as each other with higher resolution sampling occurring before 600 µ-sec to place emphasis on the first part of the waveforms in the correlation analysis. This is illustrated in FIG. 6H. At 618, a correlation between the template and neural response waveforms is calculated. For example, the Pearson correlation between the template and neural response waveform may be calculated. This is illustrated in FIG. 6I. At 620, it is determined whether the correlation between the neural response and the eCAP template indicates that the neural response comprises an eCAP. For example, if the correlation is the Pearson correlation and the correlation value is larger than 0.6 and the estimated eCAP amplitude is greater than 5 uV (noise floor of the device), an eCAP is determined to be present. Otherwise, an eCAP is determined to not be present. FIG. 6J illustrates an example correlation where the neural response is an eCAP (correlation value greater than 0.6), and FIG. 6K illustrates an example correlation where the neural response is not an eCAP (less than 0.6 correlation value). It is to be appreciated that different methods of determining the correlation between the eCAP template and the neural response are contemplated within the scope of this disclosure as well as differing correlation values for deciding whether a neural response comprises an eCAP, or not.

In various implementations, the above-described methods determine whether a neural response comprises an eCAP. In some instances implementations of the method may be used in post-surgery software package for clinicians to optimize and personalize the cochlear implant settings for individual patients. The specific settings that could be informed include "Enable/disable an electrode" and "pulse phase duration." Furthermore, implementations of the method could be included in clinical and/or research software platforms as the disclosed technology reduces the advanced training that an audiologist would need to apply objective measures into clinical practice.

### II. ESTIMATING SURVIVAL OF AUDITORY NERVE FIBERS. This section II describes examples useful for understanding the invention.

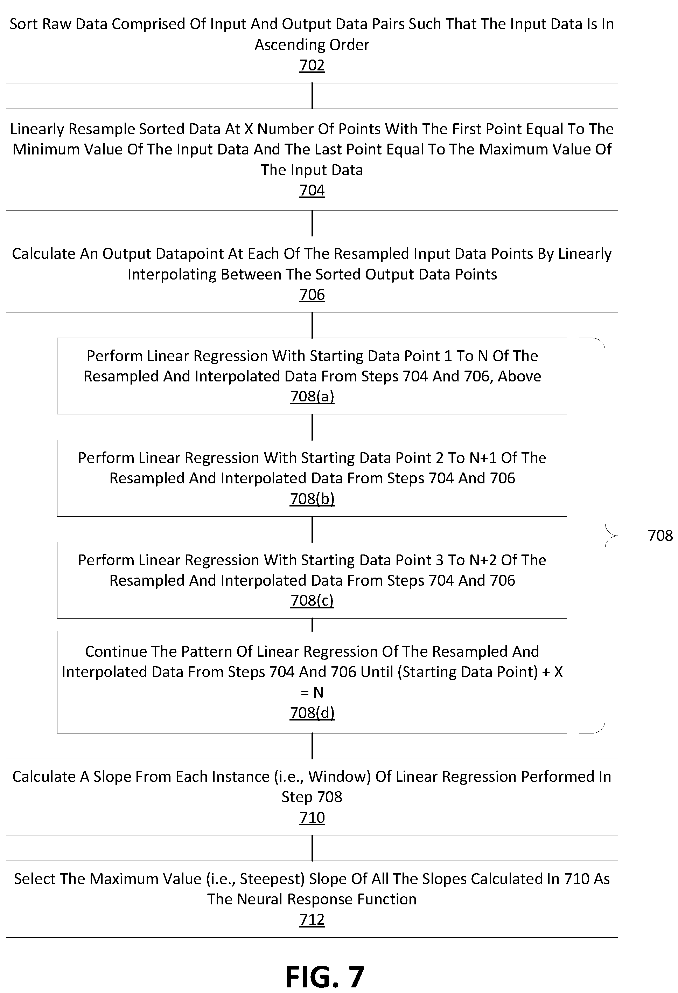

FIG. 7 illustrates an exemplary flowchart for a method of providing an estimate for the slope of a neural response function characterized by a series of stimulation levels (i.e. input data) and corresponding amplitude of neural responses (i.e. output data). These functions are often called amplitude growth functions (AGFs) or input/output (I/O) functions. For example, the raw data comprises x,y pairs of stimulation level (input data) and corresponding eCAP amplitudes (output data). The stimulation level can be specified in any units. Microamps or nanocoulombs are typical. The amplitudes of neural responses can be specified in any units. Microvolts is typical.

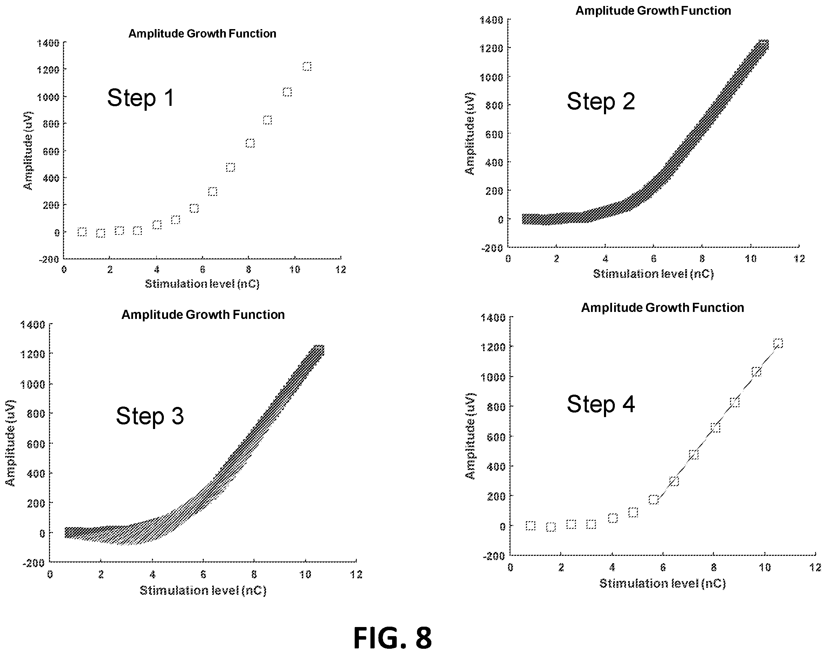

One embodiment of the method comprises 702, sorting raw data comprised of input and output data pairs such that the input data is in ascending order. At 704, the sorted input data from 702 is linearly resampled at X number of points with the first point equal to the minimum value of the input data and the last point equal to the maximum value of the input data. X can be any positive integer equal to or greater than the number of data points in the input data. A typical value for X is 100. This is illustrated in the panel labeled "Step 1" of FIG. 8. At 706, an output datapoint is calculated at each of the resampled input data points from 704 by linearly interpolating between the sorted output data points. This is illustrated in the panel labeled "Step 2" of FIG. 8. At 708, linear regression is performed on a moving window of N number of data points. N is any positive integer less than or equal to X. For example, at 708(a) linear regression is performed with starting data point 1 to N of the resampled and interpolated data from steps 704 and 706. At 708(b), linear regression is performed with starting data point 2 to N+1 of the resampled and interpolated data from steps 704 and 706. At 708(c), linear regression is performed with starting data point 3 to N+2 of the resampled and interpolated data from steps. At 708(d), this pattern of linear regression continues until (starting data point) + N = X. At 710, a slope is calculated from each instance (i.e., window) of linear regression in step 708. This is illustrated in the panel labeled "Step 3" of FIG. 8. Slope is defined as m in the linear regression equation y = mx+b. At 712, the maximum value (i.e., steepest) of all the slopes calculated in step 710 is selected as the slope of the neural response function. This is illustrated in the panel labeled "Step 4" of FIG. 8.

In some instances, the method of FIG. 7 comprises an adaptive and iterative algorithm that chooses the number of points (X and N) depending on the input and output data. It is also to be noted that a steeper slope correlates with a greater number of neuron survival in the cochlear nerve (i.e., greater values of slope indicate greater neural survival and/or function).

### A. Examples of Estimating Survival of Auditory Nerve Fibers

The following examples are set forth below to illustrate the methods and results according to the disclosed subject matter. These examples are not intended to be inclusive of all aspects of the subject matter disclosed herein, but rather to illustrate representative methods and results. These examples are not intended to exclude equivalents and variations of the present disclosure which are apparent to one skilled in the art.

Efforts have been made to ensure accuracy with respect to numbers (e.g., amounts, temperature, etc.) but some errors and deviations should be accounted for. Unless indicated otherwise, parts are parts by weight, temperature is in °C or is at ambient temperature, and pressure is at or near atmospheric. There are numerous variations and combinations of reaction conditions, e.g., component concentrations, temperatures, pressures and other reaction ranges and conditions that can be used to optimize the product purity and yield obtained from the described process.

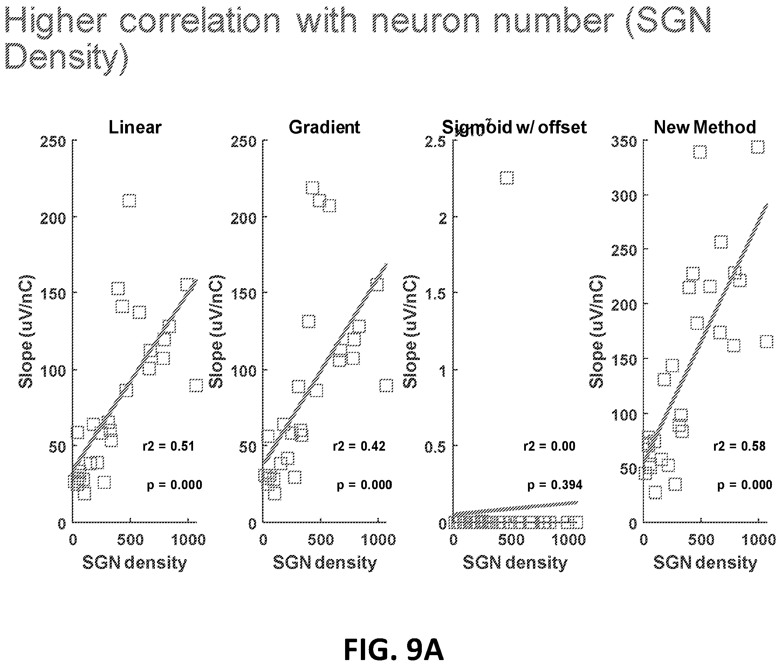

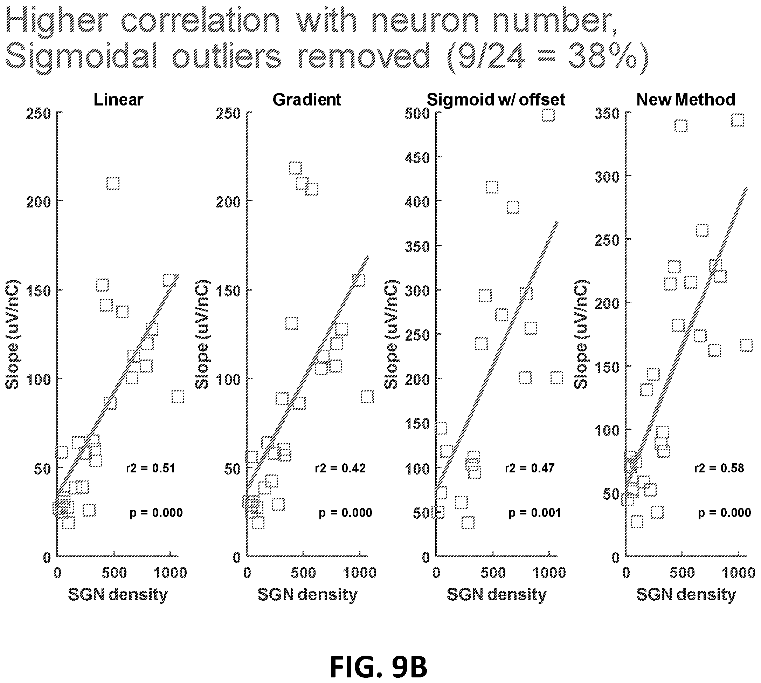

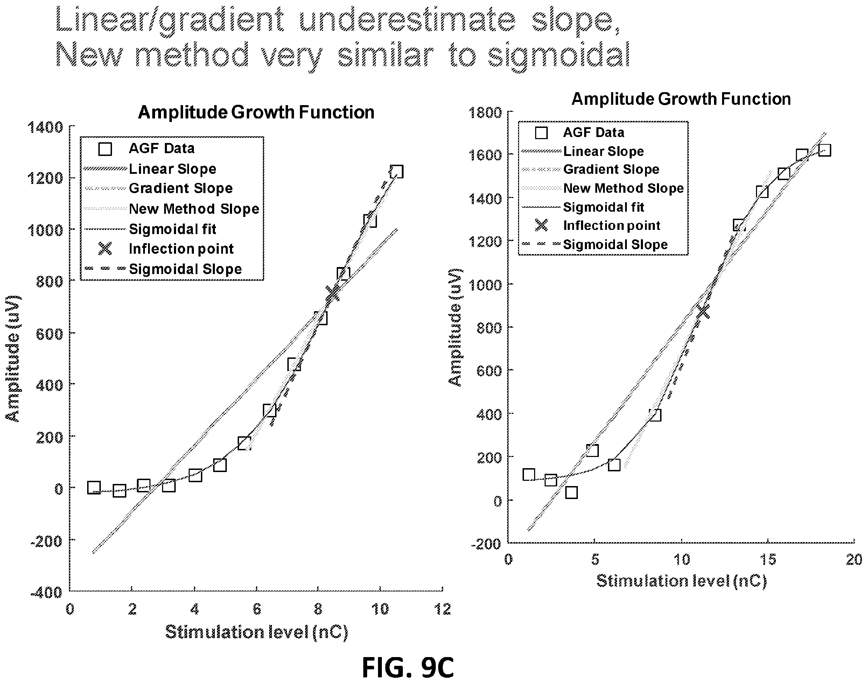

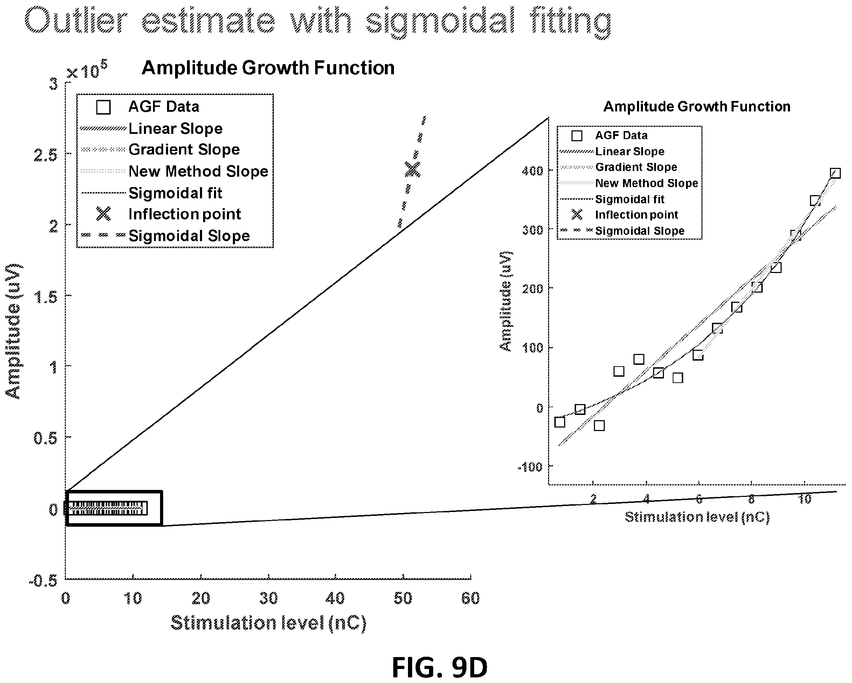

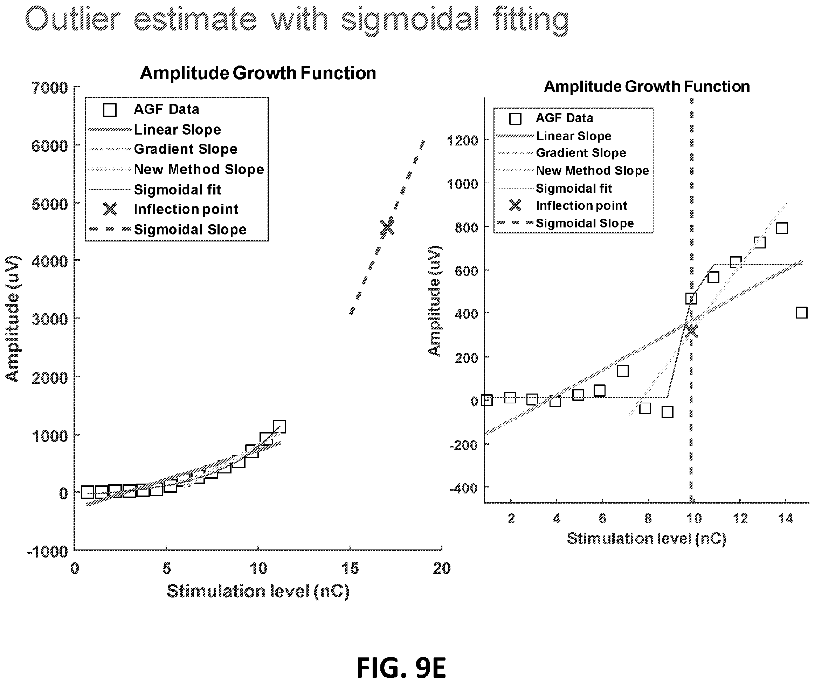

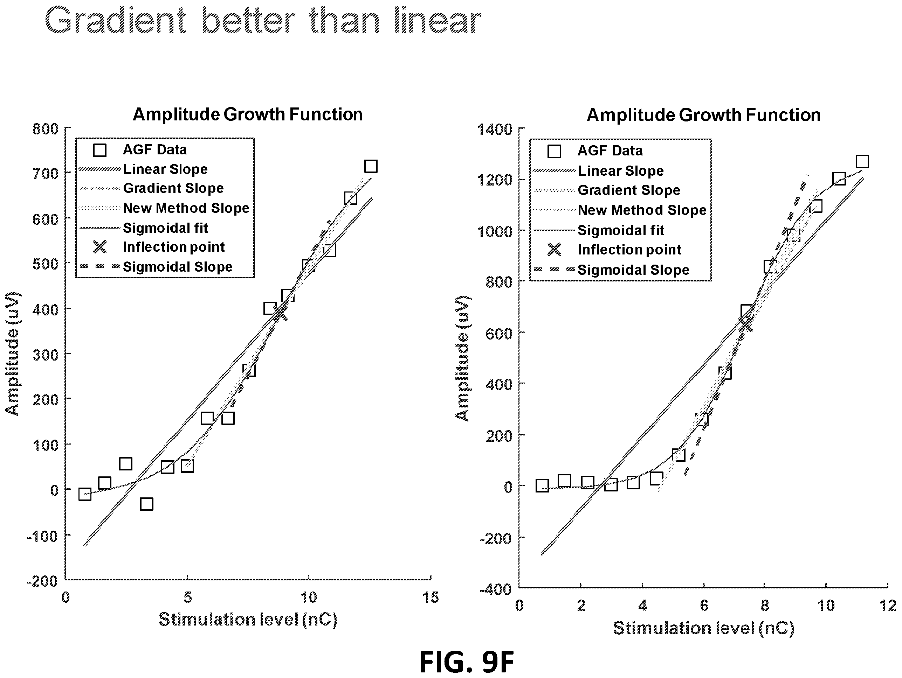

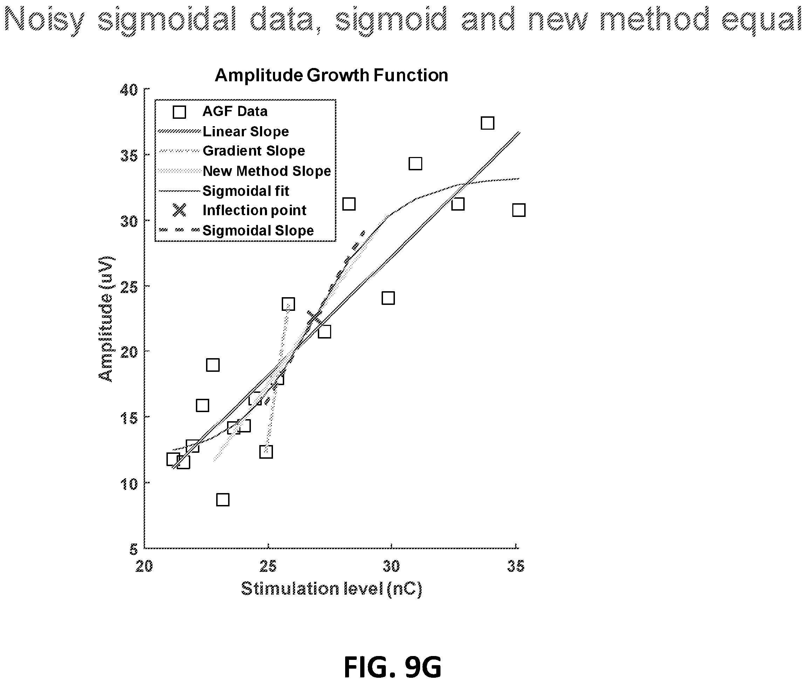

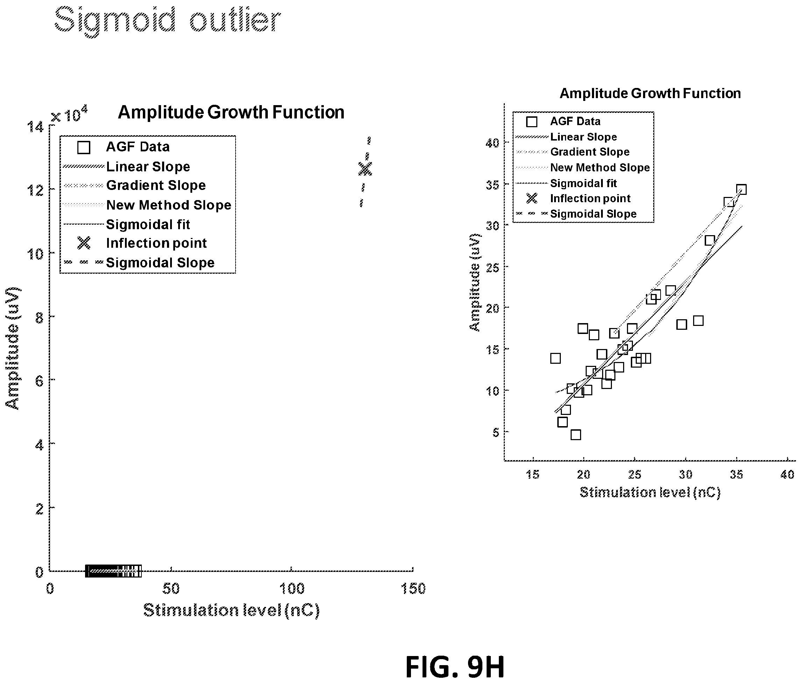

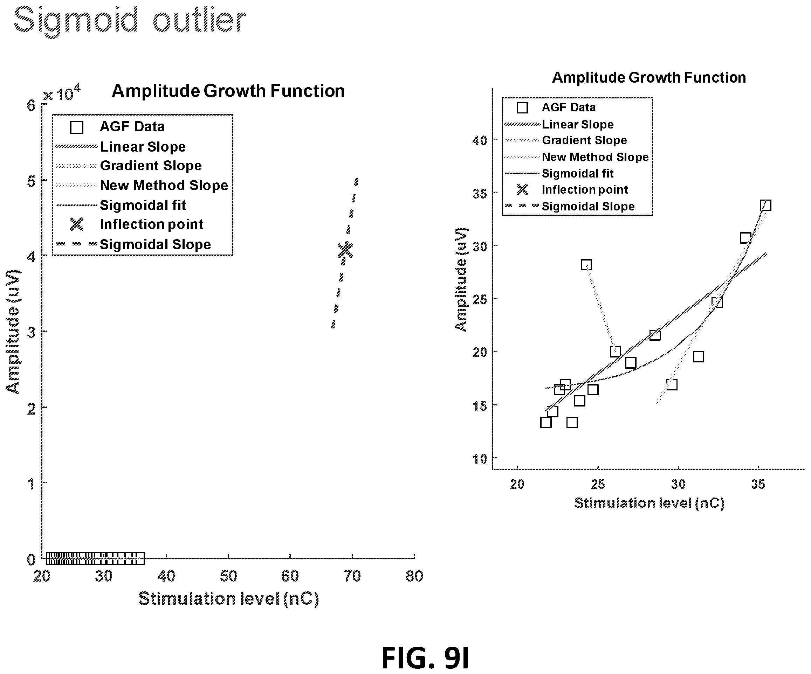

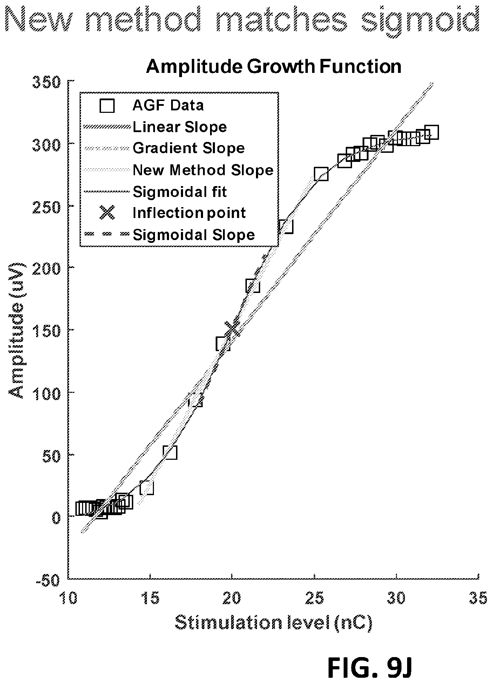

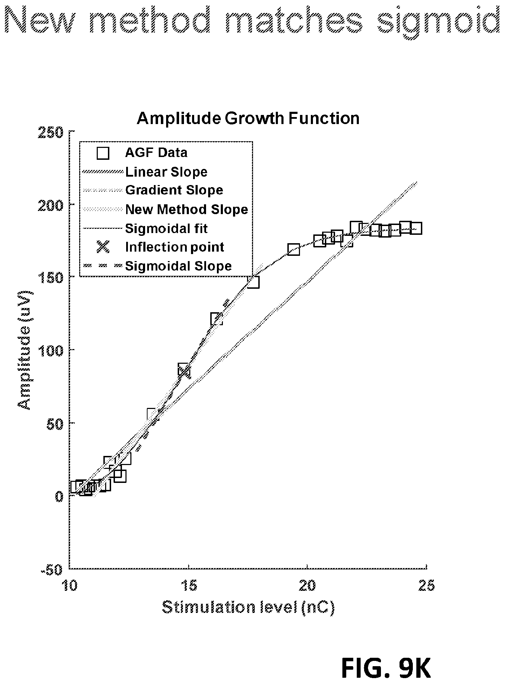

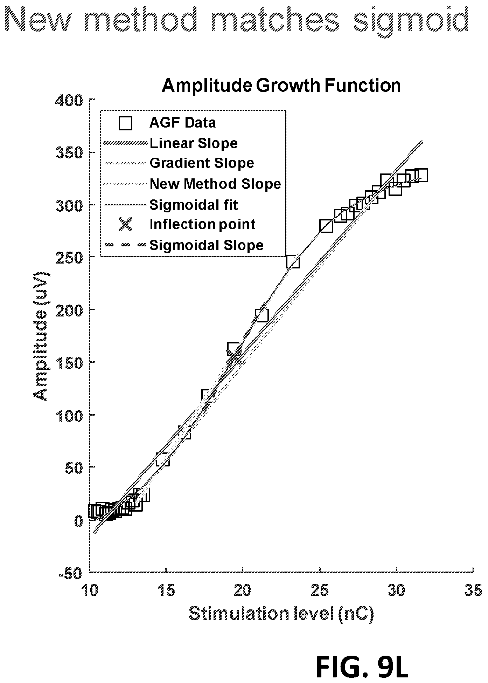

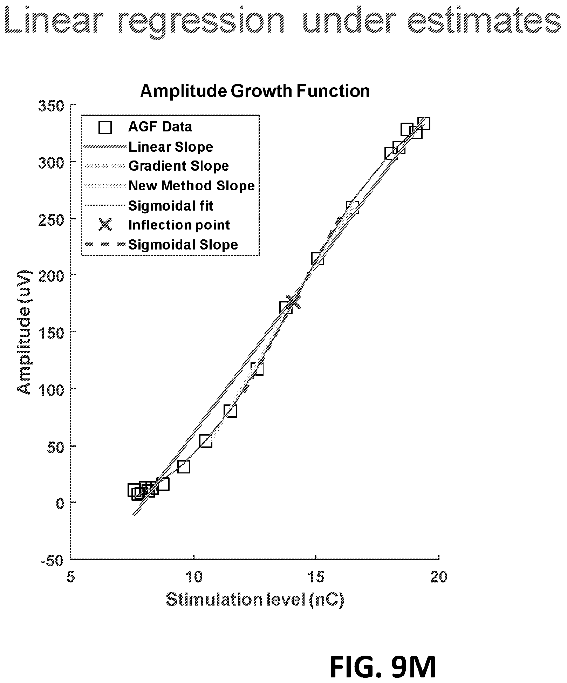

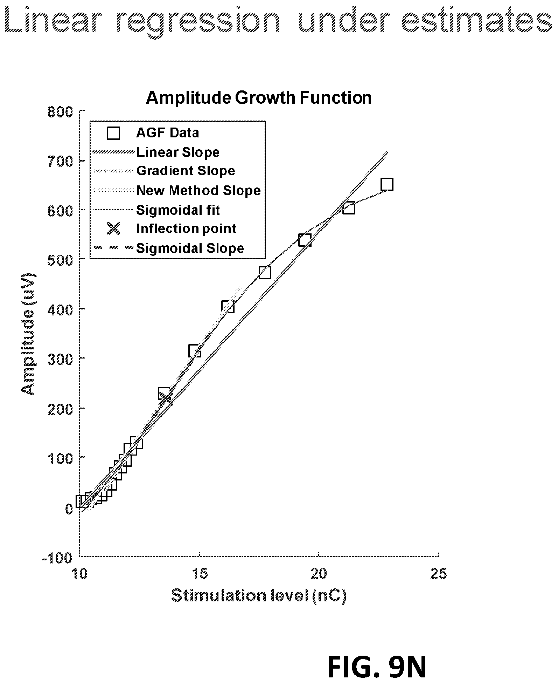

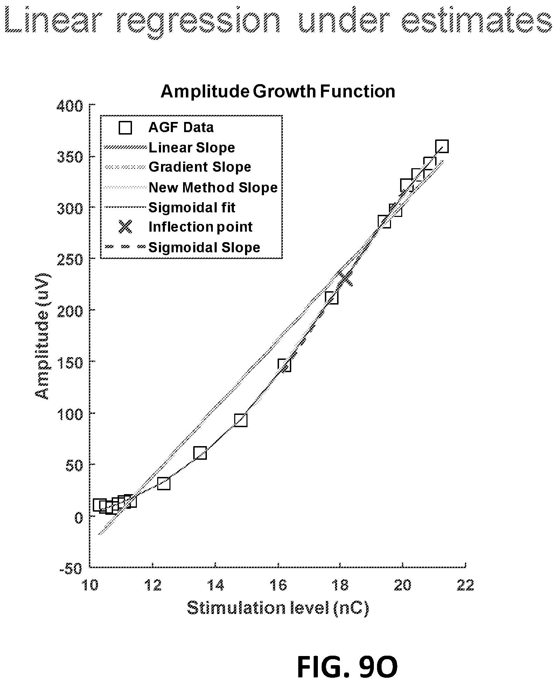

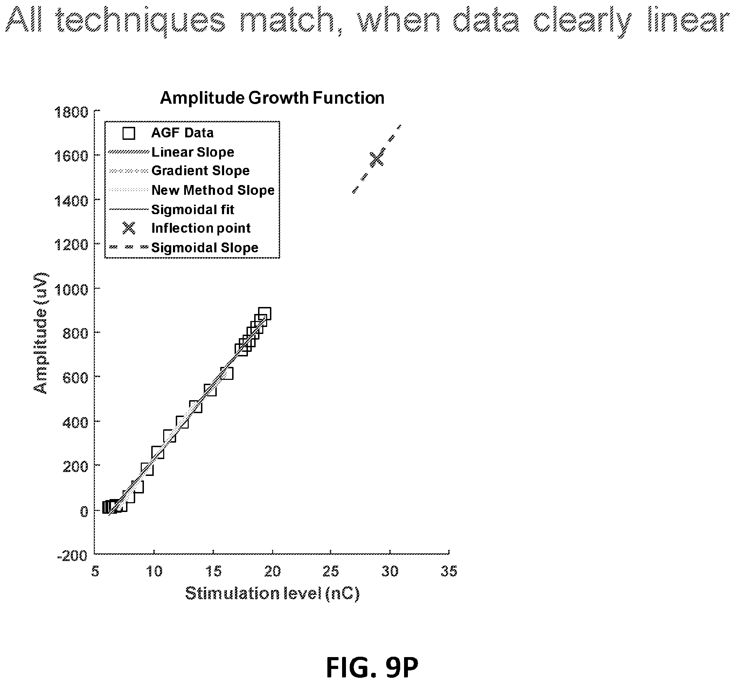

FIGS. 9A-9P illustrate comparisons of calculating the slope of the AGF data using the disclosed embodiments and conventional methods of calculating the slope, in both animals and humans. FIGS. 9A-9F are based on animal data. FIGS. 9G-9P are based on human data.

### B. Appendix to Estimating Survival of Auditory Nerve Fibers

Attached hereto and made a part hereof is Appendix A, "Prediction of the functional status of the cochlear nerve in individual cochlear implant users using machine learning and electrophysiological measures,". This Appendix A describes development of a cochlear nerve index in which the slope of the sigmoid curves (i.e. eCAP AGFs), as determined by the systems and methods described herein, is used as a factor in determination of the cochlear nerve index.

### C. Conclusion to Estimating Survival of Auditory Nerve Fibers

Advantages of the disclosed embodiments include 1) it works for all AGFs, which means that (a) It finds the same slope as the sigmoidal slope for animal and human AGFs that reach saturation; (b) it finds the linear portion of the AGF for AGF's that don't reach saturation; it works regardless of the number of data points in the AGF, including data points that are uniformly or non-uniformly distributed. In human patients, these are usually non-uniformly distributed which causes issues for other methods of calculating the slope of the raw data; (c) it works with noisy, raw (i.e. no preprocessing) AGFs. For example, in one exemplary test, the disclosed method gives very similar slope estimations for 28 animals whether or not the data points less than 100 uV (i.e., excluding outliers) are included or not; (d) it does not require user input. This is related to (c). For example, a threshold of 100uV to exclude data does not need to be provided. Given the thousands of human AGFs that are possible, it would be impossible to find a suitable threshold below which to exclude the nonlinear portion of the AGF. Therefore, this feature is especially advantageous from a clinical perspective, in which a slope estimation could be provided without the clinician making any decision on how to process the AGF (i.e. include/exclude data points); (e) it does not create unrealistic estimations of the slope. This is difficult to quantify, but there were no unrealistic, extreme values for the 28 AGFs from guinea pigs, 317 AGFs from adults, and 963 AGFs from children that were tested using the disclosed embodiments. In contrast, the sigmoidal slope with offset had 33 % unrealistic estimates (slope > 2000 uV/nC) for the guinea pig data. It was about 9% of the adult AGFs and 15% of the children AGFs.

Applications for the disclosed embodiments include clinical care for cochlear implant patients. Specifically, this includes 1) optimizing fine tuning of cochlear implant programming settings for individual patients, and 2) improving proprietary internal programming settings of cochlear implants

In current clinical practice, patients receive a cochlear implant with default settings. There is a very wide variety of speech recognition performance among cochlear implant patients. Some patients do really well with the default settings, some patients don't do well at all. After surgery, these parameters can be adjusted by the clinician by "trial and error." The clinician will modify some parameters and the patient will report back if they think they are able to hear better or worse. This current practice is inefficient, time-consuming, frustrating, and subjective. The disclosed embodiments allow clinicians to objectively estimate the function of the patient's nerves near each electrode location. Based on these results, they can make informed decisions on how to modify the programming settings. Below is an outline how a clinical visit would be conducted using the disclosed embodiments:
a. The hardware and software from the manufacturer of the device that the patient has implanted is used to collect a series of measurements to create an amplitude growth function (AGF). This is a standard feature of all manufacturers' available software and hardware.
b. The slope of the eCAP AGF is calculated using the disclosed embodiments. This could be implemented either (i) incorporated as a part of the manufacturer's software; (ii) as a stand-alone-software package. Each manufacturer allows the raw datapoints to be exported. So they could be imported into a separate software package to calculate the slope.
c. The slope of the eCAP AGF provides the clinician an estimate of how well the nerves function for the specific patient and can adjust the programming settings accordingly. These adjustments can include deactivating electrodes of the cochlear implant near nerves that do not function properly and/or passing more stimulation/information through regions with the best functioning nerves.
d. Repeat a-c for each electrode in the cochlear implant; resulting in
e. Optimized settings of the cochlear implant that help the patient hear better,

Generally, the internal workings of cochlear implants is proprietary and a "black box." However, in some instances outputs of the disclosed embodiments can be used as feedback to the cochlear implant to dynamically "tune" it according to a patient's specific neural responses.

### III. USE OF eCAP: DETERMINING EFFECTS OF ADVANCED AGE ON THE ELECTRODE-NEURON INTERFACE. This section III describes examples useful for understanding the invention.

A study was conducted with participants included 30 post-lingually deafened (i.e., lost hearing after twelve years of age) adult CI users. All participants were implanted with a Cochlear^{™} 135 Nucleus^{®} device (Cochlear Ltd., Sydney, NSW, Australia) with a full electrode insertion in the test ear. Twenty participants were implanted unilaterally and ten participants (A03, 137 A07, A08, A11, A19, A27, A34, A48, A49, and A50) were implanted bilaterally. For all participants, only one ear was tested for this study. For the bilateral CI users, the test ear was selected pseudo-randomly using a pseudorandom number generator.

The participants were separated by age at testing into two study groups with 15 participants in each group: younger (age ≤ 68 years) and older (age > 68 years). For the younger study group, the age at testing ranged from 48.4 to 67.6 years (mean: 60.3 years, SD: 5.9 years). For the older study group, the age at testing ranged from 69.0 to 83.2 years (mean: 75.8 years, SD: 4.6 years). Detailed demographic information of the study 145 participants is provided in Table 1, below.

**Table 1**

| Subject Number | Sex | Ear Tested | AAI (yrs) | AAT (yrs) | Internal Device and Electrode Array | Ebology of Hearing Loss | Electrodes Tested |
|---|---|---|---|---|---|---|---|
| Younger: | | | | | | | |
| A03 | M | L | 58.8 | 61.8 | CI512 | SHL | 3, 12, 21 |
| A07 | M | R | 43.3 | 52.7 | 24RE (CA) | Unknown | 3, 12, 21 |
| A08 | F | R | 54.4 | 67.6 | 24RE (CA) | Hereditary | 3,12, 21 |
| A17 | M | R | 56.2 | 62.5 | 24RE | Unknown | 6, 12, 21 |
| A19 | F | L | 54.6 | 55.4 | CI532 | Rubella | 4, 12, 21 |
| A20 | M | R | 60.3 | 62.7 | CI522 | Trauma | 6, 12, 21 |
| *A27* | F | R | 44.5 | 46.4 | CI422 | Unknown | 3, 12, 21 |
| A29 | F | R | 48.5 | 59.6 | 24RE (CA) | Hereditary | 3, 12, 21 |
| A30 | F | R | 64.9 | 65.6 | CI532 | Unknown | 3, 12, 21 |
| A40 | M | R | 59.0 | 59.5 | CI532 | Unknown | 3, 12, 21 |
| A47 | F | R | 59.1 | 66.9 | 24RE (CA) | Unknown | 3, 12, 21 |
| A48 | F | L | 57.3 | 59.7 | CI532 | Hereditary | 3, 12, 21 |
| A49 | M | L | 51.5 | 52.7 | CI532 | Unknown | 3, 12, 21 |
| A51 | M | R | 55.8 | 62.4 | 24RE (CA) | Unknown | 3, 12, 21 |
| A52 | M | L | 65.2 | 67.6 | CI532 | Tumor | 3, 12, 21 |

| Older: | | | | | | | |
|---|---|---|---|---|---|---|---|
| A06 | M | L | 60.7 | 69.0 | CI512 | Meniere's | 3, 12, 18 |
| A11 | M | R | 77.5 | 80.7 | 24RE (CA) | Noise | 3, 12, 21 |
| A25 | M | L | 74.9 | 83.2 | 24RE (CA) | Unknown | 3, 12, 21 |
| A34 | M | R | 68.7 | 70.4 | CI532 | Trauma | 3, 12, 21 |
| A35 | F | L | 72.4 | 76.6 | CI422 | Noise | 3, 12, 21 |
| A38 | M | L | 74.0 | 79.0 | CI422 | Ototoxicity | 3, 12, 21 |
| A41 | F | R | 73.3 | 79.8 | 24RE (CA) | Hereditary | 3, 12, 21 |
| A44 | M | L | 68.4 | 70.2 | CI532 | Noise | 3, 11, 20 |
| A45 | F | L | 67.5 | 79.4 | 24RE (CA) | Autoimmune | 3, 12. 21 |
| A46 | M | R | 76.2 | 78.3 | CI532 | None | 3, 12, 21 |
| A50 | M | R | 75.2 | 76.7 | CI522 | Noise | 3, 12, 21 |
| A54 | M | L | 69.3 | 69.7 | CI622 | Noise | 3, 12, 21 |
| A55 | M | R | 68.2 | 70.9 | CI532 | Unknown | 3, 12, 21 |
| A56 | F | L | 65.9 | 77.3 | 24RE (CA) | Noise | 6, 12, 21 |
| A57 | M | R | 73.7 | 76.5 | CI532 | Unknown | 3, 12, 21 |

| | | | | | | | |
|---|---|---|---|---|---|---|---|
| Demographic information of all subjects who participated in this study, listed by study group. Definitions: AAT, age at implantation; AAT, age at testing; 24RE (CA): Freedom Contour Advance electrode array; SHL, sudden hearing loss | | | | | | | |

All participants were tested for eCAP measures at three electrode locations across the electrode array, typically electrodes 3, 12, and 21 (see Table 1). All participants had a full electrode array insertion with a Cochlear^{®} Nucleus^{™} CI (Cochlear Ltd., Sydney, NSW, Australia), which means that electrodes 1 and 22 were placed near the base and the apex of the cochlea, respectively. Therefore, the three testing electrodes were referred to as the "basal", the "middle" and the "apical" electrode based on their relative locations along the electrode array.

The procedures for obtaining the eCAP in adult CI users were the same as those used in a previous study (Skidmore et al., 2021a - see Appendix A, attached hereto and made a part hereof). Briefly, eCAP measures were acquired using the Advanced Neural Response Telemetry function via the Custom Sound EP (v. 4.3 or 5.1) software interface (Cochlear Ltd, Sydney, NSW, Australia). The stimulus was a symmetric, cathodic-leading, biphasic pulse with an interphase gap of 7 µs and a pulse phase duration of 25 µs/phase. Other recording parameters included a 15 Hz probe rate, an amplifier gain of 50 dB, sampling delays between 98 and 122 µs, an effective sampling rate of 20 kHz, and 50 sweeps per averaged eCAP response. The stimulus was presented to individual CI electrodes in a monopolar-coupled configuration via a N6 sound processor that was connected to a programming pod.

The eCAP refractory recovery function (RRF) was obtained with two electrical pulses (marker pulse and probe pulse) using a modified template subtraction method (Miller et al., 2000). The masker pulse was presented at the participants' maximum comfortable level (i.e., C level), and the probe pulse was presented at 10 current levels (CLs) below C level. A series of eCAPs were recorded as the MPI was systematically increased from 400 µs to 10 ms. The top panels of FIG. 10 show traces recorded at different masker-probe intervals (MPIs) for electrical stimulations at electrode 3 in one younger adult (A08) and one older adult (A54).

The eCAP AGF was obtained using the forward-masking-paradigm (Brown et al., 1990). The masker pulse, which was always presented at 10 CLs higher than the probe pulse, was initially presented at C level and decreased by 1 CL for at least five measurements. This was followed by a systematic decrease in steps of 5 CLs until no eCAP response could be visually identified. The stimulation level was then subsequently increased in steps of 1 CL until at least five eCAPs were measured using this small step size. The top panels of FIG. 11 show eCAPs recorded at different stimulation levels at electrode 3 in one younger adult (A08) and one older adult (A54).

### A. Data Analysis

The eCAP amplitudes measured at different MPIs were normalized to the eCAP amplitude measured at 10 ms and plotted as a function of MPI to generate the eCAP RRF. An estimate of the absolute refractory period (i.e., *t*₀) was found using statistical modeling with the exponential decay function ${eCAP}_{N} = A \left(1-{e}^{- \left(\frac{MPI - {t}_{0}}{τ}\right)}\right)$ where *eCAP_{N}* is the normalized eCAP amplitude, *A* represents the maximum normalized eCAP amplitude, *MPI* is the masker probe interval in ms, and *τ* is an estimate of the relative refractory period. The absolute refractory period has been estimated using this equation in several previously published studies (e.g., Morsnowski et al., 2006; Botros & 199 Psarros, 2010; Wiemes et al., 2016; He et al., 2018). The bottom panels of FIG. 10 show the eCAP RRFs obtained from the eCAP recordings shown in the top panels, along with the fitted exponential decay functions used to estimate the absolute refractory period. No eCAPs were visually identified in the traces recorded for participant A54 at MPIs of 400 and 496 µs and so they were excluded from the eCAP RRF.

The eCAP AGF was generated by plotting the eCAP amplitudes (in µV) as a function of the corresponding stimulation level (in dB re 1 nanocoulomb [nC]). The bottom panels of FIG. 11 show the eCAP AGFs obtained from the eCAP recordings shown in the top panels, along with an estimate of the maximum slope of the eCAP AGF.

For each eCAP AGF, the maximum slope was estimated using the slope-fitting method described herein with reference to FIGS. 7, 8, and 9A-9P, above. Briefly, the 'window' method is a broadly applicable method that can robustly estimate the slope of the eCAP AGF for multiple patient populations and for various morphologies (linear and non-linear) of the eCAP AGF. This study included eCAP AGFs from three patient populations (children with CND, children with NSCNs, and adults) with various morphologies of the eCAP AGF. Therefore, the 'window' method was the most appropriate method for calculating the slope of each eCAP AGF.

The 'window' method was implemented by first resampling the original eCAP AGF (i.e., the recorded data points) at 11 data points in order to handle missing data points or non-uniformly sampled data in the original AGF. Then, a series of eight linear regression analyses were performed on sequential subsets of four data points (i.e., sliding window linear regression). Finally, the maximum slope was selected from among all subsets of data points (i.e., windows).

Building on the analytical models for quantifying the overall functional status of the CN using supervised machine learning techniques based on results of electrophysiological measures of the eCAP as described in Appendix A, a new analytical model is developed to estimate the quality of the ENI at individual electrode locations for the present study.

Just like in the original models described in Appendix A, the input/predictor variables of this new analytical model were eCAP parameters derived from the eCAP RRF and eCAP AGF. These parameters included *t*0, the eCAP threshold, the slope of the eCAP AGF, and the N1 latency of the eCAP with the maximum amplitude. However, the original models combined these four eCAP parameters measured at three electrode locations (i.e., 12 input variables) to predict the overall functional status of the CN (i.e., 1 output variable per subject). In contrast, the new analytical model was applied at each electrode location (i.e., four input variables and 1 output variable per electrode tested). Specifically, the four eCAP parameters recorded at a single electrode were combined to predict the quality of the ENI at that specific electrode location. The output for the electrode-specific model was a value between 0 and 100, where 0 and 100 represented the poorest and best ENI across all participants and electrodes, respectively. This electrode-specific number between 0 and 100 was defined as the local ENI index.

The training dataset for training the model included eCAP measures from 23 children with CND and 29 children with NSCNs recorded at three electrode locations. This is the same dataset that was used to train the original models and is further described in Appendix A. In the present study, the eCAP parameters in the training dataset were grouped together independent of electrode position. This was a modification from the original models in order to create a model that was applicable for any electrode location tested, and not just the electrode locations included in the training dataset. In other words, the grouping of the training data together eliminated any bias in the new model parameters that could be present due to the electrode location at which the eCAPs were measured.

Before finding the model parameters, each eCAP parameter was standardized across all participants to eliminate any model bias due to differences in scale between the eCAP parameters. Specifically, each eCAP parameter measured for each participant was standardized according to $x = \frac{x ′ - μ}{σ}$ where ***x*** was a vector containing the normalized value for the eCAP parameter, ***x'** was* a vector containing the non-normalized value for the eCAP parameter, and *µ* and *σ* were the mean and standard deviation of ***x'*,** respectively.

The four standardized eCAP parameters were then used to find the model parameters (i.e., coefficients) of a linear regression model that separated the quality of the local ENI between the children with CND and the children with NSCNs. Specifically, the four standardized eCAP parameters were the input variables (*x*₁, *x*₂, *x*₃, *x*₄) and the output variable (y) was determined by patient population, where y=0 for children with CND and y=1 for children with NSCNs. The model parameters (*β*₀, *β*₁, *β*₂, *β*₃, *β*₄) were found according to ${min}_{β , {β}_{0}} \left[\frac{1}{N}{\sum }_{i = 1}^{N} {\left({β}^{T}{x}_{i}+{β}_{0}-{y}_{i}\right)}^{2}\right]$ where ***β**=*[*β*₁ *β*₂ *β*₃ *β*₄]*, **x***=[*x*₁ *x*₂ *x*₃ *x*₄]*, β*₀ was the model intercept term, *τ* represents the vector transpose, and *N* = 156 (52 participants x 3 electrodes) was the number of observations in the training data set.

In the original models, three different machine learning algorithms (linear 273 regression, support vector machine regression, and logistic regression) were used to create estimates of the functional status of the CN (i.e., CN indices) for individual CI patients. Linear regression was chosen for the creation of the model in the present study because linear regression produced CN indices that had the highest correlation with speech perception scores in adult CI patients among the three machine learning algorithms (*see* Appendix A).

### B. Model Validation

The model was validated with eCAP results from the 18 children with NSCNs (Participants: S1-S18) and the 18 children with CND and measurable eCAPs (Participants: CND2-CND18 and CND23) who were included in the study reported in He et al. (2018). Details regarding the testing procedures, electrodes tested, eCAP results, and participants' demographic information are provided He et al. (2018). Briefly, eCAP measures were recorded at seven electrodes (typically electrodes 3, 6, 9, 12, 15, 18, and 21) for children with NSCNs. The number and location of electrodes tested varied considerably in children with CND due to variations in the number of electrodes with measurable eCAPs among this patient population. The electrodes tested for each participant are listed in Table 1 of He et al. (2018).

The recorded eCAP parameters from each electrode for each participant in the validation dataset were standardized using the means and standard deviations calculated with the training data according to Equation 2 prior to model prediction. The standardized eCAP data from each participant were then mapped through the model function to obtain a predicted output variable (*yₚ*) for each participant according to ${y}_{p} = {β}^{T} x + {β}_{0}$
where ***β**=*[*β*1 *β*2 *β*3 *β*4], ***x***=[*x*1 *x*2 *x*3 *x*4], *β*0 was the model intercept term as before. Finally, the local ENI index was calculated for each participant and electrode by scaling the output variable into the interval [0, 100] according to $Local ENI index = 100 ∗ \frac{{y}_{p} - {y}_{min}}{{y}_{max} - {y}_{min}}$
where *yₘᵢₙ* and *yₘₐₓ* were the minimum and maximum predicted output variables from the training dataset, respectively.

### C. Model Application

The validated model was then used to test the study hypotheses with the eCAP data from the adult study participants. As before with the validation dataset, the eCAP data were standardized according to Equation 2, and the local ENI index was calculated according to Equations 4 and 5. This process was repeated for each electrode listed in Table 1 resulting in a local ENI index corresponding to each electrode tested for each participant.

### D. Statistical Analysis

All statistical modeling and analysis for this study was performed using MATLAB (v. 2019b) software (Mathworks Inc., Natick, MA, USA). The trust-region-reflective algorithm was used to estimate parameters of the mathematical functions used in statistical modeling. Each eCAP parameter was compared between children with CND and children with NSCNs in the training dataset with an unpaired, two-sample Welch's t-test. Welch's t-test was used because of unequal variances in the eCAP parameters between those two patient populations. As an initial comparison between children with CND and children with NSCNs in the validation dataset, an one-tailed, unpaired, two-sample t-test was used to compare the local ENI indices between these two groups, independent of the participant number and electrode location. For the adult study participants, the effects of study group and electrode location on each eCAP parameter and on local ENI index were assessed using generalized linear mixed effects models (GLMMs), with study group and electrode location as fixed effects and participant as a random effect. Pairwise comparisons of significant effects were evaluated using Tukey's honest significant difference (HSD). The effect of advanced age on the quality of local ENI was quantified with the slope obtained by linear regression with local ENI index as the dependent variable and age at testing as the independent variable.

### E. Results

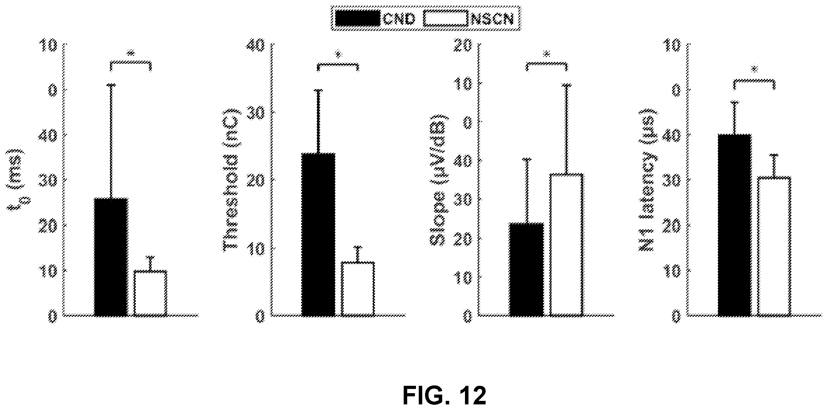

The means and standard deviations of eCAP parameters used in the model from both participant groups in the training dataset are shown in FIG. 12. As observed in the figure, the CND group had larger *t*₀ and eCAP threshold values, smaller slopes of the eCAP AGF, and longer N1 latencies than the NSCN group. Results from unpaired two-sample Welch's t-tests confirm this result and showed significant differences between the two groups for all four eCAP parameters [*t*₀: t(72.7)=5.3, p<0.001; threshold: t(77.7)=14.0, p<0.001; slope: t(165.7)=-5.1, p<0.001; N1 latency: t(119.6)=9.3, p<0.001].

The coefficients for each model parameter in the predictive model are provided in Table 2. When excluding the model intercept term (*β*₀), the coefficient *β*₂ had a magnitude greater than two times the magnitude of any of the other coefficients (*β*₁, *β*₃, or *β*₄).

**Table 2.**

| eCAP parameter | Model parameter | Model coefficient |
|---|---|---|
| | *β*₀ | 0.571 |
| *t*₀ | *β*₁ | -0.041 |
| Threshold | *β*₂ | -0.287 |
| Slope | *β*₃ | 0.038 |
| N1 latency | *β*₄ | -0.110 |

| | | |
|---|---|---|
| Coefficients for each model parameter in the predictive model listed by corresponding eCAP parameter | | |

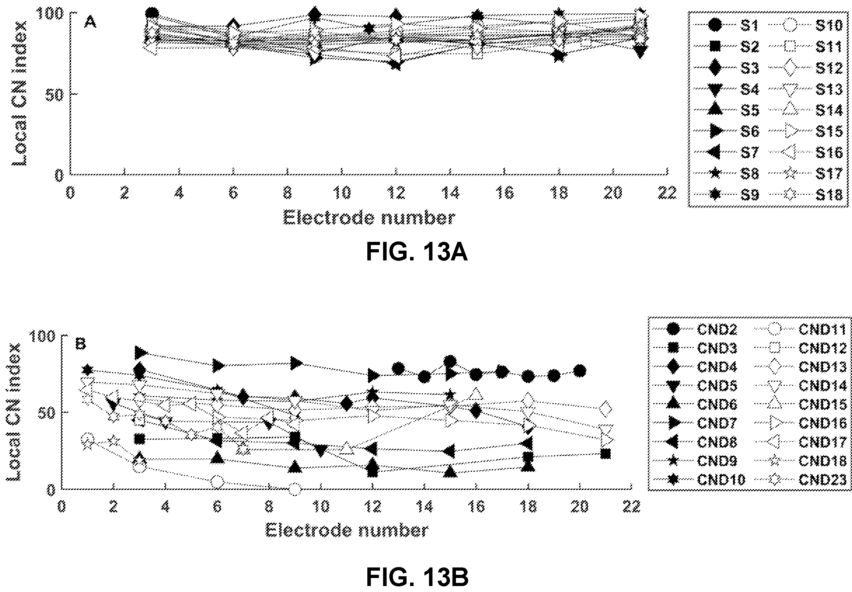

The local ENI index at each electrode tested for each participant in the validation dataset is shown in FIGS. 13A and 13B. It can be observed that the children with NSCNs generally had larger local ENI indices than children with CND at all seven electrode locations tested. Results of a one-tailed, unpaired, two-sample t-test that compared all of the local ENI indices between these two groups confirmed this observation [t(217)=19.1, p<0.001]. The children with NSCNs also had much smaller variation in local ENI indices across participants and electrode locations than the CND group. This is shown by the flat lines close to each other in FIG. 13A. On average, the local ENI index at the most apical electrode tested was greater than the local ENI index at the most basal electrode by only 0.94 (SD 6.4) in children with NSCNs.

In contrast, there was much more variability in local ENI indices across participants and electrodes for the children with CND. Specifically, some children had large local ENI indices at all electrodes tested that were within the range of the children with NSCNs (e.g., CND2 and CND7), while other children had very small local ENI indices (e.g., CND6 358 and CND11). There was also a general trend of decreasing local ENI index as the electrode location moved from the base to the apex of the cochlea. For the children with CND, 15 out of 17 (88%) participants had a smaller local ENI index at the most apical electrode tested than at the most basal electrode location. On average, the local ENI index at the most apical electrode tested was smaller than the local ENI index at the most basal electrode by 14.9 (SD 17.6). Participant CND12 was not included in these calculations of the change in local ENI index over electrode locations because only one electrode had a measurable eCAP.

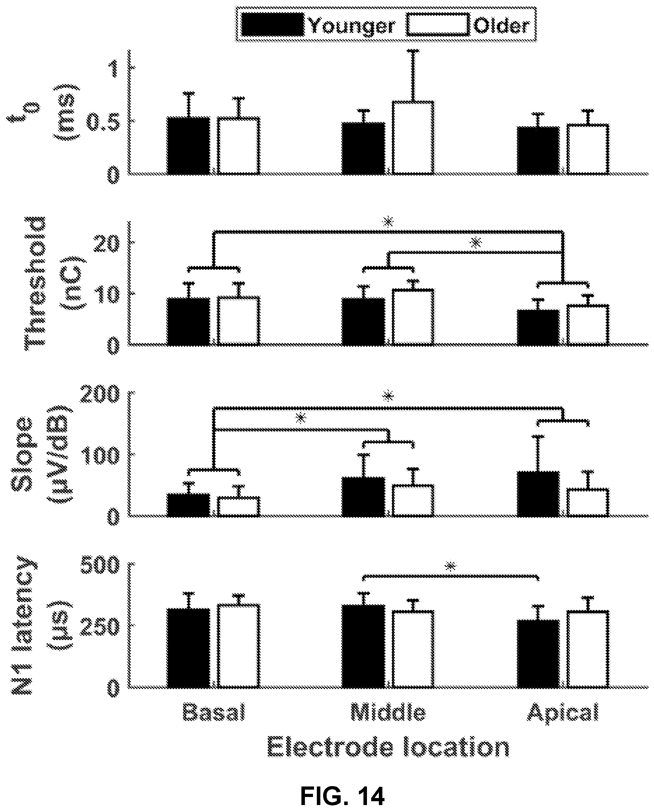

The means and standard deviations of eCAP parameters used for calculating local ENI indices for the adult study groups recorded at three electrode locations are shown in FIG. 14. As observed in the figure, the older adults on average had higher *t*0 and eCAP threshold values and smaller slopes of the eCAP AGF at all three electrode locations. However, results from the GLMMs indicated that there were no significant group differences for any of the eCAP parameters tested [*t*0: F(1,84)=1.52, p=0.221; eCAP threshold: F(1,84)=2.91, p=0.09; slope of eCAP AGF: F(1,84)=2.23, p=0.139; N1 latency: F(1,84)=0.68, p=0.412].

Another trend observed in FIG. 14 is that both study groups have lower eCAP thresholds and greater slopes at the apical electrode location than at the basal and middle electrode locations. Results from the GLMMs confirm an overall electrode effect on eCAP threshold [F(2,84)=13.58, p<0.001] and slope [F(2,84)=10.19, p<0.001]. There was also a significant effect of electrode location on N1 latency [F(2,84)=5.59, p=0.005], as well as a significant interaction between study group and electrode location [F(2,84)=3.37, p=0.039]. No other interactions were significant for any of the other eCAP parameters (F(2,84)≤2.15, p≥0.123). There was also not a significant effect of electrode location on *t*0 [F(2,84)=2.93, p=0.059]. The results from the GLMMs for each eCAP parameter, as well as significant post hoc comparisons, are provided in Table 3.

**Table 3**

| | Main effects | | Interaction effect |
|---|---|---|---|
| | Group | Electrode | Group x Electrode |
| t₀ | F_{(1.84)}=1.52, p=0.221 | F_{(2,84)}=2.93, p=0.059 | F_{(2,84)}=2.15, p=0.123 |
| eCAP threshold | F_{(1,84)}=2.91, p=0.091 | F_{(2,84)}=13.56, p<0.001 | F_{(2,84)}=0.96, p=0.378 |
| | | B>A, p=0.008 | |
| | | M>A, p=0.002 | |
| Slope of eCAP AGF | F_{(1.84)}=2.23, p=0.139 | F_{(2,84)}=10.19, p<0.001 | F_{(2,84)}=1.90, p=0.157 |
| | | B<M, p=0.032 | |
| | | B<A, p=0.021 | |
| N1 latency | F_{(1.84)}=0.68, p=0.412 | F_{(2,84)}=5.59, p=0.005 | F_{(2,84)}=3.37, p=0.039 |
| | | M>A, p=0.026^{#} | |
| Local ENI index | F_{(1.84)}=4.05, p=0.047 | F_{(2,84)}=1.56, p=0.216 | F_{(2,84)}=0.32, p=0.724 |

| | | | |
|---|---|---|---|
| Results from GLMMs and significant post hoc comparisons for each eCAP parameter and the local ENI index. GLMM: generalized linear mixed-effects model; eCAP: electrically-evoked Compound Action Potential; ENI: electrode-neuron interface; AGF: amplitude growth function; B: basal electrode location; M: middle electrode location; A: apical electrode location; #: post hoc comparisons are for the younger study group due to a significant interaction effect and nonsignificant post hoc comparisons for the older study group. | | | |

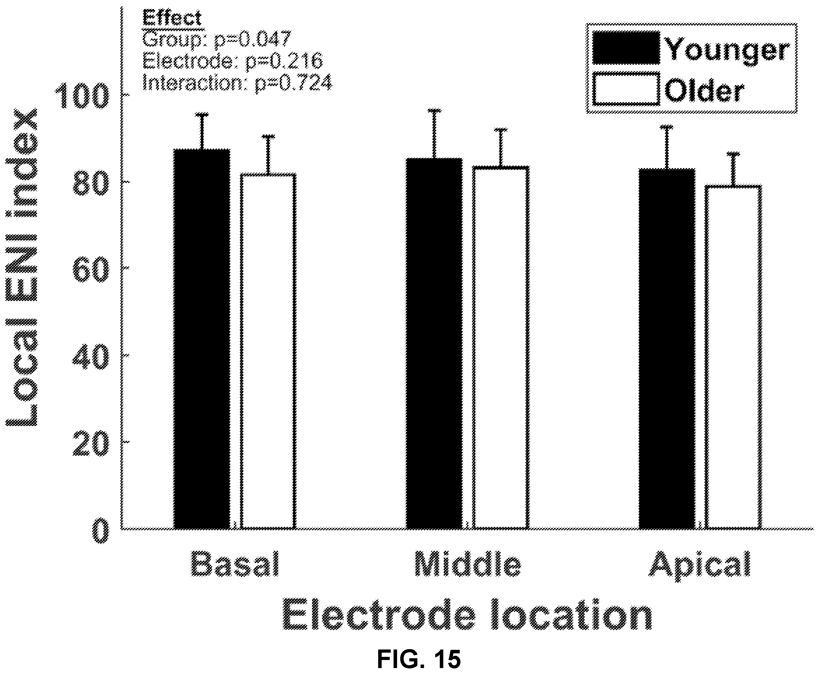

The means and standard deviations of the local ENI index calculated for both study groups at three electrode locations are shown in FIG. 15. As observed in the figure, the older adults on average had smaller local ENI indices at all three electrode locations. Results from the GLMMs confirmed this observation and indicated a significant group effect [F(1,84)=4.05, p=0.047]. There was not a significant effect of electrode location [F(2,84)=1.56, p=0.216] or a significant interaction [F(2,84)=0.32, p=0.724].

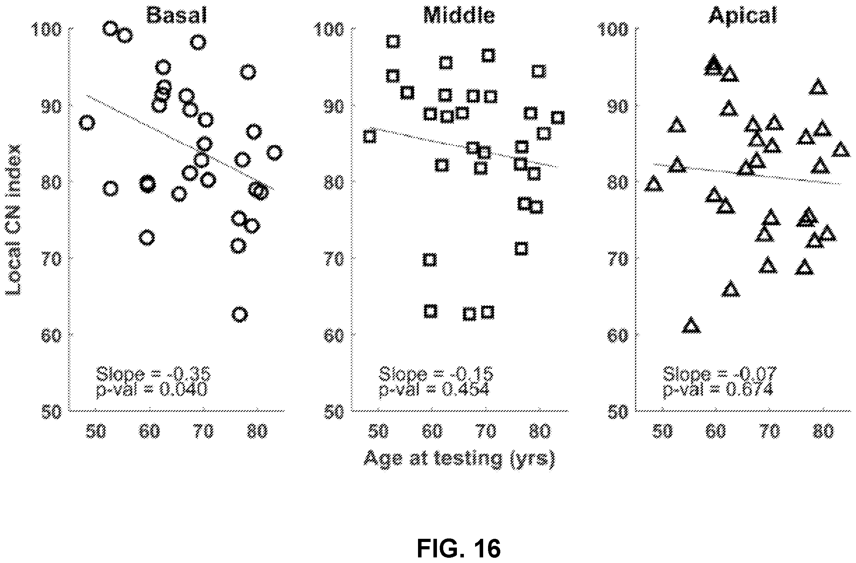

FIG. 16 shows the results of local ENI indices as a function of age at testing calculated at three electrode locations for all adults who participated in the study. Results of linear regression analyses are also included in each panel. Overall, the slope of the regression line ranged from -0.07 to -0.35, indicating a trend of decreasing local ENI index with increasing age. Also, the magnitude of the slope of the regression line decreased as the stimulating electrode moved from the base to the apex of the cochlea, indicating that the greatest effect of age on local ENI index occurred in the basal region of the cochlea. Results from the linear regression analyses indicated that the slope of the regression line was significantly different from zero for the electrode located in the basal region (p=0.040), but not for the electrodes located in the middle and apical regions of the cochlea (p=0.454 and p = 0.674, respectively). The significant slope of -0.35 at the basal electrode implies a decrease of 3.5% in the quality of the ENI in the basal region of the cochlea per decade of increased age.

### F. Discussion of Determining Effects of Advanced Age on the Electrode-Neuron Interface

The relative magnitude of standardized regression coefficients can be used as a measure of the importance of each input variable in predicting the output variable (Mehmood et al., 2010). Therefore, the magnitudes of the model parameters that scale the eCAP parameters (i.e., *β*₁-*β*₄) represent the relative importance of each eCAP parameter in creating the local ENI index. As seen in Table 2, the regression coefficient for the eCAP threshold (i.e., *β*₂) had the highest magnitude among all other regression coefficients (excluding the offset term *β*₀) by at least double. This suggests that the eCAP 421 threshold is an important indicator for the quality of the ENI.

This expectation is supported by a recent study with CI patients that showed linear increases in eCAP thresholds with increased electrode distance from the mid-modiolar axis (MMA) and the medial wall (Schvartz-Leyzac et al., 2020). In the study, Schvartz-Leyzac et al. (2020) presented a linear model with the electrode-to-MMA distance which suggested that the eCAP threshold increased by 1.47 dB for every 1-mm increase in MMA distance. Similarly, Long et al. (2014) presented a linear model that suggested an average increase of 11 dB in psychophysical detection threshold for every 1-mm increase in electrode-to-modiolus distance. Cohen et al. (2001) also showed increased psychophysical detection thresholds with increased electrode-to-modiolus distance in a study with three CI patients. Collectively, these studies suggest that eCAP threshold is an important indicator of the quality of the ENI, specifically related to the position of the electrode in relation to the targeted CN fibers.

The local ENI index was validated with eCAP results from children with NSCNs and children with CND. It was expected that children with NSCNs would have greater local ENI indices than children with CND. The validation results followed that expectation. Specifically, there was a significant group difference in local ENI indices. Moreover, a much larger range of local ENI indices were generated for children with CND compared to children with NSCNs (FIG. 13). The local ENI index for children with CND ranged from very poor to high quality. This result agrees with studies that have reported outcomes for children with CND that range from no awareness of environmental sounds to open-set speech (Young et al., 2012; Vincenti et al., 2014; Birman et al., 2016; Han et al., 2019). Additionally, there was a general trend for children with CND to have smaller local ENI indices in the apical region than in the basal region on the cochlea. This general trend is expected because the cochlea forms in a base-to-apex sequence and CND is likely caused by prematurely halted inner ear development during embryogenesis (Jackler et 448 al., 1987). Therefore, the quality of the ENI should generally decrease along the length of the cochlea, with the level of damage related to when the inner ear stopped developing. This expectation is also supported by the result that the percentage of electrodes with recordable eCAP responses declined in children with CND as the testing electrode moved from the base to the apex of the cochlea (He et al., 2018). In contrast, eCAPs were recorded at all electrode locations for all children with NSCNs. Therefore, CI patients with fewer electrodes with recordable eCAPs would also be expected to have quickly declining quality of ENI as the test electrode moved toward more apical regions of the cochlea. This expectation is supported by the present results in which the children with CND who had few electrodes with recordable eCAPs also had sharp declines in local ENI index as the electrode tested moved more apically (FIG. 13). In contrast, the children with NSCNs and the children with CND who had recordable eCAPs along the length of the electrode array also had local ENI indices that did not change as dramatically with the change in electrode tested.

### G. Effect of Advanced Age on Individual eCAP Parameters and Local ENI Index

Results show that there was not a significant difference in absolute refractory periods, eCAP thresholds, slopes of eCAP AGFs, or N1 latencies between adults younger than 68 years and adults older than 68 years (Table 3). These results appear to be inconsistent with results that have demonstrated that children and young adults have steeper eCAP AGF linear slopes and higher eCAP thresholds than older individuals (Hughes et al., 2001; Cafarelli Dees et al., 2005; Brown et al., 2010; Jahn & Arenberg, 2020). However, these differences in age groups may be reflective of differences in etiology between patients who are pre-lingually deafened vs post-lingually deafened (Bodmer et al., 2007; Brown et al., 2010; Zarowski et al., 2020).

Interestingly, individual eCAP parameters by themselves have not been consistently shown to be correlated with speech perception scores. This non-significant association has been reported for the eCAP threshold (Brown et al., 1990; Cosetti et al., 2010; Franck & Norton, 2001; Kiefer et al., 2001; Turner et al., 2002; El Shennawy et al., 2015), the maximum eCAP amplitude (Brown et al., 1990), and slope of the eCAP AGF (Brown et al., 1990; Gantz et al., 1994; Franck & Norton, 2001; Turner et al., 2002; Kim et al., 2010). However, a significant group effect is observed when these eCAP parameters are combined by the predictive model to form the local ENI index (Table 3). These results suggest that individual eCAP parameters may not have sufficient predictive power by themselves, but do in combination.

Results also show that there was a significant effect of advanced age on the local ENI index in the basal region, but not in the middle and apical regions of the cochlea (FIG. 16). This result agrees with a recent histological study with human temporal bones in which the loss of CN fibers with age was significantly higher in the basal region than in the apical region of the cochlea (Wu et al., 2019). A different study with human temporal bones showed slightly more age-related degeneration of cell bodies of SGNs in the basal region than in the apical region of the cochlea (Makary et al., 2011). However, the difference in rates of degeneration of SGN cell bodies between regions of the cochlea did not reach a level of significance. This apparent discrepancy may be explained by the observation that CN fibers are lost almost three times faster than SGN cell bodies or inner hair cells (Wu et al., 2019).

### H. Clinical Application

Deactivating electrodes with poor ENIs from the clinical programming map may improve speech perception for CI patients by reducing interactions between channels and only stimulating highly-functional regions of the cochlea. Indeed, several studies have shown improvements in speech perception when excluding electrodes based on CT-imaging techniques (Noble et al., 2016), modulation detection thresholds (Garadat et al. 2013), detection thresholds (Zhou, 2017), and electrode discrimination scores (Zwolan et al., 1997; Saleh et al., 2013). However, other studies did not show a group difference in speech perception scores when excluding electrodes based on electrode discrimination scores (Vickers et al., 2016), detection thresholds (Bierer & Litvak, 2016) or the magnitude of the polarity effect (cathodic- vs anodic-dominant triphasic pulse) on detection thresholds (Goehring et al., 2019). Reasons for the inconsistency in results between these studies remain unknown, but may be influenced by differences in the number of electrodes excluded, the tests used to evaluate speech performance, and the criteria used to determine poor ENI.

The disclosed details a model that generated an index for the quality of the ENI at individual electrode locations by optimally combining four electrophysiological measures of the eCAP. While not explored in the present study, previous studies showed that a single index of overall CN function (i.e., CN index), created with machine learning algorithms and multiple measures derived from the eCAP AGF and eCAP RRF, was significantly correlated with speech perception in quiet (see Appendix A). In contrast, individual eCAP parameters have not been able to predict speech perception scores (Brown et al., 1990; Cosetti et al., 2010; Franck & Norton, 2001; Kiefer et al., 2001; Turner et al., 2002; El Shennawy et al., 2015). Therefore, deactivating electrodes in a programming map based on a combination of multiple factors, such as was done in this study, may be more successful than deactivating electrodes based on a single factor.

### I. Conclusions

The quality of the ENI at individual electrode locations can be quantified using a local ENI index generated using the newly developed analytical model. The new model demonstrates that the quality of the ENI declines with advanced age, especially in the basal region of the cochlea. Therefore, age-related decline in the quality of the ENI may contribute to speech perception deficits observed in older CI patients.

### IV. COMPUTING ENVIRONMENT. The parts of this section IV that refer to embodiments of sections II and III describes examples useful for understanding the invention.

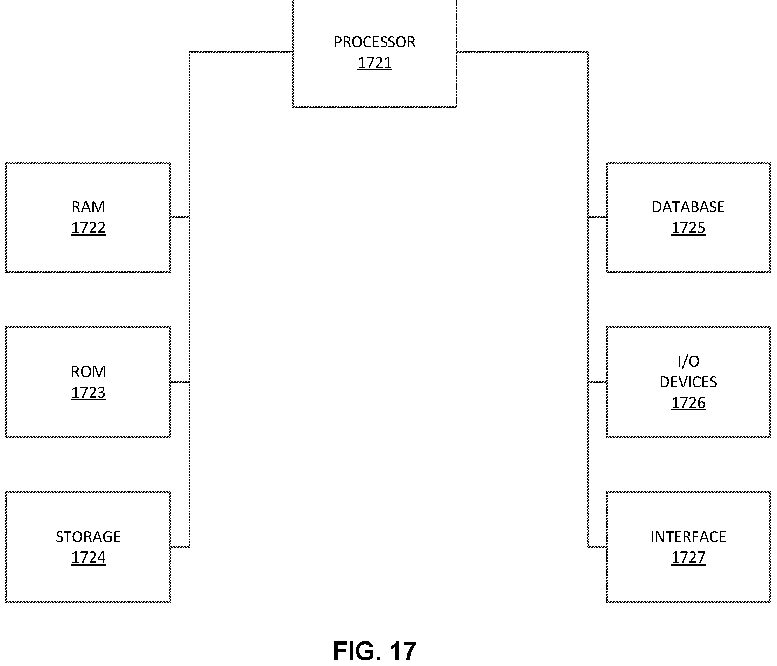

The above-described methods may be implemented on a computing system. The system has been described above as comprised of units. One skilled in the art will appreciate that this is a functional description and that the respective functions can be performed by software, hardware, or a combination of software and hardware. A unit can be software, hardware, or a combination of software and hardware. The units can comprise software for methods of determining if a response is an eCAP, refining raw data of an eCAP AGF and utilizing maximum (i.e., steepest) slope from moving linear regression to effectively estimate cochlear nerve function, and determining quality of an ENI using a model developed from eCAP attributes. In one exemplary aspect, the units can comprise a computing device that comprises a processor 1721 as illustrated in FIG. 17 and described below.

FIG. 17 illustrates an exemplary computer that can be used to determine if a response is an eCAP, refine raw data of an eCAP AGF and utilizing maximum (i.e., steepest) slope from moving linear regression to effectively estimate cochlear nerve function, and determine quality of an ENI using a model developed from eCAP attributes. As used herein, "computer" may include a plurality of computers. The computers may include one or more hardware components such as, for example, a processor 1721, a random access memory (RAM) module 1722, a read-only memory (ROM) module 1723, a storage 1724, a database 1725, one or more input/output (I/O) devices 1726, and an interface 1727. All of the hardware components listed above may not be necessary to practice the methods described herein. Alternatively and/or additionally, the computer may include one or more software components such as, for example, a computer-readable medium including computer executable instructions for performing a method associated with the exemplary embodiments. It is contemplated that one or more of the hardware components listed above may be implemented using software. For example, storage 1724 may include a software partition associated with one or more other hardware components. It is understood that the components listed above are exemplary only and not intended to be limiting.

Processor 1721 may include one or more processors, each configured to execute instructions and process data to perform one or more functions associated with a computer for discriminating tissue of a specimen. Processor 1721 may be communicatively coupled to RAM 1722, ROM 1723, storage 1724, database 1725, I/O devices 1726, and interface 1727. Processor 1721 may be configured to execute sequences of computer program instructions to perform various processes. The computer program instructions may be loaded into RAM 1722 for execution by processor 1721.

RAM 1722 and ROM 1723 may each include one or more devices for storing information associated with operation of processor 1721. For example, ROM 423 may include a memory device configured to access and store information associated with the computer, including information for identifying, initializing, and monitoring the operation of one or more components and subsystems. RAM 1722 may include a memory device for storing data associated with one or more operations of processor 1721. For example, ROM 1723 may load instructions into RAM 1722 for execution by processor 1721.

Storage 1724 may include any type of mass storage device configured to store information that processor 1721 may need to perform processes consistent with the disclosed embodiments. For example, storage 1724 may include one or more magnetic and/or optical disk devices, such as hard drives, CD-ROMs, DVD-ROMs, or any other type of mass media device.

Database 1725 may include one or more software and/or hardware components that cooperate to store, organize, sort, filter, and/or arrange data used by the computer and/or processor 1721. For example, database 1725 may store raw data, as described herein and computer-executable instructions for determining if a response is an eCAP, refining raw data of an eCAP AGF and utilizing maximum (i.e., steepest) slope from moving linear regression to effectively estimate cochlear nerve function, and determining quality of an ENI using a model developed from eCAP attributes. It is contemplated that database 1725 may store additional and/or different information than that listed above.

I/O devices 1726 may include one or more components configured to communicate information with a user associated with computer. For example, I/O devices may include a console with an integrated keyboard and mouse to allow a user to maintain a database of digital images, results of the analysis of the digital images, metrics, and the like. I/O devices 1726 may also include a display including a graphical user interface (GUI) for outputting information on a monitor. I/O devices 1726 may also include peripheral devices such as, for example, a printer for printing information associated with the computer, a user-accessible disk drive (e.g., a USB port, a floppy, CD-ROM, or DVD-ROM drive, etc.) to allow a user to input data stored on a portable media device, a microphone, a speaker system, or any other suitable type of interface device.

Interface 1727 may include one or more components configured to transmit and receive data via a communication network, such as the Internet, a local area network, a workstation peer-to-peer network, a direct link network, a wireless network, or any other suitable communication platform. For example, interface 1727 may include one or more modulators, demodulators, multiplexers, demultiplexers, network communication devices, wireless devices, antennas, modems, and any other type of device configured to enable data communication via a communication network.

### V. CONCLUSION

While the methods and systems have been described in connection with preferred embodiments and specific examples, it is not intended that the scope be limited to the particular embodiments set forth, as the embodiments herein are intended in all respects to be illustrative rather than restrictive.

Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; the number or type of embodiments described in the specification.

Throughout this application, various publications may be referenced. The publications are the following:

| | |
|---|---|
| A. | Abbas, P. J., Hughes, M., Brown, C. J., et al. (2004). Channel Interaction in Cochlear Implant Users Evaluated Using the Electrically Evoked Compound Action Potential. 548 Audiol Neurootol. 9. 203-13. |
| B. | Bierer, J. A. (2010). Probing the electrode-neuron interface with focused cochlear implant stimulation. Trends Amplif, 14, 84-95. |
| C. | Bierer, J. A., & Litvak, L. (2016). Reducing channel interaction through cochlear implant programming may improve speech perception: Current focusing and channel deactivation. Trends Hear, 20, 1-12. |
| D. | Birman, C. S., Powell, H. R., Gibson, W. P., et al. (2016). Cochlear implant outcomes in cochlea nerve aplasia and hypoplasia. Otol Neurotol, 37, 438-445. |
| E. | Bodmer, D., Shipp, D. B., Ostroff, J. M., et al. (2007). A comparison of postcochlear implantation speech scores in an adult population. Laryngoscope, 117(8), 1408-1411. |
| F. | Botros, A., & Psarros, C. (2010). Neural Response Telemetry Reconsidered: II. The Influence of Neural Population on the eCAP Recovery Function and Refractoriness. Ear Hear, 31, 380-391. |
| G. | Brown, C. J., Abbas, P. J., Gantz, B. (1990). Electrically evoked whole-nerve |
| | action potentials: data from human cochlear implant users. J Acoust Soc Am, 88, 1385-1391. |
| H. | Brown, C. J., Abbas, P. J., Etler, C. P., O'Brien, S., & Oleson, J. J. (2010). Effects of long-term use of a cochlear implant on the electrically evoked compound action potential. J Am Acad Audiol, 21(1), 5-15. |
| I. | Budenz, C. L., Cosetti, M. K., Coelho, D. H., et al. (2011). The effects of cochlear implantation on speech perception in older adults. J Am Geriatr Soc, 59, 446-453. |
| J. | Cafarelli Dees, D., Dillier, N., Lai, W. K., et al. (2005). Normative findings of electrically evoked compound action potential measurements using the neural response telemetry of the Nucleus CI24M cochlear implant system. Audiol Neurootol, 10, 105-116. |
| K. | Cohen, L. T., Saunders, E., & Clark, G. M. (2001). Psychophysics of a prototype peri-modiolar cochlear implant electrode array. Hear Res, 155(1-2), 63-81. |
| L. | Cosetti, M. K., Shapiro, W. H., Green, J. E., et al. (2010). Intraoperative neural response 574 telemetry as a predictor of performance. Otol Neurotol. 31:1095-1099. |
| M. | Eisen, M. D., & Franck, K. H. (2004). Electrically evoked compound action potential amplitude growth functions and HiResolution programming levels in pediatric CII implant subjects. Ear Hear, 25, 528-538. |
| N. | El Shennawy, A. M., Mashaly, M. M., Shabana, M. I., Sheta, S. M. (2015). Telemetry changes over time in cochlear implant patients. Hearing Balance Commun. 13:24-31. |
| O. | Franck, K. H., Norton, S. J. (2001). Estimation of psychophysical levels using the electrically evoked compound action potential measured with the neural response telemetry capabilities of Cochlear Corporation's CI24M device. Ear Hear. 22:289-299. |
| P. | Friedland, D. R., Runge-Samuelson, C., Baig, H., et al. (2010). Case-control analysis of cochlear implant performance in elderly patients. Arch Otolaryngol Head Neck Surg, 136, 432-438. |
| Q. | Gantz, B. J., Brown, C. J., Abbas, P. J. (1994). Intraoperative measures of electrically evoked auditory nerve compound action potential. Am J Otol. |
| | 15:137-144. |
| R. | Garadat, S. N., Zwolan, T. A., & Pfingst, B. E. (2013). Using temporal modulation sensitivity to select stimulation sites for processor MAPs in cochlear implant listeners. Audiol Neurotol, 18(4), 247-260. |
| S. | Goehring, T., Archer-Boyd, A., Deeks, J. M., et al. (2019). A site-selection strategy based on polarity sensitivity for cochlear implants: effects on spectro-temporal resolution and speech perception. J Assoc Res Otolaryngol, 20(4), 431-448. |
| T. | Han, J. J., Suh, M. W., Park, M. K., et al. (2019). A Predictive Model for Cochlear Implant Outcome in Children with Cochlear Nerve Deficiency. Sci Rep, 9(1), 1154. |
| U. | He, S., Shahsavarani, B. S., McFayden, T. C., et al. (2018). Responsiveness of the electrically stimulated cochlear nerve in children with cochlear nerve deficiency. Ear Hear, 39, 238-250. |
| V. | Hughes, M. L., Vander Werff, K. R., Brown, C. J., et al. (2001). A longitudinal study of electrode impedance, the electrically evoked compound action potential, and behavioral measures in nucleus 24 cochlear implant users. Ear hear, 22(6), 471-486. |
| W. | Jackler, R. K., Luxford, W. M., House, W. F. (1987). Congenital malformations of the inner ear: a classification based on embryogenesis. Laryngoscope, 97(3 Pt 2; Suppl 40), 2-14. |
| X. | Jahn, K. N., & Arenberg, J. G. (2020). Electrophysiological Estimates of the Electrode-Neuron Interface Differ Between Younger and Older Listeners with Cochlear Implants. Ear Hear, 41(4), 948-960. |
| Y. | Kiefer, J., Hohl, S., Sturzebecher, E., et al. (2001). Comparison of speech recognition with different speech coding strategies (SPEAK, CIS, and ACE) and their relationship to telemetric measures of compound action potentials in the nucleus CI 24M cochlear implant system. Audiology. 40:32-42. |
| Z. | Kim, J.R., Abbas, P.J., Brown, C.J., et al. (2010). The relationship between electrically evoked compound action potential and speech perception: a study in cochlear implant users with short electrode array. Otol Neurotol, 31, 1041-1048. |
| AA. | Kirby, A. E., & Middlebrooks, J. C. (2010). Auditory temporal acuity probed |
| | with cochlear implant stimulation and cortical recording. J Neurophysiol, 103(1), 531-542. |
| BB. | Kirby, A. E., & Middlebrooks, J. C. (2012). Unanesthetized auditory cortex exhibits multiple codes for gaps in cochlear implant pulse trains. J Assoc Res Otolaryngol, 13(1), 67-80. |
| CC. | Lenarz, M., Sonmez, H., Joseph, G., et al. (2012). Cochlear implant performance in geriatric patients. Laryngoscope, 122, 1361-1365. |
| DD. | Lin, H. W., Furman, A. C., Kujawa, S. G., et al. (2011). Primary neural degeneration in the Guinea pig cochlea after reversible noise-induced threshold shift. J Assoc Res Otolaryngol, 12, 605-616. |
| EE. | Lin, F. R., Chien, W. W., Li, L., et al. (2012). Cochlear implantation in older adults. Medicine, 91(5), 229. |
| FF. | Long, C. J., Holden, T. A., McClelland, G. H., et al. (2014). Examining the electro-neural interface of cochlear implant users using psychophysics, CT scans, and speech understanding. J Assoc Res Otolaryngol, 15(2), 293-304. |
| GG. | Makary, C. A., Shin, J., Kujawa, S. G., et al. (2011) Age-Related Primary Cochlear Neuronal Degeneration in Human Temporal Bones. JARO 12, 711-717. |
| HH. | Mehmood, T., Liland, K. H., Snipen, L., et al. (2012). A review of variable selection methods in Partial Least Squares Regression. Chemometrics and Intelligent Laboratory Systems, 118, 62-69. |
| II. | Miller, C. A., Abbas, P. J., Brown, C. J. (2000). An Improved Method of Reducing Stimulus Artifact in the Electrically Evoked Whole-Nerve Potential. Ear Hear, 21, 280-290. |
| JJ. | Morsnowski, A., Charasse, B., Collet, L., et al. (2006). Measuring the Refractoriness of the Electrically Stimulated Auditory Nerve. Audiol Neurootol, 11, 389-402. |
| KK. | Noble, J. H., Hedley-Williams, A. J., Sunderhaus, L., et al. (2016). Initial results with image-guided cochlear implant programming in children. Otol Neurotol, 37, e63-e69. |
| LL. | Pfingst, B.E., Colesa, D.J., Watts, M.M., et al. (2017). Neurotrophin gene therapy in deafened ears with cochlear implants: long-term effects on nerve survival and functional measures. J Assoc Res Otolaryngol, 18, 731-750. |
| MM. | Pfingst, B.E., Hughes, A.P., Colesa, D.J., et al. (2015). Insertion trauma and recovery of function after cochlear implantation: evidence from objective functional measures. Hear Res, 330, 98-105. |
| NN. | Ramekers, D., Versnel, H., Strahl, S.B., et al. (2014). Auditory-nerve response to varied inter-phase gap and phase duration of the electric pulse stimulus as predicators for neuronal degeneration. J Assoc Res Otolaryngol, 15, 187-202. |
| OO. | Roberts, D. S., Lin, H. W., Herrmann, B. S., et al. (2013). Differential cochlear implant outcomes in older adults. Laryngoscope, 123, 1952-1956. |
| PP. | Saleh, S. M., Saeed, S. R., Meerton, L., et al. (2013). Clinical use of electrode differentiation to enhance programming of cochlear implants. Cochlear implants int, 14(sup4), 16-18. |
| QQ. | Schvartz-Leyzac, K. C., & Pfingst, B. E. (2016). Across-site patterns of electrically evoked compound action potential amplitude-growth functions in multichannel cochlear implant recipients and the effects of the interphase gap. Hear Res, 341, 50-65. |
| RR. | Schvartz-Leyzac, K. C., & Pfingst, B. E. (2018). Assessing the relationship between the electrically evoked compound action potential and speech recognition abilities in bilateral cochlear implant recipients. Ear Hear, 39, 344-358. |
| SS. | Schvartz-Leyzac, K. C., Holden, T. A., Zwolan, T. A., et al. (2020). Effects of electrode location on estimates of neural health in humans with cochlear implants. J Assoc Res Otolaryngol, 21(3), 259-275. |
| TT. | Shepherd, R. K., Roberts, L. A., Paolini, A. G. (2004). Long-term sensorineural hearing loss induces functional changes in the rat auditory nerve. Eur J Neurosci, 20, 3131-3140. |
| UU. | Skidmore, J., Xu, L., Chao, X., et al. (2021a). Prediction of the Functional Status of the Cochlear Nerve in Individual Cochlear Implant Users Using Machine Learning and Electrophysiological Measures. Ear Hear. 42(1), 180-192. |
| VV. | Skidmore, J., Ramekers, D., Colesa, D., et al. (2021b). A Robust and Broadly Applicable Method for Characterizing the Slope of the Electrically-evoked Compound Action Potential Amplitude Growth Function. Association for Research in Otolaryngology (ARO) 44th MidWinter Meeting. |
| WW. | Sladen, D. P., & Zappler, A. (2015). Older and younger adult cochlear implant users: Speech recognition in quiet and noise, quality of life, and music perception. Am J Audiol, 24(1), 31-39. |
| XX. | Turner, C., Mehr, M., Hughes, M., et al. (2002). Within-subject predictors of speech recognition in cochlear implants: a null result. Acoust Res Lett Online. 3:95-100. |
| YY. | Vickers, D., Degun, A., Canas, A., et al. (2016). Deactivating cochlear implant electrodes based on pitch information for users of the ACE strategy. In Physiology, psychoacoustics and cognition in normal and impaired hearing (pp. 115-123). Springer, Cham. |
| ZZ. | Vincenti, V., Ormitti, F., Ventura, E., et al. (2014). Cochlear implantation in children with cochlear nerve deficiency. Int J Pediatr Otorhinolaryngol, 78, 912-917. |
| AAA. | Wiemes, G. R. M., Hamerschmidt, R., Moreira, A. T. R., et al. (2016). Auditory Nerve Recovery Function in Cochlear Implant Surgery with Local Anesthesia and Sedation versus General Anesthesia. Audiol Neurootol, 21, 150-157. |
| BBB. | Wu, P. Z., Liberman, L. D., Bennett, K., et al. (2019). Primary Neural Degeneration in the Human Cochlea: Evidence for Hidden Hearing Loss in the Aging Ear. Neuroscience, 407, 8-20. |
| CCC. | Young, N. M., Kim, F. M., Ryan, M. E., et al. (2012). Pediatric cochlear implantation of children with eighth nerve deficiency. Int J Pediatr Otorhinolaryngol, 76, 1442-1448. |
| DDD. | Young, N. M., & Grohne, K. M. (2001). Comparison of pediatric Clarion recipients with and without the electrode positioner. Otol Neurotol, 22, 195-199. |
| EEE. | Zhou, N. (2017). Deactivating stimulation sites based on low-rate thresholds improves spectral ripple and speech reception thresholds in cochlear implant users. J Acoust Soc Am, 141, EL243. |
| FFF. | Zarowski, A., Molisz, A., De Coninck, L., et al. (2020). Influence of the pre-or postlingual status of cochlear implant recipients on behavioural T/C-levels. Int J Pediatr Otorhinolaryngol, 131, 109867. |
| GGG. | Zekveld, A., Kramer, S.E., & Festen, J. (2011). Cognitive load during speechperception in noise: the influence of age, hearing loss, and cognition on pupil response. Ear Hear, 32, 498-510. |
| | |
| HHH. | Zwolan, T. A., Collins, L. M., & Wakefield, G. H. (1997). Electrode discrimination and speech recognition in postlingually deafened adult cochlear implant subjects. J Acoust Soc Am. 102, 3673-3685. |

### ABSTRACT

**Objective:** This study aimed to create an objective predictive model for assessing the functional status of the cochlear nerve (CN) in individual cochlear implant (CI) users.

**Design:** Study participants included 23 children with cochlear nerve deficiency (CND), 29 children with normal-sized CNs (NSCNs), and 20 adults with various etiologies of hearing loss. Eight participants were bilateral CI users and were tested in both ears. As a result, a total of 80 ears were tested in this study. All participants used Cochlear^{®} Nucleus^{™} CIs in their test ears. For each participant, the CN refractory recovery function (RRF) and input/output (I/O) function were measured using electrophysiological measures of the electrically-evoked compound action potential (eCAP) at three electrode sites across the electrode array. Refractory recovery time constants were estimated using statistical modeling with an exponential decay function. Slopes of I/O functions were estimated using linear regression. The eCAP parameters used as input variables in the predictive model were absolute refractory recovery time estimated based on the RRF, eCAP threshold, slope of the eCAP I/O function, and negative-peak (i.e., N1) latency. The output variable of the predictive model was CN index, an indicator for the functional status of the CN. Predictive models were created by performing linear regression, support vector machine regression, and logistic regression with eCAP parameters from children with CND and the children with NSCNs. One-way analysis of variance with post hoc analysis with Tukey's honest significant difference criterion was used to compare study variables among study groups.

**Results:** All three machine learning algorithms created two distinct distributions of CN indices for children with CND and children with NSCNs. Variations in CN index when calculated using different machine learning techniques were observed for adult CI users. Regardless of these variations, CN indices calculated using all three techniques were significantly correlated with Consonant-Nucleus-Consonant word and AzBio sentence scores measured in quiet.

**Conclusions:** The functional status of the CN for individual CI users could be predicted based on our newly developed analytical models. Model predictions of CN function for individual adult CI users were positively and significantly correlated with speech perception performance. The models presented in this study may be useful for understanding and/or predicting CI outcomes for individual patients.

**Key Words:** cochlear implant, auditory nerve, electrically evoked auditory compound action potentials, supervised machine learning

### INTRODUCTION

A puzzling challenge in providing care for patients with cochlear implants (CIs) arises from unexplained variability and large individual differences in CI patient outcomes (e.g. Firszt et al., 2004; Lazard et al., 2012; Holden et al., 2013; Moberly et al., 2016). Substantial variability in CI outcomes exists even in adults with post-lingual deafness (Blamey et al., 2013; Beyea et al., 2016), a patient population that would be expected to have generally good CI outcomes because of their previous normal language development (Moberly et al., 2016). Many factors contribute to the observed variability in CI patient outcomes and can be described by three broad categories: auditory sensitivity, linguistic skills, and neurocognitive function (Moberly et al., 2016). Deficits in any of these "bottom-up" or "top-down" processes may negatively impact CI patient outcomes.

Theoretically, good auditory sensitivity depends on the presence of a sufficiently large group of cochlear nerve (CN) fibers that are able to encode and transmit electrical representations of auditory information to the central nervous system. Therefore, the number of CN fibers and their responsiveness to electrical stimulation (i.e., functional status of the CN) should be influential in CI outcomes. The results from studies in animal models (e.g., Ramekers et al., 2014; Pfingst et al., 2017) coupled with results from studies with human listeners (Kim et al., 2010; Teagle et al., 2010; Zhou & Pfingst, 2014; Schvartz-Leyzac & Pfingst, 2018; He et al., 2018) support this hypothesis. Indeed, there is a growing body of literature supporting the importance of the underlying neural function for CI outcomes (e.g., Kirby & Middlebrooks, 2010, 2012; Kim et al., 2010; Teagle et al., 2010; Garadat et al., 2012; Long et al., 2014; Zhou & Pfingst, 2014; Schvartz-Leyzac & Pfingst, 2018; He et al., 2018). Therefore, accurately estimating the functional status of the CN for individual patients may be important for understanding inter-patient variations and predicting CI outcomes for individual patients.

While the number of CN fibers and their responsiveness to electrical stimulation cannot be directly measured in human listeners, results of animal studies show that basic neural response properties are determined by the functional status of the CN (Shepherd et. al, 2004; Pfingst et al., 2015b). Specifically, rats with smaller densities of spiral ganglion neurons (SGNs) have higher response thresholds and longer absolute refractory periods than normal control rats (Shepherd et. al, 2004). Pfingst et al. (2015b) showed that detection thresholds decreased as a function of stimulus duration at a slower rate in guinea pigs with worse neural survival. These neural response properties can also be derived from electrophysiological measures of the electrically-evoked compound action potential (eCAP) in human listeners. The eCAP is a near-field neural response that is generated by CN fibers responding synchronously to electrical stimulation (Brown et al., 1990; Miller et al., 2000; Abbas et al., 2004). The eCAP typically consists of a negative peak (N1) followed by a smaller positive peak or plateau (P2) occurring within the time windows of 0.2-0.4 ms and 0.6-0.8 ms after stimulus onset, respectively (Brown et al., 1998; Abbas et al., 1999). The presence of the eCAP response depends on a sufficient number of neurons firing synchronously, and therefore, can provide useful insight into the functional status of CN fibers in human listeners (He et al., 2018, 2019a; Luo et al., 2019).

Results of studies with animal models and human listeners indicate that several eCAP parameters are associated with the functional status of CN fibers (e.g. Ramekers et al., 2004; Pfingst et al., 2017; He et al., 2018). Two parameters that have been studied are the absolute refractory period (ARP) and the relative refractory period (RRP, Morsnowski et al., 2006; Botros & Psarros, 2010; Fulmer et al., 2010; He et al., 2018). ARP refers to the time immediately following a previous stimulation in which a neuron cannot produce an action potential regardless of the magnitude of the stimulus. The RRP follows the ARP and refers to the period in which the probability of the neuron firing gradually increases such that the neuron can be activated by a sufficiently strong stimulus. The ARP and the RRP can be estimated based on the eCAP refractory recovery function (RRF).

The eCAP RRF is typically measured with two biphasic, charge balanced, electrical pulses using a modified template subtraction method (Miller et al., 2000) in which the time between the masker and probe [i.e. masker-probe interval (MPI)] is systematically varied from 300 to 10,000 µs (He et al., 2017). As the MPI increases, the auditory nerve gradually recovers from the refractoriness induced by the masker, which results in larger eCAPs at longer MPIs. The eCAP RRF has frequently been modeled as an exponential decay function (e.g., Morsnowski et al., 2006; Botros & Psarros, 2010; Fulmer et al., 2010; He et al., 2018) where the x-intercept (i.e. t₀) and the rate of decay (i.e. *τ*) are estimates of the ARP and RRP, respectively. t₀ has been shown to be larger in CI users with poorer auditory function (He et al., 2018). Several studies that estimated the RRP showed no significant changes in *τ* due to etiology of hearing loss (Fulmer et al., 2010; He et al., 2018), type of anesthesia (Wiemes et al., 2016), or age (Lee et al., 2012). Therefore, results from these studies suggest that lower *t*₀ values are associated with better neural function while *τ* does not appear to be correlated with neural function.

Other parameters associated with neural function of CN fibers include slope of the eCAP input/output (I/O) function, the eCAP threshold (i.e., the lowest stimulation level that evokes an eCAP), and the eCAP amplitude measured at the maximum comfortable level (i.e., C level). These three parameters can be derived from the eCAP I/O function which is created by plotting eCAP amplitudes as a function of stimulation level. The relationship between parameters derived from the eCAP I/O function and the functional status of the CN have been investigated in animal models (Prado-Guitierrez et al., 2006; Ramekers et al., 2014; Pfingst et al., 2015a, 2017) as well as in human listeners (Kim et al., 2010; Van de Heyning et al., 2016; He et al., 2018, 2019a). Steeper slopes of the eCAP I/O function (Kim et al., 2010; Ramekers et al., 2014; Pfingst et al., 2015a, 2017; He et al., 2018, 2019a), lower eCAP thresholds (He et al., 2018, 2019a), and larger eCAP amplitudes at C level (Ramekers et al., 2014; He et al., 2018, 2019a) suggest superior neural function.

The use of machine learning techniques to create predictive models in CI research has grown rapidly in recent years (see Crowson et al., 2020 for recent review). Most pertinent to the present work is the use of machine learning algorithms in the prediction of CI outcomes. Specifically, support vector machines (SVMs) have been used to predict speech perception and language skills from preoperative data in pediatric CI users (Tan et al., 2015; Feng et al., 2018). For adult CI users, the k-nearest neighbors algorithm has been combined with linear regression (Ramos-Miguel et al., 2015) or logistic regression (Guerra-Jimenez et al., 2016) to predict CI outcomes. While several studies have applied machine learning techniques to automating eCAP detection and measurement (e.g. Botros et al., 2007; van Dijk et al., 2007; Gartner et al., 2010), there are still no studies that have integrated machine learning techniques to predict auditory neural function.

In this study, analytical models for predicting the functional status of the CN for individual CI users were created using supervised machine learning techniques based on eCAP results. The specific electrophysiological parameters used in this study are derived from the eCAP RRF and eCAP I/O function and are described in detail in the methods section. The structure of the predictive models is founded on neurophysiological evidence from a recent study showing significantly poorer CN function in children with cochlear nerve deficiency (CND) than in children with normal-sized CNs (NSCNs, He et al., 2018). CND refers to a small or absent CN as revealed by results of high-resolution magnetic resonance imaging (MRI) scans (Glastonbury et al., 2002; Adunka et al., 2006; Buchman et al., 2006; Kutz et al, 2011; Clemmens et al., 2013). The CN is considered to be small when the nerve is evident on the MRI scan but substantially smaller than the contralateral CN (Adunka et al., 2006; Buckman et al., 2006), other nerves in the internal auditory canal (Buchman et al., 2006; Kutz et al., 2011), or expected size in normal ears (Adunka et al., 2006; Clemmens et al., 2013). The CN is considered to be absent when it cannot be visually identified with a MRI scan (Glastonbury et al., 2002; Adunka et al., 2006; Buchman et al., 2006; Kutz et al, 2011; Clemmens et al., 2013). Irrespective of the size of the CN, cochlear implantation is considered as a treatment option for children with CND. Substantial variability in CI outcomes among children with CND have been reported, ranging from no sound awareness to understanding open-set speech (e.g., Young et al., 2012; Vincenti et al., 2014; Birman et al., 2016; Han et al., 2019). Despite individual differences, children with CND as a group have been shown to have significantly worse CI outcomes than age-matched CI patients with NSCNs (Kang et al., 2010; Wei et al., 2017). Therefore, due to the large disparity in neural function and CI outcomes, results measured in children with CND and in children with NSCNs served as a suitable training dataset for the models developed in this study.

Three regression algorithms (linear, SVM, and logistic) were used in this study for several reasons. First, the function that best stratifies patients according to CN function is unknown. Second, these techniques have been used in other CI studies (Ramos-Miguel et al., 2015; Tan et al., 2015; Guerra-Jimenez et al., 2016; Feng et al., 2018). Third, the underlying assumptions of these algorithms were met. Specifically, the eCAP parameters used in regression were verified to be linearly independent and residuals were verified to be homoscedastic. Therefore, all of these regression algorithms were appropriate for developing the models of this study.

We hypothesized that the models would stratify individual patients according to the functional status of the CN. Based on this hypothesis, we expected distinct distributions (i.e., clear separation) of CN index for children with CND and children with NSCNs. We further expected that CN indices for adult study participants would be generally worse than those for children with NSCNs but better than those for children with CND because CN function deteriorates with advanced age (e.g. McFadden et al., 1997; Makary et al., 2011; Viana et al., 2015; Wu et al., 2019). Finally, we expected that the CN index would be positively correlated with speech outcome measures because recent studies have suggested that CI outcomes are related to the underlying CN function (Kim et al., 2010; Zhou & Pfingst, 2014; Schvartz-Leyzac & Pfingst, 2018; He et al., 2018).

### MATERIALS AND METHODS

### Study Participants

A total of 72 participants from three patient populations were enrolled in this study. The eCAP results from each study group were used for either model creation or model validation. Study participants whose eCAP results were used for training the model included 23 children with CND (CND1-CND23) and 29 children with NSCNs (NSCN1-NSCN29). Results from 20 adults (A1-A20) with various etiologies of hearing loss were used to validate the model and to explore the potential application of the model developed in this study for clinical application. One child with CND (CND23), three children with NSCNs (NSCN22, NSCN25, and NSCN26), and four adults (A4, A5, A7 and A13) were bilateral CI users and were tested in both ears. Results recorded in 14 children with CND (CND7-CND20) and 9 children with NSCNs (NSCN7-NSCN15) have been reported in He et al. (2018). Detailed demographic information of all participants included in this study is listed in Table 1.

### Insert Table 1 about here

The anatomical status of the CN and the inner ear was determined based on results of high resolution MRI and Computed Tomography (CT) temporal bone scans following the same protocol and criteria as described in our previous studies (He et al., 2018; Luo et al., 2019).

Participants were recruited and tested in one of three locations: The Ohio State University (OSU; CND23, NSCN25-29, and A1-20), The University of North Carolina (UNC) at Chapel Hill (UNC-CH; CND7-10, NSCN7, NSCN9 and NSCN12), or Shandong ENT Hospital (SENTP; CND1-6, CND11-22, NSCN1-6, NSCN8, NSCN10-11, and NSCN13-24). The biomedical institutional review board (IRB) of each institution approved this study (IRB study #: OSU, 2017H0131 and 2018H0344; UNC-CH, 12-1737; SENTP, 2016-2).

Prior to data collection, written informed consent was obtained from all participants and/or their legal guardians when applicable. All participants received financial compensation for their participation.

### Procedures

### Testing Electrodes

All participants, except for CND5 and CND6, had a full electrode array insertion, which means that electrodes 1 and 22 of Cochlear^{®} Nucleus^{™} CIs (Cochlear Ltd., Macquarie, NSW, Australia) were placed near the base and the apex of the cochlea, respectively. The electrode array was partially inserted in CND5 and CND6 due to Incomplete Partition Type II (IP-II). For all participants, three electrodes were tested for eCAP measures. For children with CND, these three testing sites ranged from the most basal to the most apical electrode location where an eCAP could be recorded and had a relatively equal separation between testing electrodes. For children with NSCNs and adult CI users, typically electrodes 3, 12 and 21 were tested. Electrodes tested in individual patients are listed in Table 1. These testing electrodes were referred to as the "basal", the "middle" and the "apical" electrode based on their relative locations among selected electrodes along the electrode array.

### eCAP Measures

Electrophysiological measures of the eCAP were acquired using the Advanced Neural Response Telemetry (NRT) function implemented in the Custom Sound EP (v. 4.3 or 5.1) software (Cochlear Ltd, Macquarie, NSW, Australia). The stimulus was a symmetric, cathodic-leading, biphasic pulse which has been used extensively in previous studies (e.g., Prado-Guitierrez et al., 2006; Kim et al., 2010; Ramekers et al., 2014; Pfingst et al., 2015a, 2015b, 2017; He et al., 2018, 2019a). The interphase gap used in all participants was 7 µs. For participants with normal-sized CNs (both adults and children), the pulse phase duration (PPD) was 25 µs/phase. For children with CND, the PPD varied across participants within the range of 37 and 100 µs/phase. Longer PPDs were needed for these participants to deliver sufficient stimulation for eCAP recording that stays within the voltage compliance limit of the device. The PPD for all participants is reported in Table 1. Other parameters used for eCAP measures included a 15 Hz probe rate, sampling delays between 98 and 122 µs, and an effective sampling rate of 20 kHz. An amplifier gain of 50 dB and 50 sweeps per averaged eCAP response were used for all adult participants and children with NSCNs. The amplifier gain was set to 40 dB and 100 sweeps were used for each averaged eCAP response measured in children with CND. These parameters are recommended by He et al. (2019b) to minimize artifact contamination in eCAPs measured in children with CND. The stimulus was presented to individual CI electrodes via a N6 sound processor that was connected to a programming pod.

For the eCAP I/O function measurement, the eCAP was measured using a two-pulse forward-masking-paradigm (Brown et al., 1990), in which the masker pulse was always presented at 10 current levels (CLs) higher than the probe pulse. The masker pulse was initially presented at the maximum comfortable level (i.e., C level), followed by a systematic decrease in steps of 5 CLs until no response could be visually identified. The stimulation level was subsequently increased in steps of 1 CL until at least five eCAPs were measured using this small step size. The MPI was 400 µs.

The eCAP RRF was obtained with two biphasic, charge balanced, electrical pulses using a modified template subtraction method (Miller et al., 2000). The masker pulse was presented at C level and the probe pulse was presented at 10 CLs below C level. eCAPs were recorded as the MPI was systematically increased from 100 µs to 10 ms. The maximum masker stimulation level for the eCAP RRF was the same as the masker stimulation level for the eCAP I/O function for all except three electrodes (A2, electrode 18; A8, electrodes 12 and 21). For these three electrodes, there was a 1 CL difference between the maximum masker stimulation levels used to measure these two functions.

### Speech Perception Scores

Participants' speech perception capabilities were evaluated using Consonant-Nucleus-Consonant (CNC) word lists (Peterson & Lehiste, 1962) and AzBio sentences (Spahr et al., 2015) in quiet. Results of several studies have shown that cognitive function plays an important role for speech perception in noise (e.g., Dryden et al., 2017; Nuesse et al., 2018). Therefore, speech perception scores were only measured in quiet in this study in order to minimize the effects of cognitive function on study results. All speech perception testing took place in sound-proof booths, using the procedure described in the new Minimum Speech Test Battery (MSTB, 2011). The auditory stimuli were presented in the sound booth via a speaker placed one meter in front of the participant at zero degrees azimuth, calibrated to 60 dB(A) sound pressure level using a sound level meter. For the four participants who are bilateral CI users (A4, A5, A7 and A13), speech perception scores were measured for each test ear separately.

### Data Analysis

### eCAP Refractory Recovery Function

The top panels of Figure 1 show eCAPs recorded at different MPIs for electrical stimulations at electrode 3 in participants CND22, NSCN9, and A12, respectively. The eCAP amplitudes measured at different MPIs were normalized to the eCAP amplitude measured at 10 ms and plotted as a function of MPI to generate the eCAP RRF. Two children with CND (CND5 and CND16) did not have a recorded eCAP with an MPI of 10 ms at the apical electrode, and so the eCAP amplitude recorded at the next longest MPI (i.e., 8.1 ms) was used for normalization. The bottom panels of Figure 1 show the normalized eCAP amplitudes along with the fitted exponential decay functions used to estimate ARP and RRP, at electrode 3 in participants CND22, NSCN9, and A12, respectively.

For all participants, estimates of the ARP (i.e., t₀) and RRP (i.e., *τ*) were found using statistical modeling with the exponential decay function ${eCAP}_{N} = A - {Ae}^{- \frac{MPI - {t}_{0}}{τ}}$ where *eCAP_{N}* is the normalized eCAP amplitude, *A* represents the maximum normalized eCAP amplitude, and *MPI* is the masker probe interval in ms. This exponential decay function has been used to create the eCAP RRF and estimate the ARP and RRP in previously published studies (e.g., Morsnowski et al., 2006; Botros & Psarros, 2010; Wiemes et al., 2016; He et al., 2018). When the ARP estimate was unreasonable (i.e., *t*₀ < 0) due to data recordings that were poorly represented by the exponential decay function, the ARP was estimated as the shortest MPI that was longer than 350 µs at which an eCAP was recorded. The shortest MPI was used instead of *t*₀ in 10% of the electrodes tested. Poor fitting of the exponential function occurred most frequently in children with CND. Substitution of shortest MPI for *t*₀ was only performed on four electrodes for participants who did not have CND (A13L, electrode 3; A14, electrode 3; NSCN27, electrodes 3 and 12).

### Insert Figure 1 about here

### eCAP I/O function

Stimulation levels were converted to units of electrical charge in nanocoulombs (nC) per phase due to variations in PPD among participants. The top panels of Figure 2 show eCAPs recorded at different stimulation levels at electrode 21 in participants CND22, NSCN2, and A11, respectively. The bottom panels of Figure 2 show eCAP amplitudes as a function of stimulation level. The slope of the eCAP I/O function was estimated using linear regression with the linear function $eCAP = a ∗ SL + b$ where *eCAP* is the eCAP amplitude in µV, *a* represents the slope of the eCAP I/O function, *SL* is the stimulation level in nC, and b represents the intercept of the function with the vertical axis. Linear regression is the most commonly used function to estimate the slope of the eCAP I/O function (e.g., Brown et al., 1990; Kim et al., 2010; Schvartz-Leyzak and Pfingst, 2016).

### Insert Figure 2 about here

### Predictive models

### Model variables

eCAP parameters used as input variables in the predictive models were derived from the eCAP RRF and eCAP I/O function. These parameters include *t*₀, the eCAP threshold, slope of the eCAP I/O function, and N1 latency of the eCAP with the maximum amplitude. The output variable is a number which represents the functional status of the CN. For model training, the value of this variable was 0 for children with CND and 1 for children with NSCNs. For model prediction, the output is a value between 0 and 100, where 0 represents the poorest neural function among study participants and 100 represents the best neural function among participants included in the study. This number between 0 and 100 is defined as the CN index.

While the relationship between CN neural function and N1 latency has not been well studied in the literature, we have observed that eCAPs recorded in children with CND have prolonged N1 latencies compared to those recorded in children with NSCNs (Xu et al., 2019). Additionally, independent two-sample t-tests comparing the CND and NSCN study groups revealed significant differences for all of the included eCAP parameters: *t*₀ (t₍₁₆₆₎ = 6.15, p<0.001), the eCAP threshold (t₍₁₆₆₎ = 15.89, p<0.001), slope of the I/O function (t₍₁₆₆₎ = 9.06, p<0.001), and N1 latency (t₍₁₆₆₎ = 12.11, p<0.001). Therefore, all of these eCAP parameters were included in the predictive models. The eCAP amplitude measured at C level was not included in the models because it is strongly correlated with the slope of the eCAP I/O function (r=0.86, p<0.001) and provides redundant information. *τ* and P2 latency were not included in the models because other studies suggest that *τ* is not related to CN function (Fulmer et al., 2010; Lee et al., 2012; Wiemes et al., 2016; He et al., 2018), and P2 latency is statistically dependent on N1 latency.

The eCAP parameters recorded at each electrode site were included together in one combined vector because the aim of this study was to create an objective model that predicts overall CN function for individual patients. The eCAP parameters were concatenated based on known patterns of neural function (i.e., "low", "medium", "high") to provide a consistent comparison across patient populations for estimating overall CN function. Specifically, the eCAP parameters were concatenated from basal to apical electrode site (i.e., "basal", "middle", "apical") for adult and NSCN groups. The eCAP parameters for the CND group were arranged in reverse order (i.e., "apical", "middle", "basal") because children with CND have better neural function in the basal region compared to the apical region (He et al., 2018), which is opposite to the neural-functional pattern of typical CI users (Propst et al., 2006; Gordon et al., 2007; Brill et al., 2009; Hughes et al., 2009).

### Model structure

eCAP parameters in children with CND and children with NSCNs were used as the training dataset for regression models that separate CN function between these two patient populations. Specifically, each eCAP parameter was standardized across all pediatric participants to eliminate any bias due to differences in scale between the eCAP parameters. Each eCAP parameter was standardized according to $x = \frac{x ′ - μ}{σ}$ where x is a vector containing the normalized value for the eCAP parameter for each pediatric participant, x' is a vector containing the non-normalized value for the eCAP parameter for each pediatric participant, and *µ* and *σ* are the is the mean and standard deviation of x', respectively. The recorded eCAP parameters for each adult participant were also standardized using the means and standard deviations from the pediatric (i.e., training) data according to Equation 3 for model prediction.

The twelve standardized eCAP parameters (4 eCAP parameters x 3 electrode locations) from the pediatric participants were used as the input variables (*x*₁, *x*₂, *... , x*₁₂), to train the predictive models. The output variable (y) used for model training was determined by study group, where y = 0 for children with CND and y = 1 for children with NSCNs. The model parameters (*β*₀, *β*₁, ..., *β*₁₂) were found by performing regression analyses with three supervised machine learning algorithms: linear regression with elastic net regularization, SVM regression with a linear kernel, and logistic regression with elastic net regularization. The mathematical formulation for each regression model is presented in Table 2.

### Insert Table 2 about here

Elastic net regularization was used for linear and logistic regression because it improves the accuracy of model predictions by preventing overfitting of the model to the training data. Moreover, elastic net regularization performs variable selection which produces a sparse model for improved interpretability of the model structure. SVM regression has L₂ norm regularization built into its default algorithm. The hyperparameters used to find the model parameters were selected by minimizing the mean square prediction error estimated through five-fold cross validation.

Once the model parameters were found, the standardized eCAP data from each participant were mapped through the model function to obtain a predicted output variable (*yₚ*) for each participant. For linear and SVM regression, *yₚ = **β**^{T}**x** + β*₀*,* where ***β*** = [*β*₁ *β*₂ ... *β*₁₂], *x =* [*x*₁*, x*₂, ... , *x*₁₂]*^{T},* and *^{T}* represents the vector transpose. For logistic regression, *yₚ =* (1 *+ e*^{*-**βTx**-β*0})⁻¹. Finally, the output variable was scaled into the interval [0, 100] to create the CN index.

The CN index was calculated for each participant from the predicted output variable from linear and SVM regression as CN index = 100 * (*yₚ* - *y*ₘᵢₙ)/(*y*ₘₐₓ - *y*ₘᵢₙ), where *y*ₘₐₓ and *y*ₘᵢₙ are the maximum and minimum predicted output variables across all participants, respectively. For logistic regression, CN index = 100 * *yₚ.*

### Statistical Analysis

Statistical modeling and analysis for this study was performed using MATLAB (Mathworks Inc., version 2019b) software. The trust-region-reflective algorithm was used to estimate parameters of the mathematical functions used in statistical modeling. The one-way analysis of variance (ANOVA) with the Tukey's honest significant difference (HSD) post-hoc test was used to compare each eCAP parameter among study groups and across electrode locations. ANOVA and Tukey's HSD criterion were also used to compare CN indices across study groups. One-tailed Pearson correlation analysis was used to evaluate the association between CN indices and CNC word scores measured in adult participants. The one-tailed Spearman rank correlation test was used to evaluate the association between CN indices and AzBio sentence scores measured in adult participants because the AzBio sentence scores were not normally distributed. All statistical analyses were performed at the 95% confidence level.

### RESULTS

### eCAP Parameters

The mean and standard deviations of eCAP parameters used in the models for all three study groups recorded at three electrode locations are shown in Figure 3. As observed in the figure, the CND group has higher *t*₀ and eCAP threshold values, smaller slopes of the I/O function, and longer N1 latencies at all electrode locations when compared to the other two study groups. There was a significant difference in these eCAP parameters among study groups at all electrode locations (F_{(2,77)} ≥ 5.62, p≤0.005). Statistically significant post-hoc comparisons between study groups are indicated with asterisks in Figure 3.

### Insert Figure 3 about here

Another observed trend for the CND group is that *t*₀ and threshold values tend to increase as the electrode location moves from the basal to more apical regions of the cochlea. Statistical analyses confirmed significantly larger *t*₀ (p=0.002) and higher threshold (p=0.002) values recorded at the apical electrode site than at the basal electrode site for children with CND. Trends that existed for the adult group included decreasing sample means of *t*₀ and N1 latency, as the electrode site moved from the base to the apex. In agreement with these observed trends, *t*₀ values were significantly smaller and N1 latencies were significantly shorter at the apical electrode than at the basal electrode (p=0.002 and p=0.007, respectively) for the adult CI users. No trend in the data was readily observed for the NSCN group. Details of statistical findings of each study group when comparing the eCAP parameters across electrode locations are listed in Table 3.

### Insert Table 3 about here

### Model Structure

The model parameters for each of the three predictive models are provided in Table 4. Each regression algorithm found model parameters which were substantially different from one another. However, *β*₂ was the largest in magnitude for all three models, when excluding the model intercept term (*β*₀).

### Insert Table 4 about here

### CN Index

The CN index calculated using each predictive model is shown for each participant in Figure 4. A line is drawn to indicate results of each participant across models. First of all, it is clearly seen that CN indices for all children with CND, with one notable exception (CND12), is smaller than CN indices for children with NSCNs for each model. It is also apparent that the adult participants, as a group, had CN indices that are comparable to the children with NSCNs and greater than children with CND. There was a significant difference in CN index between study groups for results of all models (Linear: F_{(2,77)} = 136.11, p<0.001; SVM: F_{(2,77)} = 136.12, p<0.001; Logistic: F_{(2,77)} = 534.23, p<0.001). Results from multiple comparisons using Tukey's HSD criterion showed that CN indices for children with CND were significantly smaller than CN indices for children with NSCNs and adult participants for results of all three models (p<0.001 for all comparisons). There was not a significant difference between the adult group and children with NSCNs for CN indices calculated using any of the models (Linear: p=0.91, SVM: p=0.99, Logistic: p=0.91).

### Insert Figure 4 about here

We also observe that the relative order of CN index among individual participants within each study group (i.e., rank) is generally consistent across models. While some lines cross each other, CN indices calculated using different models are generally in the same region. The change in individual rank between models, averaged across each group of study participants, was 2.25 (SD: 1.70) for children with CND, 5.19 (SD: 3.48) for children with NSCNs, and 2.75 (SD: 1.96) for adults.

### Speech Perception Scores

Figure 5 shows the results of speech perception tests as a function of CN index calculated using each of the three models for all adults who participated in the study. Results of correlation analyses are included in the lower right-hand corner of each panel. Overall, correlation coefficients ranged from 0.49 to 0.73, showing that higher CN indices were associated with better performance on the speech perception tests. Results of each of the correlation tests were statistically significant and were similar for CN indices calculated using different models.

### Insert Figure 5 about here

### DISCUSSION

### eCAP Parameters

Results of this study showed that children with CND had significantly longer absolute refractory periods, higher eCAP thresholds, flatter slopes of I/O functions, and longer N1 latencies than children with normal-size CNs. These results are consistent with those reported in He et al. (2018, 2019a) and Xu et al. (2019).

### Model Structure

### Weighting Coefficients

The relative magnitude of standardized regression coefficients can be used as a measure of the importance of each input variable in predicting the output variable (Mehmood et al., 2010). Therefore, the magnitudes of the model parameters that scale the eCAP parameters (i.e., *β*₁ - *β*₁₂) represent the relative importance of each eCAP parameter in creating the CN index. As seen in Table 4, the regression coefficient for the eCAP threshold at the electrode location with the lowest level of expected neural function (i.e., *β*₂) had the highest magnitude among all other regression coefficients (excluding the offset term *β*₀) in all three models. This suggests that the eCAP threshold is an important indicator for CN function, especially in regions of poorer neural function. This expectation is supported by an animal study which showed that rats with smaller densities of SGNs had higher response thresholds than normal control rats (Shepherd et. al, 2004). However, Pfingst et al. (2015b) presented results that eCAP thresholds did not predict neural survival in five guinea pigs with various degrees of SGN densities. Factors accounting for the discrepancy in these study results are unclear but may include small sample sizes tested in Pfingst et al. (2015b) and differences in species tested among studies.

### Machine Learning Algorithms

This study utilized the supervised machine learning algorithms of linear regression, logistic regression, and SVM regression. These techniques have been found to be useful in predicting CI outcomes (Tan et al., 2015; Ramos-Miguel et al., 2015; Guerra-Jimenez et al., 2016; Feng et al., 2018). In this study, machine learning techniques were used to create predictive models for estimating the functional status of the CN. Each algorithm produced CN indices for the adult study group that were significantly correlated with speech perception scores (Figure 5). Additionally, the relative ranking of CN index between participants of all study groups was consistent across models (Figure 4). This consistency provides strong support for the validity of the overall concept and the robustness of the approach used in this study.

While not the primary focus of this study, the machine learning algorithms utilized in this study can also be used as classification algorithms, with minor modification (see Text, Supplemental Digital Content 1, which describes the methodology). Each algorithm performs very well with classification accuracies of 91-95% (see Table A1, Supplemental Digital Content 1, which details the performance of each machine learning algorithm in classifying children with CND and children with NSCNs). This accuracy is comparable with the best classification algorithms reported for CI studies which range from 49-94% (Tan et al., 2015; Ramos-Miguel et al., 2015; Guerra-Jimenez et al., 2016; Feng et al., 2018).

### CN Index

### Pediatric Study Groups

This study tested the hypothesis that the predictive models created in this study would accurately stratify individual patients based on the functional status of the CN. We expected that distributions of CN index for children with CND and children with NSCNs would be distinct. Our results showed a clear separation in CN index between these two participant groups regardless of the machine learning algorithm used. These results were consistent with the study hypothesis and followed the expected data trend.

A much larger range of CN functional statuses were predicted by the linear and SVM regression model for children with CND compared to children with NSCNs, as shown by the CN index values (Figure 4). The predicted status for children with CND ranged from very poor to good functional status. This result agrees with studies that have reported large ranges in CI outcomes for children with CND (e.g., Young et al., 2012; Vincenti et al., 2014; Birman et al., 2016; Han et al., 2019). Specifically, some children with CND had no awareness of environmental sounds with Cls, while a few patients could understand speech without visual cues (Young et al., 2012; Vincenti et al., 2014; Birman et al., 2016; Han et al., 2019). Considering that CND is likely caused by arrested inner ear development during embryogenesis (Jackler et al. 1987), varying degrees of development of the CN would be expected depending on when the inner ear stops developing. This is supported by a recent study which reported large differences in the number of electrodes with recordable eCAPs in children with CND (He et al., 2018). Results of that study showed that some children with CND had recordable eCAPs at all electrode locations while eCAPs could not be measured at any electrode location in several patients tested in that same study. In contrast, eCAPs were recorded at all electrode locations for all children with NSCNs. Therefore, a wider range of CN functional status for children with CND compared to children with NSCNs, as estimated by the CN index in this study, would be expected.

One patient (CND12) had a predicted functional status within the range of children with NSCNs. Therefore, this particular patient would be expected to have outcomes similar to typical CI users. Supporting this expectation, this particular patient has developed open-set speech skills. We are currently following up with other pediatric participants tested in this study for their speech and language skill development.

### Adult Study Group

For adult study participants, we expected that CN indices would be generally smaller than those for children with NSCNs but greater than those for children with CND. As we expected, the results showed that the adults had significantly greater CN index values than children with CND. However, the average CN index for the adult study group was not significantly different from the average CN index for children with NSCNs. The lack of statistical significance may be due to high CN indices measured in young adult CI users.

As a preliminary investigation into the relationship between CN function and age, we compared CN indices between the youngest and oldest adults. Specifically, CN indices were compared for the five youngest adults (Participants: A3, A7, A9, A10, and A15; Mean age at testing: 44.16, SD 11.48 years), the five oldest adults (Participants: A5, A14, A16, A17, and A19; Mean age at testing: 78.52, SD 2.61 years) and all of the children with NSCNs. Participants A5 and A7 are bilateral CI users, so a total of six ears were included in both the youngest and oldest adult groups for these comparisons. The means and standard deviations of CN indices calculated from each of the predictive models are shown in Figure 6 for children with NSCNs, the youngest adult participants, and the oldest adult participants.

### Insert Figure 6 about here

As observed in the figure, the mean CN index value was similar for the youngest adults and the children with NSCNs, both of which were larger than that of the oldest adults for results of all three models. There was a significant difference in CN index among these groups for results of all three models (Linear: F_{(2,41)} = 4.44, p=0.018, SVM: F_{(2,41)} = 3.61, p=0.036, logistic F_{(2,41)} = 4.93, p=0.012). Multiple comparisons with Tukey's HSD criterion indicated that the oldest adult group had statistically smaller CN indices than children with NSCNs for the linear (p=0.017) and logistic (p=0.001) models, but not for the SVM model (p=0.053). The youngest adults had statistically greater CN indices than the oldest adults for the SVM model (p=0.046), but not for the linear or logistic model (p=0.050 and p=0.055, respectively). Finally, there was no statistical difference in CN index between the youngest adult group and children with NSCNs for results of any of the predictive models (Linear: p=0.962, SVM: p=0.702, Logistic: p=1.000).

The results of this exploratory investigation supports the idea that the wide range of age at testing (28.73 - 88.80 years) of adult participants may partially account for the non-significant difference in CN indices between the NSCN and the adult study group. Moreover, these data suggest that older patients have worse CN function (as indicated by smaller CN indices), which agrees with other studies showing deteriorating CN function with advanced age (e.g., McFadden et al., 1997; Makary et al., 2011; Viana et al., 2015; Wu et al., 2019). A comprehensive analysis of the effect of aging on CN function is currently under investigation as a separate study. Nevertheless, the present investigation provides additional support for the idea that the CN index developed in this study reflects CN functional status.

We also expected that CN index would be positively correlated with speech outcome measures. Confirming this expectation, CN index was positively and significantly correlated with speech perception of CNC words and AzBio sentences in quiet (Figure 5). Significant correlations were observed for CN indices calculated using each predictive model. This result supports the idea that the CN index represents overall CN function because CI outcomes are related to CN function (Kim et al., 2010; Zhou & Pfingst, 2014; Schvartz-Leyzac & Pfingst, 2018; He et al., 2018). Furthermore, this result shows the utility and benefit of employing machine learning approaches to predict CI outcomes, which may have implications for patient treatment and counseling.

### Study Limitations

One potential study limitation is the assumption that patterns of neural function for an individual patient follow the trend of its patient population. Specifically, the CN index is derived with the assumption that children with CND have better CN function in basal regions compared to apical regions, and vice versa for children with NSCNs and adult patients. While the literature confirms these overall trends (e.g., Propst et al., 2006; Gordon et al., 2007; Brill et al., 2009; Hughes et al., 2009; He et al., 2018), individual variations exist. Any deviations from the expected pattern will affect the CN index calculation. This limitation could be eliminated with a future optimized model that predicts CN function at individual electrode sites.

Another potential limitation is that the eCAP is a neurophysiological response that depends on a sufficient number of CN fibers responding synchronously to electrical stimulations. Therefore, it is a composite measure of overall CN neural survival and the integrity of individual CN fibers. As such, it is not known if poorer eCAP responses are due to a decreased number of CN fibers or due to degeneration of existing CN fibers. Moreover, this may be different across patient populations. For example, eCAP responses might primarily be affected by the few number of intact CN fibers for children with CND, whereas, children with NSCNs and adult patients may have a large number of degenerated CN fibers. Currently, the effect of various pathologies on eCAP responses is not well understood. Nevertheless, the eCAP is a useful response for characterizing the functional status of the CN.

Finally, the models created in this study are not able to predict CN function at individual electrode sites. Rather, the models presented in this study predict overall CN function across multiple electrodes. We are currently investigating methods for predicting neural function at individual electrode sites, which may be useful in programming individual patient CI settings.

### CONCLUSIONS

This study presented models created using three supervised machine learning techniques that generate an index for the functional status of the CN based on eCAP recordings for individual patients. All three models successfully stratified CI patients based on their CN functional statuses. Specifically, children with NSCNs had significantly better predicted CN functions than children with CND. Adult CI users had a range of predicted CN functions that were positively and significantly correlated with scores on speech perception tests. Results of this study suggested that these models may be useful for developing objective clinical tools for optimizing CI programming settings and predicting CI outcomes for individual CI patients.

### REFERENCES

Abbas, P. J., Brown, C. J., Shallop, J. K., et al. (1999). Summary of results using the nucleus CI24M implant to record the electrically evoked compound action potential. Ear Hear, 20, 45-59.
Abbas, P. J., Hughes, M., Brown, C. J., et al. (2004). Channel Interaction in Cochlear Implant Users Evaluated Using the Electrically Evoked Compound Action Potential. Audiol Neurootol. 9. 203-13.
Adunka, O. F., Roush, P. A., Teagle, H. F., et al. (2006). Internal auditory canal morphology in children with cochlear nerve deficiency. Otol Neurotol, 27, 793-801.
Beyea, J. A., Mcmullen, K. P., Harris, M. S., et al. (2016). Cochlear Implants in Adults. Otol Neurotol, 37, 1238-1245.
Birman, C. S., Powell, H. R., Gibson, W. P., et al. (2016). Cochlear implant outcomes in cochlea nerve aplasia and hypoplasia. Otol Neurotol, 37, 438-445.
Blamey, P., Artieres, F., Ba 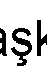 kent, D., et al. (2013). Factors affecting auditory performance of postlinguistically deaf adults using cochlear implants: an update with 2251 patients. Audiol Neurootol, 18(1), 36-47.
Botros, A., Psarros, C. (2010). Neural Response Telemetry Reconsidered: II. The Influence of Neural Population on the ECAP Recovery Function and Refractoriness. Ear Hear, 31, 380-391.
Botros, A., van Dijk., B., Killian M. (2007). AutoNR: An automated system that measures ECAP thresholds with the Nucleus Freedom cochlear implant via machine intelligence. Artif Intell Med. 40:15-28.
Brill, S., Müller, J., Hagen, R., et al. (2009). Site of cochlear stimulation and its effect on electrically evoked compound action potentials using the MED-EL standard electrode array. BioMed Eng OnLine 8, 40.
Brown, C. J., Abbas, P. J., Gantz, B. (1990). Electrically evoked whole-nerve action potentials: data from human cochlear implant users. J Acoust Soc Am, 88, 1385-1391.
Brown, C. J., Abbas, P. J., Gantz, B. (1998). Preliminary experience with Neural Response Telemetry in the Nucleus CI24M cochlear implant. Am. J. Otol. 19, 320-327.
Buchman, C. A., Roush, P. A., Teagle, H. F., et al. (2006). Auditory neuropathy characteristics in children with cochlear nerve deficiency. Ear Hear, 27, 399-408.
Clemmens, C. S., Guidi, J., Caroff, A., et al. (2013). Unilateral cochlear nerve deficiency in children. Otolaryngol Head Neck Surg, 149, 318-325.
Crowson, M. G., Lin, V., Chen, J. M., & Chan, T. C. (2020). Machine Learning and Cochlear Implantation-A Structured Review of Opportunities and Challenges. Otol Neurotol. 41(1), e36-e45.
Dryden, A., Allen, H.A., Henshaw, H., et al. (2017). The association between cognitive performance and speech-in-noise perception for adult listeners: a systematic literature review and meta-analysis. Trend Hear, 21, 1-21.
Feng, G., Ingvalson, E. M., Grieco-Calub, T. M., et al. (2018). Neural preservation underlies speech improvement from auditory deprivation in young cochlear implant recipients. Proc Natl Acad Sci USA. 115:E1022-31.
Firszt J. B., Holden L. K., Skinner M. W., et al. (2004). Recognition of speech presented at soft to loud levels by adult cochlear implant recipients of three cochlear implant systems. Ear Hear, 25, 375-87.
Fulmer, S. L., Runge, C.L., Jensen, J.W., et al. (2010). Rate of Neural Recovery in Implanted Children with Auditory Neuropathy Spectrum Disorder. JAMA Otolaryngol Head Neck Surg, 144, 274-279.
Garadat, S. N., Zwolan, T. A., & Pfingst, B. E. (2012). Across-site patterns of modulation detection: relation to speech recognition. J Acoust Soc Am, 131(5), 4030-4041.
Gartner, L., Lenarz, T., Joseph, G., Buchner, A. (2010). Clinical use of a system for the automated recording and analysis of electrically evoked compound action potentials (ECAPs) in cochlear implant patients. Acta Otolaryngol. 130:724-32.
Glastonbury, C. M., Davidson, H. C., Harnsberger, H. R., Butler, J., Kertesz, T. R. and Shelton, C. (2002). Imaging findings of cochlear nerve deficiency. AJNR Am J Neuroradiol, 23(4), pp.635-643.
Gordon, K., Papsin, B., Harrison, R. (2007). Auditory brainstem activity and development evoked by apical versus basal cochlear implant electrode stimulation in children. Clin Neurophysiol, 118, 1671-1684.
Guerra-Jimenez, G., Ramos De Miguel, A., Falcon Gonzalez, J. C. et al. (2016). Cochlear implant evaluation: Prognosis estimation by data mining system. J Int Adv Otol. 12:1-7.
Han, J. J., Suh, M. W., Park, M. K., et al. (2019). A Predictive Model for Cochlear Implant Outcome in Children with Cochlear Nerve Deficiency. Sci Rep, 9(1), 1154.
He, S., Shahsavarani, B. S., McFayden, T. C., et al. (2018). Responsiveness of the electrically stimulated cochlear nerve in children with cochlear nerve deficiency. Ear Hear, 39, 238-250.
He, S., Xu, L., Skidmore, J., et al. (2019a). The effect of interphase gap on neural response of the electrically-stimulated cochlear nerve in children with cochlear nerve deficiency and children with normal-sized cochlear nerves. Ear Hear, Oct. 31 (Epub ahead of print).
He, S., Chao, X.H., Wang, R.J., et al. (2019b). Recommendations for measuring the electrically evoked compound action potential in children with cochlear nerve deficiency. Ear Hear, Sep. 13 (Epub ahead of print).
He, S., Teagle, H.F.B., Buchman, C. (2017). The electrically evoked compound action potential: from laboratory to clinic. Front Neurosci, 11:339.
Holden, L.K., Finley, C.C., Firszt, J.B., et al. (2013). Factors affecting open-set word recognition in adults with cochlear implants. Ear Hear, 34, 342-360.
Hughes, M. L., & Stille, L. J. (2009). Psychophysical and physiological measures of electrical-field interaction in cochlear implants. J. Acoust. Soc. Am., 125(1), 247-260.
Jackler, R. K., Luxford, W. M., House, W. F. (1987). Congenital malformations of the inner ear: a classification based on embryogenesis. Laryngoscope, 97(3 Pt 2; Suppl 40), 2-14.
Kang, W. S., Lee, J. H., Lee, H. N., & Lee, K. S. (2010). Cochlear implantations in young children with cochlear nerve deficiency diagnosed by MRI. Otolaryngology-Head and Neck Surgery, 143(1), 101-108.
Kim, J.R., Abbas, P.J., Brown, C.J., et al. (2010). The relationship between electrically evoked compound action potential and speech perception: a study in cochlear implant users with short electrode array. Otol Neurotol, 31, 1041-1048.
Kirby, A. E., & Middlebrooks, J. C. (2010). Auditory temporal acuity probed with cochlear implant stimulation and cortical recording. J Neurophysiol, 103(1), 531-542.
Kirby, A. E., & Middlebrooks, J. C. (2012). Unanesthetized auditory cortex exhibits multiple codes for gaps in cochlear implant pulse trains. J Assoc Res Otolaryngol, 13(1), 67-80.
Kutz, J. W. Jr, Lee, K. H., Isaacson, B., et al. (2011). Cochlear implantation in children with cochlear nerve absence or deficiency. Otol Neurotol, 32, 956-961.
Lazard, D.S., Vincent, C., Venail, F., et al. (2012). Pre-, per- and postoperative factors affecting performance of postlinguistically deaf adults using cochlear implants: a new conceptual model over time. PLoS One, 7, e48739.
Lee, E.R., Friedland, D.R., Runge, C.L. (2012). Recovery From Forward Masking in Elderly Cochlear Implant Users. Otol Neurotol, 33, 355-363.
Long, C. J., Holden, T. A., McClelland, G. H., et al. (2014). Examining the electro-neural interface of cochlear implant users using psychophysics, CT scans, and speech understanding. J Assoc Res Otolaryngol, 15(2), 293-304.
Luo, J.F., Xu, L., Chao, X.H., et al. (2019). The effects of GJB2 and SLC26A4 gene mutations on neural response of the electrically-stimulated auditory nerve in children. Ear Hear, (in press).
Makary, C. A., Shin, J., Kujawa, S. G., et al. (2011) Age-Related Primary Cochlear Neuronal Degeneration in Human Temporal Bones. JARO 12, 711-717.
McFadden, S .L., Campo, P., Quaranta, N., et al. (1997). Age-related decline of auditory function in the chinchilla (Chinchilla laniger). Hear. Res., 111, 114-126.
Mehmood, T., Liland, K. H., Snipen, L., et al. (2012). A review of variable selection methods in Partial Least Squares Regression. Chemometrics and Intelligent Laboratory Systems, 118, 62-69.
Miller, C. A., Abbas, P. J., Brown, C. J. (2000). An Improved Method of Reducing Stimulus Artifact in the Electrically Evoked Whole-Nerve Potential. Ear Hear, 21, 280-290.
Moberly, A.C., Bates, C., Harris, M.S., et al. (2016). The Enigma of Poor Performance by Adults with Cochlear Implants. Otol Neurotol, 37, 1522-1528.
Morsnowski, A., Charasse, B., Collet, L., et al. (2006). Measuring the Refractoriness of the Electrically Stimulated Auditory Nerve. Audiol Neurootol, 11, 389-402.
MSTB (2011). Minimum speech test battery for adult cochlear implant users. http://auditorypotential.com/MSTBfiles/MSTBManual2011-06-20.pdf. Accessed Nov 12, 2019.
Nuesse, T., Steenken, R., Neher, T., et al. (2018). Exploring the link between cognitive abilities and speech recognition in the elderly under different listening conditions. Front Psychol, 9, 678.
Peterson, G. E., & Lehiste, I. (1962). Revised CNC lists for auditory tests. J Speech Hear Disord., 27(1), 62-70.
Pfingst, B.E., Colesa, D.J., Watts, M.M., et al. (2017). Neurotrophin gene therapy in deafened ears with cochlear implants: long-term effects on nerve survival and functional measures. J Assoc Res Otolaryngol, 18, 731-750.
Pfingst, B.E., Hughes, A.P., Colesa, D.J., et al. (2015a). Insertion trauma and recovery of function after cochlear implantation: evidence from objective functional measures. Hear Res, 330, 98-105.
Pfingst, B. E., Zhou, N., Colesa, D. J., et al. (2015b). Importance of cochlear health for implant function. Hear Res, 322, 77-88.
Prado-Guitierrez, P., Fewster, L.M., Heasman, J.M., et al. (2006). Effect of interphase gap and pulse duration on electrically evoked potentials is correlated with auditory nerve survival. Hear Res, 215, 47-55.
Propst, E.J., Papsin, B.C., Stockley, T.L., et al. (2006). Auditory responses in cochlear implant users with and without GJB2 deafness. Laryngoscope, 116, 317-327.
Ramekers, D., Versnel, H., Strahl, S.B., et al. (2014). Auditory-nerve response to varied inter-phase gap and phase duration of the electric pulse stimulus as predicators for neuronal degeneration. J Assoc Res Otolaryngol, 15, 187-202.
Ramos-Miguel, A., Perez-Zaballos, T., Perez, D., et al. (2015). Use of data mining to predict significant factors and benefits of bilateral cochlear implantation. Eur Arch Otorhinolaryngol. 272:3157-62.
Schvartz-Leyzac, K. C., Pfingst, B. E. (2018). Assessing the relationship between the electrically evoked compound action potential and speech recognition abilities in bilateral cochlear implant recipients. Ear Hear, 39, 344-358.
Shepherd, R. K., Roberts, L. A., Paolini, A. G. (2004). Long-term sensorineural hearing loss induces functional changes in the rat auditory nerve. Eur J Neurosci, 20, 3131-3140.
Spahr, A. J., Dorman, M. F., Litvak, L. M., et al. (2012). Development and validation of the AzBio sentence lists. Ear Hear, 33(1), 112.
Tan, L., Holland, S. K., Deshpande, A. K., et al. (2015). A semi-supervised Support Vector Machine model for predicting the language outcomes following cochlear implantation based on preimplant brain fMRI imaging. Brain Behav. 5:1-25.
Teagle, H. F., Roush, P. A., Woodard, J. S., et al. (2010). Cochlear implantation in children with auditory neuropathy spectrum disorder. Ear Hear, 31, 325-335.
Van de Heyning, P., Arauz, S. L., Atlas, M., et al. (2016). Electrically evoked compound action potentials are different depending on the site of cochlear stimulation. Cochlear Implants Int, 17, 251-262.
van Dijk, B., Botros, A. M., Battmer R. D., et al. (2007). Clinical results of AutoNRT™, a completely automatic ECAP recording system for cochlear implants. Ear Hear. 28:558-70.
Viana, L. M., O'Malley, J. T., Burgess, B. J., et al. (2015). Cochlear neuropathy in human presbycusis: Confocal analysis of hidden hearing loss in post-mortem tissue. Hear Res, 327, 78-88.
Vincenti, V., Ormitti, F., Ventura, E., et al. (2014). Cochlear implantation in children with cochlear nerve deficiency. Int J Pediatr Otorhinolaryngol, 78, 912-917.
Wei, X., Li, Y., Chen, B., et al. (2017). Predicting auditory outcomes from radiological imaging in cochlear implant patients with cochlear nerve deficiency. Otol Neurotol, 38(5), 685-693.
Wiemes, G. R. M., Hamerschmidt, R., Moreira, A. T. R., et al. (2016). Auditory Nerve Recovery Function in Cochlear Implant Surgery with Local Anesthesia and Sedation versus General Anesthesia. Audiol Neurootol, 21, 150-157.
Wu, P. Z., Liberman, L. D., Bennett, K., et al. (2019). Primary Neural Degeneration in the Human Cochlea: Evidence for Hidden Hearing Loss in the Aging Ear. Neuroscience, 407, 8-20.
Xu, L., Skidmore, J., Luo, J., et al. (2019). The effect of pulse polarity on neural response of the electrically-stimulated cochlear nerve in children with cochlear nerve deficiency and children with normal-sized cochlear nerves. Ear Hear. (Accepted)
Young, N. M., Kim, F. M., Ryan, M. E., et al. (2012). Pediatric cochlear implantation of children with eighth nerve deficiency. Int J Pediatr Otorhinolaryngol, 76, 1442-1448.
Zekveld, A., Kramer, S.E., & Festen, J. (2011). Cognitive load during speech perception in noise: the influence of age, hearing loss, and cognition on pupil response. Ear Hear, 32, 498-510.
Zhou, N., Pfingst, B.E. (2014). Relationship between multipulse integration and speech recognition with cochlear implants. J Acoust Soc Am, 136, 1257-1268.

### FIGURES

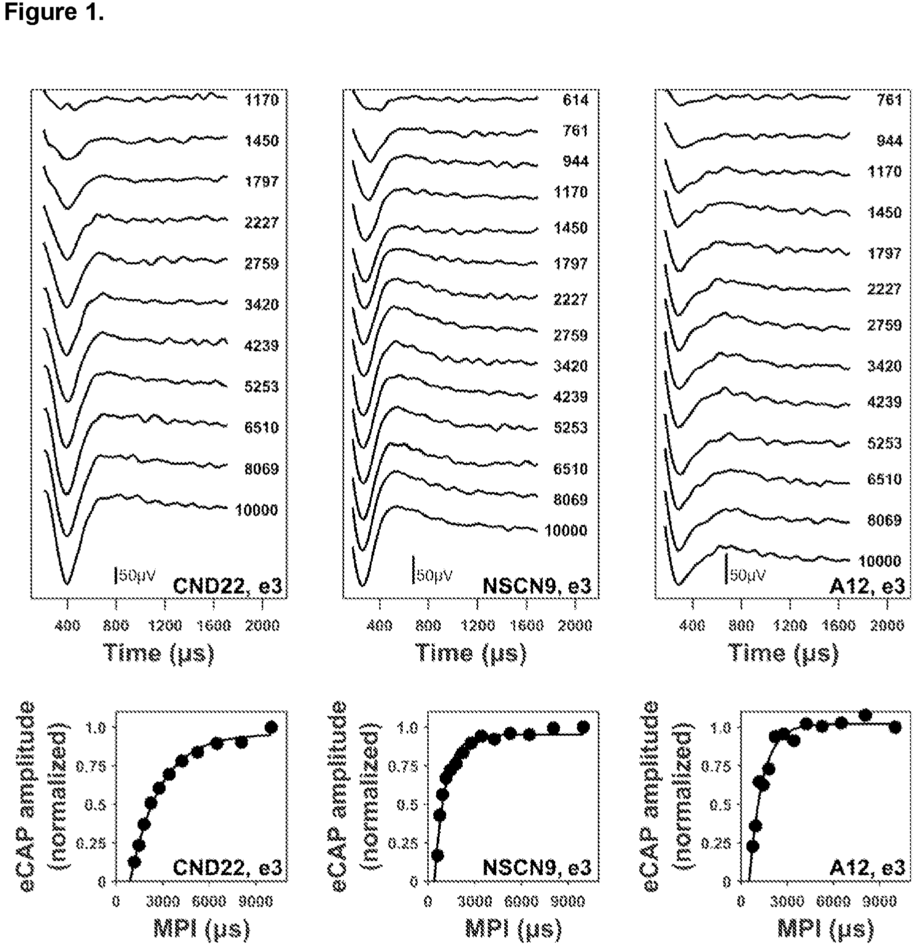

Upper panels: electrically-evoked compound action potential (eCAP) waveforms measured at different masker-probe intervals (MPIs) for stimulating electrode 3 in one child with cochlear nerve deficiency (CND; CND22), one child with normal-sized cochlear nerve (NSCN; NSCN9), and one adult (A12). eCAPs are arranged based on MPI duration, with responses evoked by short MPIs displayed at the top. Each waveform is labeled with the corresponding MPI duration in µs. Lower panels: refractory recovery functions (round symbols) obtained from the waveforms in the upper panels. The fitted exponential decay function for each refractory recovery function (black line) is also provided. The participant and electrode number are included in the lower right corner of each panel. The stimulations were performed at the maximum comfortable level for each participant and electrode.

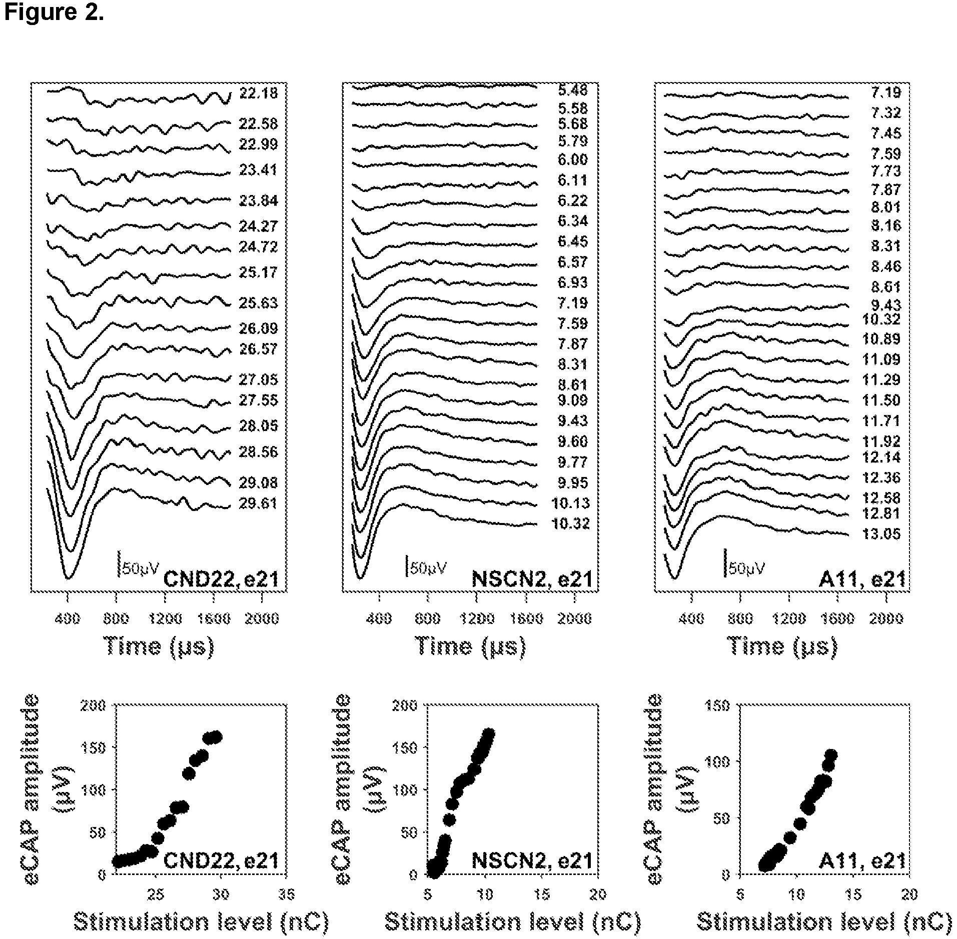

Upper panels: electrically-evoked compound action potential (eCAP) waveforms measured at different stimulation intensities for electrode 21 in one child with cochlear nerve deficiency (CND; CND22), one child with normal-sized cochlear nerve (NSCN; NSCN2), and one adult (A11). eCAPs are arranged based on stimulation level, with responses evoked by the smallest stimulation level (i.e., eCAP threshold) displayed at the top. Each waveform is labeled with the corresponding probe stimulation level in nanocoulombs (nC). The largest stimulation level presented was the maximum comfortable level (i.e., C level). Lower panels: eCAP input/output functions (round symbols) obtained from the waveforms in the upper panels. The participant and electrode number are included in the lower right corner of each panel.

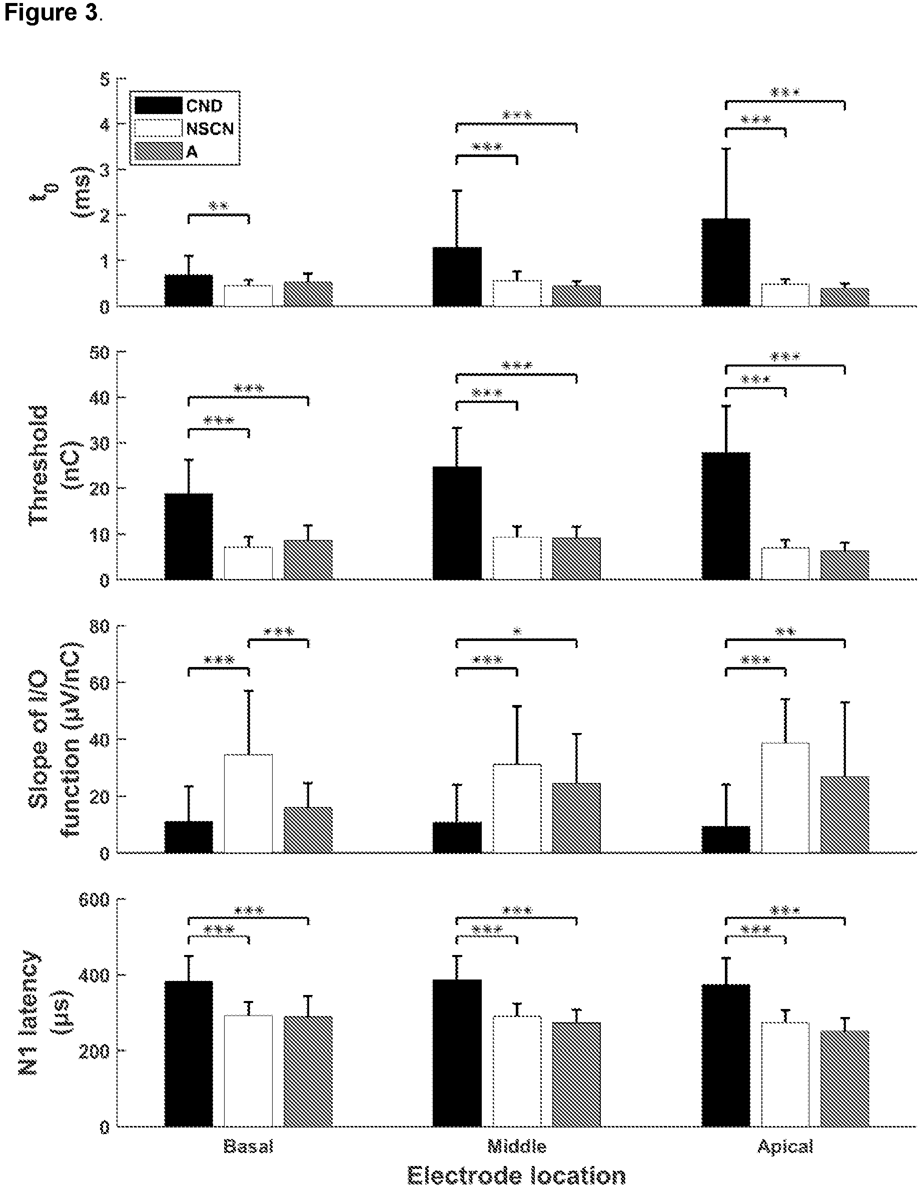

Results of eCAP parameters (mean and standard deviation) measured for three study groups. Ordered in rows from top to bottom are the estimated absolute refractory recovery times (i.e., *t*₀), eCAP thresholds, slopes of eCAP I/O functions, and N1 peak latencies. Results measured at the basal, middle and apical electrode location are provided in the left, middle and right columns, respectively. Statistically significant group comparisons are indicated with asterisks. *: p<0.05, **: p<0.01, ***: p<0.001.

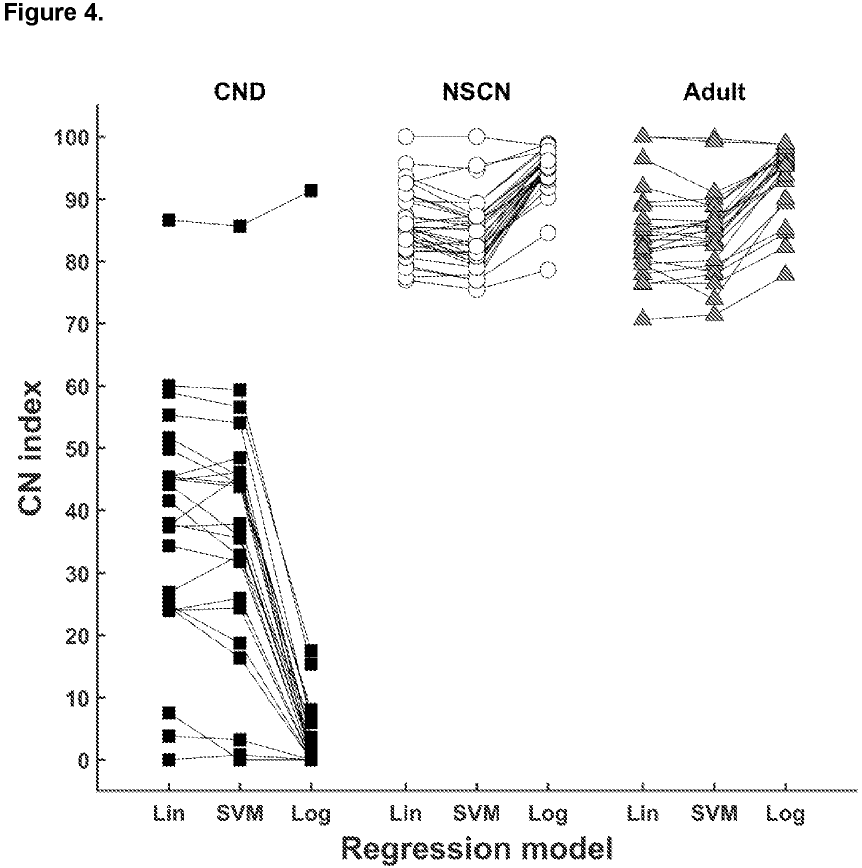

Results of cochlear nerve (CN) indices obtained from linear (Lin) regression, support vector machine (SVM) regression, and logistic (Log) regression for three study groups.

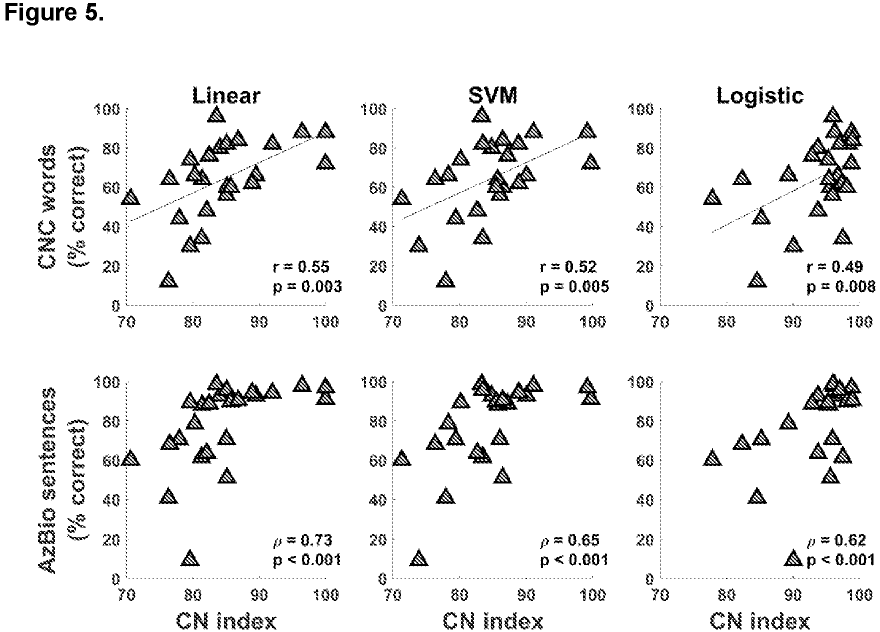

Results of speech perception of Consonant-Nucleus-Consonant (CNC) words (top row) and AzBio sentences (bottom row) as a function of cochlear nerve (CN) index for all adult participants. Results are displayed for CN indices obtained from linear regression, support vector machine (SVM) regression, and logistic regression. Results of Pearson and Spearman correlation tests are also provided in the bottom right corner of all panels.

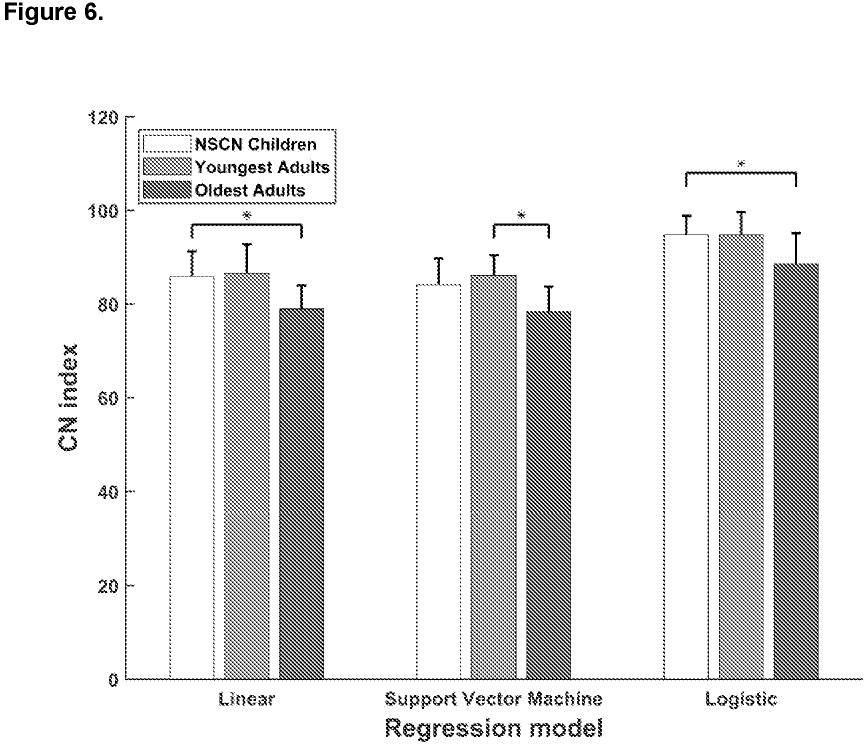

The means and standard deviations of cochlear nerve (CN) indices calculated from three predictive models for children with normal-sized CNs, the youngest adult participants, and the oldest adult participants. Statistically significant differences (p<0.05) are indicated by an asterisk.

### TABLES

**TABLE 1. Demographic information of all subjects who participated in this study**

| Subject Number | Sex | Ear Tested | AAI (yrs) | AAT (yrs) | Electrodes Tested | PPD (µs) | Internal Device and Electrode Array | MRI Result | |
|---|---|---|---|---|---|---|---|---|---|
| | | | | | | | | CN | Cochlea |
| CND1 | F | L | 1.2 | 2.5 | 1, 5, 10 | 50, 50, 50 | 24RE (CA) | * | IP-2 |
| CND2 | M | R | 2.1 | 4.1 | 1, 5, 8 | 50, 50, 50 | 24RE (CA) | * | Normal |
| CND3 | M | R | 6.6 | 8.5 | 3, 6, 10 | 50, 50, 50 | 24RE (CA) | # | Normal |
| CND4 | F | L | 1.3 | 3.3 | 3, 12, 21 | 50, 50, 50 | 24RE (CA) | * | Normal |
| CND5 | F | L | 3.4 | 5.4 | 3, 10, 18 | 50, 50, 50 | 24RE (CA) | * | Normal |
| CND6 | M | L | 1.9 | 2.4 | 1, 7, 14 | 50, 50, 50 | 24RE (CA) | * | Normal |
| CND7 | M | L | 4.7 | 10.8 | 15, 19, 22 | 37, 37, 37 | 24RE (ST) | Small | IP-2 |
| CND8 | F | L | 1.69 | 3.81 | 6, 12, 18 | 75, 75, 88 | 24RE (CA) | Small | Normal |
| CND9 | F | L | 2.1 | 8.2 | 3, 11, 16 | 75, 50, 50 | 24RE (CA) | Absent | Normal |
| CND10 | F | L | 2.10 | 5.50 | 2, 4, 10 | 75, 100, 100 | 24RE (CA) | # | Normal |
| CND11 | M | L | 7.84 | 10.03 | 3, 12, 21 | 75, 75, 75 | 24RE (CA) | Small | Normal |
| CND12 | M | L | 2.10 | 4.50 | 3, 12, 17 | 25, 25, 25 | 24RE (CA) | # | Normal |
| CND13 | M | L | 9.40 | 9.71 | 3, 12, 18 | 50, 50, 50 | 24RE (CA) | Small | Normal |
| CND14 | F | L | 5.10 | 7.20 | 3, 9, 15 | 88, 88, 88 | CI512 | Small | Normal |
| CND15 | M | L | 4.10 | 7.90 | 1, 6, 9 | 50, 50, 50 | 24RE (CA) | Absent | Normal |
| CND16 | M | R | 3.76 | 4.09 | 1, 6, 9 | 75, 75, 75 | 24RE (CA) | Small | Normal |
| CND17 | M | R | 2.50 | 4.76 | 6, 15, 21 | 50, 50, 50 | 24RE (CA) | # | Normal |
| CND18 | F | R | 3.96 | 4.96 | 3, 12, 21 | 37, 37, 37 | 24RE (CA) | * | Normal |
| CND19 | F | R | 2.93 | 6.49 | 1, 5, 8 | 50, 50, 50 | 24RE (CA) | * | Normal |
| CND20 | F | R | 2.10 | 4.20 | 1, 4, 7 | 50, 50, 50 | 24RE (CA) | Small | Normal |
| CND21 | F | L | 1.90 | 2.50 | 3, 12, 21 | 50, 50, 50 | 24RE (CA) | # | Narrow |
| CND22 | M | L | 2.10 | 4.70 | 3, 12, 21 | 37, 37, 50 | 24RE (CA) | * | Normal |
| CND23L | F | L | 10.83 | 15.03 | 3, 6, 9 | 37, 50, 62 | 24RE (CA) | Small | IP-2 |
| CND23R | F | R | 3.84 | 15.13 | 3, 12, 21 | 50, 50, 50 | 24RE (CA) | Small | IP-2 |
| NSCN1 | F | R | 2.41 | 3.00 | 3, 12, 21 | 25, 25, 25 | CI422 | | |
| NSCN2 | M | L | 3.52 | 4.37 | 4, 12, 21 | 25, 25, 25 | 24RE (CA) | | |
| NSCN3 | F | R | 0.94 | 2.14 | 3, 12, 21 | 25, 25, 25 | | CI422 | |
| NSCN4 | M | R | 2.31 | 6.78 | 3, 12, 21 | 25, 25, 25 | | 24RE (CA) | |
| NSCN5 | M | L | 1.57 | 3.17 | 3, 12, 21 | 25, 25, 25 | | 24RE (CA) | |
| NSCN6 | M | R | 3.60 | 6.28 | 3, 12, 21 | 25, 25, 25 | | 24RE (CA) | |
| NSCN7 | F | R | 3.50 | 6.50 | 3, 12, 21 | 25, 25, 25 | | CI512 | |
| NSCN8 | M | L | 8.50 | 9.35 | 3, 12, 21 | 25, 25, 25 | | 24RE (CA) | |
| NSCN9 | M | L | 4.30 | 8.40 | 3, 12, 21 | 25, 25, 25 | | CI512 | |
| NSCN10 | F | R | 3.50 | 6.50 | 3, 15, 21 | 25, 25, 25 | | CI512 | |
| NSCN11 | F | R | 3.00 | 11.50 | 3, 12, 21 | 25, 25, 25 | | 24RE (CA) | |
| NSCN12 | M | L | 6.52 | 7.73 | 3, 12, 21 | 25, 25, 25 | | 24RE (CA) | |
| NSCN13 | F | R | 2.09 | 4.3 | 3, 12, 21 | 25, 25, 25 | | 24RE (CA) | |
| NSCN14 | F | R | 1.65 | 2.49 | 3, 12, 21 | 25, 25, 25 | | 24RE (CA) | |
| NSCN15 | F | L | 6.32 | 8.54 | 3, 12, 19 | 25, 25, 25 | | 24RE (CA) | |
| NSCN16 | M | R | 0.96 | 2.91 | 3, 12, 19 | 25, 25, 25 | | 24RE (CA) | |
| NSCN17 | M | R | 3.33 | 5.6 | 3, 12, 19 | 25, 25, 25 | | 24RE (CA) | |
| NSCN18 | F | L | 5.71 | 8.08 | 3, 12, 21 | 25, 25, 25 | | 24RE (CA) | |
| NSCN19 | M | L | 2.41 | 3.41 | 3, 12, 19 | 25, 25, 25 | | 24RE (CA) | |
| NSCN20 | M | L | 1.66 | 3.95 | 3, 12, 19 | 25, 25, 25 | | 24RE (CA) | |
| NSCN21 | M | R | 2.69 | 3.02 | 3, 12, 19 | 25, 25, 25 | | 24RE (CA) | |
| NSCN22L | M | L | 1.28 | 2.64 | 3, 12, 19 | 25, 25, 25 | | CI512 | |
| NSCN22R | M | R | 1.28 | 2.64 | 3, 12, 19 | 25, 25, 25 | | 24RE (CA) | |
| NSCN23 | M | R | 1.80 | 3.96 | 3, 12, 19 | 25, 25, 25 | | 24RE (CA) | |
| NSCN24 | M | R | 6.12 | 9.83 | 3, 12, 21 | 25, 25, 25 | | 24RE (CA) | |
| NSCN25L | M | L | 0.96 | 12.94 | 3, 12, 21 | 25, 25, 25 | | 24RE (CA) | |
| NSCN25R | M | R | 0.96 | 12.94 | 4, 12, 21 | 25, 25, 25 | | 24RE (CA) | |
| NSCN26L | M | L | 2.41 | 3.41 | 3, 12, 21 | 25, 25, 25 | | 24RE (CA) | |
| NSCN26R | M | L | 1.66 | 3.95 | 4, 12, 21 | 25, 25, 25 | | 24RE (CA) | |
| NSCN27 | M | R | 3.17 | 6.22 | 4, 12, 21 | 25, 25, 25 | | CI512 | |
| NSCN28 | M | L | 1.28 | 2.64 | 3, 12, 22 | 25, 25, 25 | | CI532 | |
| NSCN29 | F | L | 0.84 | 11.94 | 3, 12, 20 | 25, 25, 25 | | 24RE (CA) | |
| A1 | M | L | 58.85 | 61.79 | 3, 12, 20 | 25, 25, 25 | | CI512 | |
| A2 | M | L | 60.67 | 69.02 | 3, 12, 18 | 25, 25, 25 | CI512 | | |
| A3 | M | R | 43.26 | 52.69 | 3, 12, 21 | 25, 25, 25 | 24RE (CA) | | |
| A4L | F | L | 56.01 | 67.52 | 3, 12, 21 | 25, 25, 25 | 24RE (CA) | | |
| A4R | F | R | 54.42 | 67.57 | 3, 12, 21 | 25, 25, 25 | 24RE (CA) | | |
| A5L | M | L | 72.76 | 80.80 | 7, 12, 21 | 25, 25, 25 | CI512 | | |
| A5R | M | R | 77.54 | 80.70 | 3, 9, 21 | 25, 25, 25 | 24RE (CA) | | |
| A6 | M | R | 52.53 | 61.47 | 3, 12, 21 | 25, 25, 25 | CI512 | | |
| A7L | F | L | 54.63 | 55.36 | 4, 12, 21 | 25, 25, 25 | CI532 | | |
| A7R | F | R | 44.71 | 54.73 | 3, 12, 21 | 25, 25, 25 | 24RE (CA) | | |
| A8 | M | R | 60.31 | 62.75 | 4, 12, 21 | 25, 25, 25 | CI522 | | |
| A9 | M | R | 25.60 | 36.79 | 3, 12, 21 | 25, 25, 25 | 24RE (CA) | | |
| A10 | F | L | 32.97 | 36.66 | 3, 12, 21 | 25, 25, 25 | CI512 | | |
| A11 | F | R | 48.47 | 59.6 | 3, 12, 21 | 25, 25, 25 | 24RE (CA) | | |
| A12 | F | R | 64.92 | 65.57 | 3, 12, 21 | 25, 25, 25 | CI532 | | |
| A13L | M | L | 70.21 | 70.43 | 3, 12, 21 | 25, 25, 25 | CI532 | | |
| A13R | M | R | 68.67 | 70.43 | 3, 12, 21 | 25, 25, 25 | CI532 | | |
| A14 | F | L | 72.46 | 76.62 | 3, 12, 21 | 25, 25, 25 | CI522 | | |
| A15 | F | R | 15.23 | 28.73 | 3, 12, 21 | 25, 25, 25 | 24RE | | |
| A16 | M | R | 73.00 | 74.19 | 3, 9, 12 | 25, 25, 25 | CI522 | | |
| A17 | M | L | 74.03 | 79.01 | 3, 12, 21 | 25, 25, 25 | CI422 | | |
| A18 | M | R | 58.98 | 59.52 | 3, 12, 21 | 25, 25, 25 | CI532 | | |
| A19 | F | R | 75.74 | 79.78 | 3, 12, 21 | 25, 25, 25 | 24RE | | |
| A20 | M | L | 68.50 | 70.23 | 11, 18, 20 | 25, 25, 25 | CI532 | | |

| | | | | | | | | | |
|---|---|---|---|---|---|---|---|---|---|
| *Two Small nerves in the auditory canal #Single nerve in the internal auditory canal (i.e. nerve diameter <3 mm). AAI, age at implantation; AAT, age at testing; PPD, pulse phase duration; CN, cochlear nerve; 24RE (CA), Freedom Contour Advance electrode array; 24RE (ST), Freedom Straight electrode array; IP-2, incomplete partition 2. | | | | | | | | | |

**TABLE 2. Mathematical formulation for three regression models.**

| Model | **Regularization** | Optimization problem | **Hyperparameters** |
|---|---|---|---|
| Linear | **Elastic net** | ${min}_{β , {β}_{0}} \left[\frac{1}{N}{\sum }_{i = 1}^{N} {\left({β}^{T}{x}_{i}+{β}_{0}-{y}_{i}\right)}^{2} + λ \left({\sum }_{j = 1}^{p} \frac{1}{2} {β}_{j}^{2} + \left\|{β}_{j}\right\|\right)\right]$ | *λ* = 0.128 |
| SVM | L₂ **norm** | ${min}_{β , {β}_{0} , s} \left[{\sum }_{j = 1}^{p} \frac{1}{2} {β}_{j}^{2} + C {\sum }_{i = 1}^{N} {s}_{i}\right] ,$ such that **\|(*β^{T}xᵢ*)*a*** + ***β*₀ *- yᵢ*\| ≤ *ε + sᵢ* and *sᵢ* ≥ 0** | *C* = 0.001 |
| | | | *ε* = 0.005 |
| | | | *a* = 2.699 |
| Logistic | **Elastic net** | ${min}_{β , {β}_{0}} \left[\begin{matrix}\frac{1}{N} \left({\sum }_{i = 1}^{N} - {y}_{i} ln \left(f\right) - \left(1-{y}_{i}\right) ln \left(1-f\right)\right) \\ + λ \left({\sum }_{j = 1}^{p} \frac{1}{2} {β}_{j}^{2} + \left\|{β}_{j}\right\|\right)\end{matrix}\right]$ where | *λ* = 0.013 |
| | | *f* = (1 *+ e⁻***^{*βTxi-β*0})**^{*-*1} | |

| | | | |
|---|---|---|---|
| **SVM, support** vector machine; *β* = [*β*₁ *β*₂ ... *β*₁₂], vector of model parameters; *β*₀, model intercept term; *N* = 56, number of observations in training data set; x, vector of electrically-evoked compound action potential (eCAP) parameters; y, output vector; *λ*, regularization parameter; p = 12, number of eCAP parameters; s, vector of slack parameters; C, box constraint; *ε*, error margin; *a*, kernel scaling factor. | | | |

**TABLE 3. Statistical results when comparing eCAP parameters across electrode locations for three study groups.**

| **eCAP Variables** | **Statistical Test** | **CND** | **NSCN** | **Adult** |
|---|---|---|---|---|
| **t₀** | **ANOVA** | **F_{(2,69)}=6.59, p=0.002** | **F_{(2,93)}=4.46, p=0.014** | **F_{(2,69)}=6.47, p=0.003** |
| | **HSD** | **B<A, p=0.002** | **M>A, p = 0.012** | **B>A, p = 0.002** |
| **eCAP threshold** | **ANOVA** | **F_{(2,69)}=6.51, p=0.003** | **F_{(2,93)}=12.40, p<0.001** | **F_{(2,69)}=6.47, p=0.003** |
| | **HSD** | **B<A, p = 0.002** | **B<M, p<0.001** | **B>A, p=0.007** |
| | | | **M>A, p<0.001** | **M>A, p=0.001** |
| **Slope of I/O function** | **ANOVA** | **F_{(2,69)}=0.11, p=0.893** | **F_{(2,93)}=1.15, p=0.322** | **F_{(2,69)}=2.20, p=0.118** |
| | **HSD** | **NS** | **NS** | **NS** |
| **N1 latency** | **ANOVA** | **F_{(2,69)}=0.22, p=0.807** | **F_{(2,93)}=2.91, p=0.060** | **F_{(2,69)}=5.00, p=0.009** |
| | **HSD** | **NS** | **NS** | **B>A, p=0.007** |

| | | | | |
|---|---|---|---|---|
| ANOVA: analysis of variance; HSD: Tukey's honest significant difference post-hoc test; CND: cochlear nerve deficiency study group; NSCN: normal-sized cochlear nerve study group; Adult, adult study group; B: basal electrode; M: middle electrode; A: apical electrode; NS: not significant; t₀: estimate of absolute refractory period derived from the refractory recovery function; eCAP: electrically-evoked Compound Action Potential; I/O: input/output | | | | |

**TABLE 4. Model parameters for three predictive models listed by eCAP parameter and the expected neural function at different electrode locations based on literature.**

| Model parameter | eCAP parameter | Expected neural function | Reqression model | | |
|---|---|---|---|---|---|
| | | | Linear | SVM | Logistic |
| *β*₀ | | | 0.571 | 0.633 | -0.584 |
| *β*₁ | *t*₀ | Low | -0.063 | -0.038 | -0.553 |
| *β*₂ | Threshold | Low | -0.170 | -0.040 | -2.438 |
| *β*₃ | Slope | Low | 0 | -0.001 | -0.037 |
| *β*₄ | N1 latency | Low | -0.008 | -0.013 | 0 |
| *β*₅ | *t*₀ | Medium | 0 | -0.009 | 0 |
| *β*₆ | Threshold | Medium | -0.023 | -0.019 | -1.918 |
| *β*₇ | Slope | Medium | 0 | 0.008 | 0 |
| *β*₈ | N1 latency | Medium | -0.053 | -0.017 | -0.055 |
| *β*₉ | *t*₀ | High | 0 | -0.008 | 0 |
| *β*₁₀ | Threshold | High | 0 | -0.014 | 0 |
| *β*₁₁ | Slope | High | 0.042 | 0.011 | 0 |
| *β*₁₂ | N1 latency | High | -0.058 | -0.024 | -0.529 |

| | | | | | |
|---|---|---|---|---|---|
| SVM: support vector machine | | | | | |

### SUPPLEMENTAL DIGITAL CONTENT

### CLASSIFICATION METHODS

We compared three different algorithms for classifying the presence of cochlear nerve deficiency (CND) for pediatric cochlear implant (CI) users from recordings of electrically-evoked compound action potentials (eCAPs). Each participant was classified as having CND and given a label of 0 or as having a normal-sized cochlear nerve (NSCN) and given a label of 1. The three classifying algorithms that were compared were linear regression with elastic net regularization, support vector machine regression with a linear kernel, and logistic regression with elastic net regularization. The classification boundary was chosen to be 0.5 for all three models. In other words, if the predicted score for an individual patient was greater than 0.5, the individual would be classified as having a NSCN (i.e., label = 1). Otherwise, the individual was classified as having CND (i.e., label = 0).

### Five-fold cross validation

The data from all participants were first randomly split into 5 folds of equal size, each fold having 1/5 of the children with CND and 1/5 of the children with a NSCN. In each run, 4 folds were used as the training data set to find the model parameters, and the remaining fold was used to test the model. A total of 5 training and testing runs were completed in which each run used a different fold as the testing fold. The model performance was reported as the average over the 5 runs.

### Evaluation Metrics

Accuracy, precision, recall and F-measure score were used to evaluate the performance of the different algorithms and are defined below. For all of these metrics, the possible values are between 0 and 1, where larger values indicate better classification performance.
Accuracy: the fraction of all correctly classified participants over all participants. Precision: the fraction of correctly classified children with CND over all the participants that were classified (either correctly or incorrectly) as having CND.
Recall: the fraction of correctly classified children with CND over the total number of children with CND.
F-measure score: The harmonic mean of precision and recall. Mathematically, this is written as $F 1 = 2 \times \frac{recall \times precision}{recall + precision}$).

### RESULTS

The performance of each machine learning algorithm in classifying children with CND and children with NSCNs are provided in Table A1 below.

**TABLE A1. Performance metrics for three classification algorithms.**

| Performance Metric | Linear | SMV | Logistic |
|---|---|---|---|
| Accuracy | 0.93 | 0.91 | 0.95 |
| Precision | 1.00 | 1.00 | 0.97 |
| Recall | 0.84 | 0.77 | 0.92 |
| F-measure | 0.90 | 0.84 | 0.94 |

| | | | |
|---|---|---|---|
| SVM: support vector machine; F-measure: Harmonic mean of precision and recall | | | |

## Claims

1. A system for determining whether an electrically evoked compound action potential, eCAP, exists in a neural response comprising:
a memory; and
a processor in communication with the memory, wherein the processor executes computer-executable instructions stored in the memory, said instructions causing the processor to:
retrieve a template eCAP waveform from the memory;
receive a recorded neural response waveform obtained from a patient with a cochlear implant;
re-sample the recorded neural response waveform;
normalize the re-sampled neural response waveform and the template eCAP waveform by subtracting mean voltages recorded in a first time period from the template eCAP waveform and the re-sampled neural response waveform;
determine a first negative peak (N1) and a trailing positive peak (P2) in each of the re-sampled neural response waveform and the template eCAP waveform;
scale the template eCAP waveform vertically, in the voltage axis, to match the first negative peak (N1) and
the trailing positive peak (P2) amplitudes from the re-sampled neural response waveform and horizontally, in the time axis, to match the first negative peak (N1) and the trailing positive peak (P2) latencies from the re-sampled neural response waveform;
trim any portion of the re-sampled neural response waveform and the scaled template eCAP waveform to a time period where both waveforms overlap;
re-sample both the re-sampled neural response waveform and the scaled template eCAP waveform at the same time periods as each other with higher resolution sampling occurring before the first time period to place emphasis on a first part of the waveforms in a correlation analysis;
calculate a correlation between the re-sampled re-sampled neural response waveform and the re-sampled scaled template eCAP waveform; and
determine whether the correlation between the re-sampled re-sampled neural response waveform and the re-sampled scaled template eCAP waveform indicates that the recorded neural response waveform comprises an eCAP.

2. The system of claim 1, wherein the template eCAP waveform comprises an eCAP waveform that was made from a neural response measured in a child with a Gap Junction Beta-2 gene, GJB2, genetic mutation that causes hearing loss without impacting the auditory nerve.

3. The system of any one of claims 1-2 wherein re-sampling the recorded neural response waveform comprises up-sampling the recorded neural response waveform via cubic spline interpolation to create a smooth re-sampled neural response waveform at a higher effective sampling rate.

4. The system of any one of claims 1-3, wherein subtracting mean voltages recorded in the first time period from the template eCAP waveform and the re-sampled neural response waveform comprises subtracting mean voltages recorded in the first 600 µ-sec from the template eCAP waveform and the re-sampled neural response waveform.

5. The system of any one of claims 1-4, wherein re-sampling both the re- sampled neural response waveform and the scaled template eCAP waveform at the same time periods as each other with higher resolution sampling occurring before the first time period to place emphasis on the first part of the waveforms in the correlation analysis, comprises re-sampling both waveforms with higher resolution sampling occurring before 600 µ-sec to place emphasis on the first part of the waveforms in the correlation analysis.

6. The system of any one of claims 1-5, wherein calculating the correlation between the re-sampled re-sampled neural response waveform and the re-sampled scaled template eCAP waveform comprises calculating a Pearson correlation between the re- sampled re-sampled neural response waveform and the re-sampled scaled template eCAP waveform.

7. The system of claim 6, wherein determining whether the correlation between the re-sampled re-sampled neural response waveform and the re-sampled scaled template eCAP waveform indicates that the recorded neural response waveform comprises an eCAP comprises determining the Pearson correlation value is larger than 0.6 and the estimated eCAP amplitude is greater than 5 uV an eCAP is determined to be present, otherwise, an eCAP is determined to not be present.

8. A computer-program product comprising computer-executable instructions stored on a non-transitory medium, said computer-executable instructions for performing a method of determining whether an electrically evoked compound action potential, eCAP, exists in a neural response, said method comprising:
receiving a template eCAP waveform;
receiving a recorded neural response waveform obtained from a patient with a cochlear implant;
re-sampling the recorded neural response waveform;
normalizing the re-sampled neural response waveform and the template eCAP waveform by subtracting mean voltages recorded in a first time period from the template eCAP waveform and the re-sampled neural response waveform;
determining a first negative peak (N1) and a trailing positive peak (P2) in each of the re-sampled neural response waveform and the template eCAP waveform;
scaling the template eCAP waveform vertically, in the voltage axis, to match the first negative peak (N1) and the trailing positive peak (P2) amplitudes from the re-sampled neural response waveform and horizontally, in the time axis, to match the first negative peak (N1) and the trailing positive peak (P2) latencies from the re-sampled neural response waveform;
trimming any portion of the re-sampled neural response waveform and the scaled template eCAP waveform to a time period where both waveforms overlap;
re-sampling both the re-sampled neural response waveform and the scaled template eCAP waveform at the same time periods as each other with higher resolution sampling occurring before the first time period to place emphasis on a first part of the waveforms in a correlation analysis;
calculating a correlation between the re-sampled re-sampled neural response waveform and the re-sampled scaled template eCAP waveform; and
determining whether the correlation between the re-sampled re-sampled neural response waveform and the re-sampled scaled template eCAP waveform indicates that the recorded neural response waveform comprises an eCAP.

9. The computer-program product of claim 8, wherein providing the template eCAP waveform comprises providing an eCAP waveform that was made from a neural response measured in a child with a Gap Junction Beta-2 gene, GJB2, genetic mutation that causes hearing loss without impacting the auditory nerve.

10. The computer-program product of any one of claims 8-9, wherein re-sampling the recorded neural response waveform comprises up-sampling the recorded neural response waveform via cubic spline interpolation to create a smooth re-sampled neural response waveform at a higher effective sampling rate.

11. The computer-program product of any one of claims 8-10, wherein subtracting mean voltages recorded in the first time period from the template eCAP waveform and the re-sampled neural response waveform comprises subtracting mean voltages recorded in the first 600 µ-sec from the template eCAP waveform and the re-sampled neural response waveform.

12. The computer-program product of any one of claims 8-11, wherein re-sampling both the re-sampled neural response waveform and the scaled template eCAP waveform at the same time periods as each other with higher resolution sampling occurring before the first time period to place emphasis on the first part of the waveforms in the correlation analysis comprises re-sampling both waveforms with higher resolution sampling occurring before 600 µ-sec to place emphasis on the first part of the waveforms in the correlation analysis.

13. The computer-program product of any one of claims 8-12, wherein calculating the correlation between the re-sampled re-sampled neural response waveform and the re- sampled scaled template eCAP waveform comprises calculating a Pearson correlation between the re-sampled re-sampled neural response waveform and the re-sampled scaled template eCAP waveform; in particular
wherein determining whether the correlation between the re-sampled re-sampled neural response waveform and the re-sampled scaled template eCAP waveform indicates that the recorded neural response waveform comprises an eCAP comprises determining the Pearson correlation value is larger than 0.6 and the estimated eCAP amplitude is greater than 5 uV**,** an eCAP is determined to be present, otherwise, an eCAP is determined to not be present.

## Patentansprüche

1. System zur Bestimmung, ob ein elektrisch evoziertes Summenaktionspotential, eCAP, in einer neuronalen Antwort vorliegt, umfassend:
einen Speicher; und
einen Prozessor in Kommunikation mit dem Speicher, wobei der Prozessor computerlesbare Anweisungen ausführt, die in dem Speicher gespeichert sind, die den Prozessor veranlassen zum:
Abrufen einer eCAP-Vorlagenwellenform aus dem Speicher;
Empfangen einer aufgezeichneten neuronalen Antwortwellenform, die von einem Patienten mit einem Cochlea-Implantat erhalten wurde;
Neuabtasten der aufgezeichneten neuronalen Antwortwellenform;
Normalisieren der neu abgetasteten neuronalen Antwortwellenform und der eCAP-Vorlagenwellenform durch Subtrahieren von mittleren Spannungen, die in einer ersten Zeitspanne aufgezeichnet wurden, von der eCAP-Vorlagenwellenform und der neu abgetasteten neuronalen Antwortwellenform;
Bestimmen eines ersten negativen Peaks (N1) und eines nachfolgenden positiven Peaks (P2) in jeder der neu abgetasteten neuronalen Antwortwellenform und der eCAP-Vorlagenwellenform;
Skalieren der eCAP-Vorlagenwellenform vertikal in der Spannungsachse, um sie an die Amplituden des ersten negativen Peaks (N1) und des nachfolgenden positiven Peaks (P2) aus der neu abgetasteten neuronalen Antwortwellenform anzupassen, und horizontal in der Zeitachse, um sie an die Latenzen des ersten negativen Peaks (N1) und des nachfolgenden positiven Peaks (P2) aus der neu abgetasteten neuronalen Antwortwellenform anzupassen;
Zuschneiden jeglichen Abschnitts der neu abgetasteten neuronalen Antwortwellenform und der skalierten eCAP-Vorlagenwellenform auf eine Zeitspanne, in der sich beide Wellenformen überlappen;
Neuabtasten sowohl der neu abgetasteten neuronalen Antwortwellenform als auch der skalierten eCAP-Vorlagenwellenform in denselben Zeitspannen wie einander, wobei eine Abtastung mit höherer Auflösung vor der ersten Zeitspanne erfolgt, um in einer Korrelationsanalyse einen ersten Teil der Wellenformen hervorzuheben;
Berechnen einer Korrelation zwischen der neu abgetasteten neu abgetasteten neuronalen Antwortwellenform und der neu abgetasteten skalierten eCAP-Vorlagenwellenform; und
Bestimmen, ob die Korrelation zwischen der neu abgetasteten neu abgetasteten neuronalen Antwortwellenform und der neu abgetasteten skalierten eCAP-Vorlagenwellenform anzeigt, dass die aufgezeichnete neuronale Antwortwellenform ein eCAP umfasst.

2. System nach Anspruch 1, wobei die eCAP-Vorlagenwellenform eine eCAP-Wellenform umfasst, die aus einer neuronalen Antwort erzeugt wurde, die bei einem Kind mit einer genetischen Mutation des Gap-Junction-Beta-2-Gens, GJB2, gemessen wurde, die einen Hörverlust verursacht, ohne den Hörnerv zu beeinträchtigen.

3. System nach einem der Ansprüche 1 bis 2, wobei das Neuabtasten der aufgezeichneten neuronalen Antwortwellenform das Aufwärtsabtasten der aufgezeichneten neuronalen Antwortwellenform mittels kubischer Spline-Interpolation umfasst, um eine glatte neu abgetastete neuronale Antwortwellenform mit einer höheren effektiven Abtastrate zu erzeugen.

4. System nach einem der Ansprüche 1 bis 3, wobei das Subtrahieren von in der ersten Zeitspanne aufgezeichneten mittleren Spannungen von der eCAP-Vorlagenwellenform und der neu abgetasteten neuronalen Antwortwellenform das Subtrahieren von in den ersten 600 µs aufgezeichneten mittleren Spannungen von der eCAP-Vorlagenwellenform und der neu abgetasteten neuronalen Antwortwellenform umfasst.

5. System nach einem der Ansprüche 1 bis 4, wobei das Neuabtasten sowohl der neu abgetasteten neuronalen Antwortwellenform als auch der skalierten eCAP-Vorlagenwellenform zu denselben Zeitspannen wie die jeweils andere, wobei eine Abtastung mit höherer Auflösung vor der ersten Zeitspanne erfolgt, um den ersten Teil der Wellenformen in der Korrelationsanalyse hervorzuheben, das Neuabtasten beider Wellenformen umfasst, wobei die Abtastung mit höherer Auflösung vor 600 µs erfolgt, um den ersten Teil der Wellenformen in der Korrelationsanalyse hervorzuheben.

6. System nach einem der Ansprüche 1 bis 5, wobei das Berechnen der Korrelation zwischen der neu abgetasteten neu abgetasteten neuronalen Antwortwellenform und der neu abgetasteten skalierten eCAP-Vorlagenwellenform das Berechnen einer Pearson-Korrelation zwischen der neu abgetasteten neu abgetasteten neuronalen Antwortwellenform und der neu abgetasteten skalierten eCAP-Vorlagenwellenform umfasst.

7. System nach Anspruch 6, wobei das Bestimmen, ob die Korrelation zwischen der neu abgetasteten neu abgetasteten neuronalen Antwortwellenform und der neu abgetasteten skalierten eCAP-Vorlagenwellenform anzeigt, dass die aufgezeichnete neuronale Antwortwellenform ein eCAP umfasst, das Bestimmen umfasst, dass der Pearson-Korrelationswert größer als 0,6 ist und die geschätzte eCAP-Amplitude größer als 5 uV ist, wobei ein eCAP als vorhanden bestimmt wird, andernfalls ein eCAP als nicht vorhanden bestimmt wird.

8. Computerprogrammprodukt, umfassend computerausführbare Anweisungen, die auf einem nichtflüchtigen Medium gespeichert sind, wobei die computerausführbaren Anweisungen zur Durchführung eines Verfahrens zum Bestimmen, ob ein elektrisch evoziertes Summenaktionspotential, eCAP, in einer neuronalen Antwort vorliegt, dienen, wobei das Verfahren umfasst:
Empfangen einer eCAP-Vorlagenwellenform;
Empfangen einer aufgezeichneten neuronalen Antwortwellenform, die von einem Patienten mit einem Cochlea-Implantat erhalten wurde;
Neuabtasten der aufgezeichneten neuronalen Antwortwellenform;
Normalisieren der neu abgetasteten neuronalen Antwortwellenform und der eCAP-Vorlagenwellenform durch Subtrahieren von mittleren Spannungen, die in einer ersten Zeitspanne aufgezeichnet wurden, von der eCAP-Vorlagenwellenform und der neu abgetasteten neuronalen Antwortwellenform;
Bestimmen eines ersten negativen Peaks (N1) und eines nachfolgenden positiven Peaks (P2) jeweils in der neu abgetasteten neuronalen Antwortwellenform und der eCAP-Vorlagenwellenform;
Skalieren der eCAP-Vorlagenwellenform vertikal, in der Spannungsachse, zur Anpassung an die Amplituden des ersten negativen Peaks (N1) und des nachfolgenden positiven Peaks (P2) aus der neu abgetasteten neuronalen Antwortwellenform und horizontal, in der Zeitachse, zur Anpassung an die Latenzen des ersten negativen Peaks (N1) und des nachfolgenden positiven Peaks (P2) aus der neu abgetasteten neuronalen Antwortwellenform;
Zuschneiden eines beliebigen Abschnitts der neu abgetasteten neuronalen Antwortwellenform und der skalierten eCAP-Vorlagenwellenform auf eine Zeitspanne, in der sich beide Wellenformen überlappen;
Neuabtasten sowohl der neu abgetasteten neuronalen Antwortwellenform als auch der skalierten eCAP-Vorlagenwellenform in jeweils denselben Zeitspannen wie einander, wobei ein Abtasten mit höherer Auflösung vor der ersten Zeitspanne erfolgt, um einen ersten Teil der Wellenformen in einer Korrelationsanalyse hervorzuheben;
Berechnen einer Korrelation zwischen der neu abgetasteten neu abgetasteten neuronalen Antwortwellenform und der neu abgetasteten skalierten eCAP-Vorlagenwellenform; und
Bestimmen, ob die Korrelation zwischen der neu abgetasteten neu abgetasteten neuronalen Antwortwellenform und der neu abgetasteten skalierten eCAP-Vorlagenwellenform anzeigt, dass die aufgezeichnete neuronale Antwortwellenform ein eCAP umfasst.

9. Computerprogrammprodukt nach Anspruch 8, wobei das Bereitstellen der eCAP-Vorlagenwellenform das Bereitstellen einer eCAP-Wellenform umfasst, die aus einer neuronalen Antwort erstellt wurde, die bei einem Kind mit einer genetischen Mutation des Gap-Junction-Beta-2-Gens, GJB2, gemessen wurde, die einen Hörverlust verursacht, ohne den Hörnerv zu beeinträchtigen.

10. Computerprogrammprodukt nach einem der Ansprüche 8 bis 9, wobei das Neuabtasten der aufgezeichneten neuronalen Antwortwellenform ein Aufwärtsabtasten der aufgezeichneten neuronalen Antwortwellenform mittels kubischer Spline-Interpolation umfasst, um eine glatte neu abgetastete neuronale Antwortwellenform mit einer höheren effektiven Abtastrate zu erzeugen.

11. Computerprogrammprodukt nach einem der Ansprüche 8 bis 10, wobei das Subtrahieren von in der ersten Zeitspanne aufgezeichneten mittleren Spannungen von der eCAP-Vorlagenwellenform und der neu abgetasteten neuronalen Antwortwellenform das Subtrahieren von in den ersten 600 µs aufgezeichneten mittleren Spannungen von der eCAP-Vorlagenwellenform und der neu abgetasteten neuronalen Antwortwellenform umfasst.

12. Computerprogrammprodukt nach einem der Ansprüche 8 bis 11, wobei das Neuabtasten sowohl der neu abgetasteten neuronalen Antwortwellenform als auch der skalierten eCAP-Vorlagenwellenform zu denselben Zeitspannen wie einander, wobei ein Abtasten mit höherer Auflösung vor der ersten Zeitspanne erfolgt, um den ersten Teil der Wellenformen in der Korrelationsanalyse hervorzuheben, das Neuabtasten beider Wellenformen umfasst, wobei das Abtasten mit höherer Auflösung vor 600 µs erfolgt, um den ersten Teil der Wellenformen in der Korrelationsanalyse hervorzuheben.

13. Computerprogrammprodukt nach einem der Ansprüche 8 bis 12, wobei das Berechnen der Korrelation zwischen der neu abgetasteten neu abgetasteten neuronalen Antwortwellenform und der neu abgetasteten skalierten eCAP-Vorlagenwellenform das Berechnen einer Pearson-Korrelation zwischen der neu abgetasteten neu abgetasteten neuronalen Antwortwellenform und der neu abgetasteten skalierten eCAP-Vorlagenwellenform umfasst; insbesondere wobei das Bestimmen, ob die Korrelation zwischen der neu abgetasteten neu abgetasteten neuronalen Antwortwellenform und der neu abgetasteten skalierten eCAP-Vorlagenwellenform anzeigt, dass die aufgezeichnete neuronale Antwortwellenform ein eCAP umfasst, das Bestimmen umfasst, dass der Pearson-Korrelationswert größer als 0,6 ist und die geschätzte eCAP-Amplitude größer als 5 uV ist, wobei ein eCAP als vorhanden bestimmt wird, andernfalls ein eCAP als nicht vorhanden bestimmt wird.

## Revendications

1. Système permettant de déterminer si un potentiel d'action composé évoqué électriquement, eCAP, existe dans une réponse neuronale comprenant :
une mémoire ; et
un processeur en communication avec la mémoire, dans lequel le processeur exécute des instructions lisibles par ordinateur stockées dans la mémoire, lesdites instructions amenant le processeur à :
récupérer une forme d'onde eCAP modèle de la mémoire ;
recevoir une forme d'onde de réponse neuronale enregistrée obtenue à partir d'un patient porteur d'un implant cochléaire ;
ré-échantillonner la forme d'onde de réponse neuronale enregistrée ;
normaliser la forme d'onde de réponse neuronale ré-échantillonnée et la forme d'onde eCAP modèle en soustrayant des tensions moyennes enregistrées dans un premier laps de temps de la forme d'onde eCAP modèle et de la forme d'onde de réponse neuronale ré-échantillonnée ;
déterminer un premier pic négatif (N1) et un pic positif suiveur (P2) dans chacune de la forme d'onde de réponse neuronale ré-échantillonnée et de la forme d'onde eCAP modèle ;
mettre à l'échelle la forme d'onde eCAP modèle verticalement, dans l'axe de la tension, pour faire correspondre les amplitudes du premier pic négatif (N1) et du pic positif suiveur (P2) de la forme d'onde de réponse neuronale ré-échantillonnée et horizontalement, dans l'axe du temps, pour faire correspondre les latences du premier pic négatif (N1) et du pic positif suiveur (P2) à partir de la forme d'onde de réponse neuronale ré-échantillonnée ;
couper toute partie de la forme d'onde de réponse neuronale ré-échantillonnée et de la forme d'onde eCAP modèle mise à l'échelle sur un laps de temps où les deux formes d'onde se chevauchent ;
ré-échantillonner à la fois la forme d'onde de réponse neuronale ré-échantillonnée et la forme d'onde eCAP modèle mise à l'échelle aux mêmes laps de temps l'une par rapport à l'autre, avec un échantillonnage à plus haute résolution se produisant avant le premier laps de temps pour mettre l'accent sur une première partie des formes d'onde dans une analyse de corrélation ;
calculer une corrélation entre la forme d'onde de réponse neuronale ré-échantillonnée et ré-échantillonnée et la forme d'onde eCAP modèle mise à l'échelle ré-échantillonnée ; et
déterminer si la corrélation entre la forme d'onde de réponse neuronale ré-échantillonnée et ré-échantillonnée et la forme d'onde eCAP modèle mise à l'échelle ré-échantillonnée indique que la forme d'onde de réponse neuronale enregistrée comprend un eCAP.

2. Système selon la revendication 1, dans lequel la forme d'onde eCAP modèle comprend une forme d'onde eCAP qui a été faite à partir d'une réponse neuronale mesurée chez un enfant porteur d'une mutation génétique du gène Gap Junction Beta-2, GJB2, qui provoque une perte auditive sans affecter le nerf auditif.

3. Système selon l'une quelconque des revendications 1 à 2, dans lequel le ré-échantillonnage de la forme d'onde de réponse neuronale enregistrée comprend le sur-échantillonnage de la forme d'onde de réponse neuronale enregistrée par l'intermédiaire d'une interpolation par spline cubique pour créer une forme d'onde de réponse neuronale ré-échantillonnée lisse à une fréquence d'échantillonnage effective plus élevée.

4. Système selon l'une quelconque des revendications 1 à 3, dans lequel la soustraction de tensions moyennes enregistrées dans un premier laps de temps de la forme d'onde eCAP modèle et de la forme d'onde de réponse neuronale ré-échantillonnée comprend la soustraction de tensions moyennes enregistrées dans les 600 premières µ-sec de la forme d'onde eCAP modèle et de la forme d'onde de réponse neuronale ré-échantillonnée.

5. Système selon l'une quelconque des revendications 1 à 4, dans lequel le ré-échantillonnage à la fois de la forme d'onde de réponse neuronale ré-échantillonnée et de la forme d'onde eCAP modèle mise à l'échelle aux mêmes laps de temps l'une par rapport à l'autre avec un échantillonnage à plus haute résolution se produisant avant le premier laps de temps pour mettre l'accent sur la première partie des formes d'onde dans l'analyse de corrélation, comprend un ré-échantillonnage des deux formes d'onde avec un échantillonnage à plus haute résolution se produisant avant 600 µ-sec pour mettre l'accent sur la première partie des formes d'onde dans l'analyse de corrélation.

6. Système selon l'une quelconque des revendications 1 à 5, dans lequel le calcul de la corrélation entre la forme d'onde de réponse neuronale ré-échantillonnée et ré-échantillonnée et la forme d'onde eCAP modèle ré-échantillonnée comprend le calcul d'une corrélation de Pearson entre la forme d'onde de réponse neuronale ré-échantillonnée et ré-échantillonnée et la forme d'onde eCAP modèle mise à l'échelle ré-échantillonnée.

7. Système selon la revendication 6, dans lequel le fait de déterminer si la corrélation entre la forme d'onde de réponse neuronale ré-échantillonnée et ré-échantillonnée et la forme d'onde eCAP modèle mise à l'échelle ré-échantillonnée indique que la forme d'onde de réponse neuronale enregistrée comprend un eCAP comprend le fait de déterminer que, si la valeur de corrélation de Pearson est supérieure à 0,6 et que l'amplitude eCAP estimée est supérieure à 5 uV, un eCAP est déterminé comme étant présent, sinon, il est déterminé qu'un eCAP n'est pas présent.

8. Produit-programme d'ordinateur comprenant des instructions exécutables par ordinateur stockées sur un support non transitoire, lesdites instructions exécutables par ordinateur permettant d'effectuer un procédé consistant à déterminer si un potentiel d'action composé évoqué électriquement, eCAP, existe dans une réponse neuronale, ledit procédé comprenant :
la réception d'une forme d'onde eCAP modèle ;
la réception d'une forme d'onde de réponse neuronale enregistrée obtenue à partir d'un patient porteur d'un implant cochléaire ;
le ré-échantillonnage de la forme d'onde de réponse neuronale enregistrée ;
la normalisation de la forme d'onde de réponse neuronale ré-échantillonnée et de la forme d'onde eCAP modèle en soustrayant des tensions moyennes enregistrées dans un premier laps de temps de la forme d'onde eCAP modèle et de la forme d'onde de réponse neuronale ré-échantillonnée ;
la détermination d'un premier pic négatif (N1) et d'un pic positif suiveur (P2) dans chacune de la forme d'onde de réponse neuronale ré-échantillonnée et de la forme d'onde eCAP modèle ;
la mise à l'échelle de la forme d'onde eCAP modèle verticalement, dans l'axe de la tension, pour faire correspondre les amplitudes du premier pic négatif (N1) et du pic positif suiveur (P2) de la forme d'onde de réponse neuronale ré-échantillonnée et horizontalement, dans l'axe du temps, pour faire correspondre les latences du premier pic négatif (N1) et du pic positif suiveur (P2) à partir de la forme d'onde de réponse neuronale ré-échantillonnée ;
le découpage de toute partie de la forme d'onde de réponse neuronale ré-échantillonnée et de la forme d'onde eCAP modèle mise à l'échelle sur un laps de temps où les deux formes d'onde se chevauchent ;
le ré-échantillonnage à la fois de la forme d'onde de réponse neuronale ré-échantillonnée et de la forme d'onde eCAP modèle mise à l'échelle aux mêmes laps de temps l'une par rapport à l'autre, avec un échantillonnage à plus haute résolution se produisant avant le premier laps de temps pour mettre l'accent sur une première partie des formes d'onde dans une analyse de corrélation ;
le calcul d'une corrélation entre la forme d'onde de réponse neuronale ré-échantillonnée et ré-échantillonnée et la forme d'onde eCAP modèle mise à l'échelle ré-échantillonnée ; et
le fait de déterminer si la corrélation entre la forme d'onde de réponse neuronale ré-échantillonnée et ré-échantillonnée et la forme d'onde eCAP modèle mise à l'échelle ré-échantillonnée indique que la forme d'onde de réponse neuronale enregistrée comprend un eCAP.

9. Produit-programme d'ordinateur selon la revendication 8, dans lequel le fait de fournir la forme d'onde eCAP modèle comprend le fait de fournir une forme d'onde eCAP qui a été faite à partir d'une réponse neuronale mesurée chez un enfant porteur d'une mutation génétique du gène Gap Junction Beta-2, GJB2, qui provoque une perte auditive sans affecter le nerf auditif.

10. Produit-programme d'ordinateur selon l'une quelconque des revendications 8 à 9, dans lequel le ré-échantillonnage de la forme d'onde de réponse neuronale enregistrée comprend le sur-échantillonnage de la forme d'onde de réponse neuronale enregistrée par l'intermédiaire d'une interpolation par spline cubique pour créer une forme d'onde de réponse neuronale ré-échantillonnée lisse à une fréquence d'échantillonnage effective plus élevée.

11. Produit-programme d'ordinateur selon l'une quelconque des revendications 8 à 10, dans lequel la soustraction de tensions moyennes enregistrées dans un premier laps de temps de la forme d'onde eCAP modèle et de la forme d'onde de réponse neuronale ré-échantillonnée comprend la soustraction de tensions moyennes enregistrées dans les 600 premières µ-sec de la forme d'onde eCAP modèle et de la forme d'onde de réponse neuronale ré-échantillonnée.

12. Produit-programme d'ordinateur selon l'une quelconque des revendications 8 à 11, dans lequel le ré-échantillonnage à la fois de la forme d'onde de réponse neuronale ré-échantillonnée et de la forme d'onde eCAP modèle mise à l'échelle aux mêmes laps de temps l'une par rapport à l'autre avec un échantillonnage à plus haute résolution se produisant avant le premier laps de temps pour mettre l'accent sur la première partie des formes d'onde dans l'analyse de corrélation, comprend un ré-échantillonnage des deux formes d'onde avec un échantillonnage à plus haute résolution se produisant avant 600 µ-sec pour mettre l'accent sur la première partie des formes d'onde dans l'analyse de corrélation.

13. Produit-programme d'ordinateur selon l'une quelconque des revendications 8 à 12, dans lequel le calcul de la corrélation entre la forme d'onde de réponse neuronale ré-échantillonnée et ré-échantillonnée et la forme d'onde eCAP modèle ré-échantillonnée comprend le calcul d'une corrélation de Pearson entre la forme d'onde de réponse neuronale ré-échantillonnée et ré-échantillonnée et la forme d'onde eCAP modèle mise à l'échelle ré-échantillonnée ; en particulier dans lequel le fait de déterminer si la corrélation entre la forme d'onde de réponse neuronale ré-échantillonnée et ré-échantillonnée et la forme d'onde eCAP modèle mise à l'échelle ré-échantillonnée indique que la forme d'onde de réponse neuronale enregistrée comprend un eCAP comprend le fait de déterminer que, si la valeur de corrélation de Pearson est supérieure à 0,6 et que l'amplitude eCAP estimée est supérieure à 5 uV, un eCAP est déterminé comme étant présent, sinon, il est déterminé qu'un eCAP n'est pas présent.
